# FASCICULE DE BREVET EUROPEEN

(11) **EP 1 453 962 B1**
(45) Date de publication et mention de la délivrance du brevet: **18.08.2010**
(21) Numéro de dépôt: 02793219.3
(22) Date de dépôt: 08.11.2002
(51) Int. Cl.: C12N 15/113, A61K 31/713, A61P 35/00

(54) **OLIGONUCLEOTIDES INHIBITEURS ET LEUR UTILISATION POUR REPRIMER SPECIFIQUEMENT UN GENE CODANT POUR UN RECEPTEUR AUX ANDROGENES**
HEMMENDE OLIGONUKLEOTIDE UND DEREN VERWENDUNG ZUR SPEZIFISCHE HEMMUNG VON EINEM GEN KODIEREND FUR EINEN ANDROGEN-REZEPTOR
INHIBITOR OLIGONUCLEOTIDES AND THEIR USE FOR SPECIFIC REPRESSION OF A GENE ENCODING AN ANDROGEN RECEPTOR

(30) Priorité: 09.11.2001 FR 0114549; 10.04.2002 FR 0204474
(43) Date de publication de la demande: 08.09.2004
(60) Demande divisionnaire: 08001984.7
(73) Titulaire: CENTRE NATIONAL DE LA RECHERCHE SCIENTIFIQUE (CNRS), 75794 Paris Cedex 16 (FR)
(72) Inventeur: HAREL-BELLAN, Annick, F-75005 Paris (FR); AIT-SI-ALI, Slimane, F-94800 Villejuif (FR); CABON-GEORGET, Florence, F-94400 Vitry sur Seine (FR); CHAUCHEREAU, Anne, F-92260 Fontenay-aux-Roses (FR); DAUTRY, François, F-75014 Paris (FR); MARTINEZ, Luis, F-75005 PARIS (FR)
(74) Mandataire: Novagraaf Technologies
(86) Numéro de dépôt international: PCT/FR2002/003843
(87) Numéro de publication internationale: WO 2003/040366

(56) Documents cités:
- EP-A- 0 769 552
- WO-A-00/20432
- WO-A-00/24885
- WO-A-00/63364
- WO-A-00/66724
- WO-A-01/36646
- WO-A-01/36646
- WO-A-01/75164
- WO-A-01/75164
- WO-A-94/18835
- WO-A-95/11301
- WO-A-96/27006
- WO-A-96/39426
- WO-A-02/055692
- WO-A-02/096927
- WO-A-03/008573
- WO-A-03/070910
- WO-A-03/070969
- WO-A-2004/022777
- WO-A-2004/042024
- US-A- 5 556 956
- ELBASHIR SAYDA M ET AL: "RNA interference is mediated by 21- and 22-nucleotide RNAs" GENES AND DEVELOPMENT, vol. 15, no. 2, 15 janvier 2001 (2001-01-15), pages 188-200, XP002204651 ISSN: 0890-9369
- ELBASHIR SAYDA M ET AL: "Duplexes of 21-nucleotide RNAs mediate RNA interference in cultured mammalian cells" NATURE, vol. 411, no. 6836, 24 mai 2001 (2001-05-24), pages 494-498, XP002206451 ISSN: 0028-0836
- ELBASHIR SAYDA M ET AL: "Knockdown of mammalian gene expression using small interfering RNAs" BIOLOGY OF THE CELL, vol. 93, no. 3-4, octobre 2001 (2001-10), page 259, XP002206454 ISSN: 0248-4900
- SEMENZA G L: "HYPOXIA, CLONAL SELECTION, AND THE ROLE OF HIF-1 IN TUMOR PROGRESSION" CRITICAL REVIEWS IN BIOCHEMISTRY AND MOLECULAR BIOLOGY, vol. 35, no. 2, avril 2000 (2000-04), pages 71-103, XP008009591 ISSN: 1040-9238
- VENTURINI FRANCESCA ET AL: "Kinetic selection of HPV 16 E6/E7-directed antisense nucleic acids: Anti-proliferative effects on HPV 16-transformed cells" NUCLEIC ACIDS RESEARCH, vol. 27, no. 7, 1 avril 1999 (1999-04-01), pages 1585-1592, XP002234550 ISSN: 0305-1048
- ELBASHIR S M ET AL: "Functional anatomy of siRNAs for mediating efficient RNAi in Drosophila melanogaster embryo lysate" EMBO JOURNAL, vol. 20, no. 23, 3 décembre 2001 (2001-12-03), pages 6877-6888, XP002225998 ISSN: 0261-4189
- JIANG M ET AL: "SELECTIVE SILENCING OF VIRAL GENE EXPRESSION IN HPV-POSITIVE HUMAN CERVICAL CARCINOMA CELLS TREATED WITH SIRNA, A PRIMER OF RNA INTERFERENCE" ONCOGENE, vol. 21, no. 39, 5 septembre 2002 (2002-09-05), pages 6041-6048, XP008011619 ISSN: 0950-9232
- MARTINEZ L A ET AL: "Synthetic small inhibiting RNAs: efficient tools to inactivate oncogenic mutations and restore p53 pathways" PROCEEDINGS OF THE NATIONAL ACADEMY OF SCIENCES OF USA, vol. 99, no. 23, 12 novembre 2002 (2002-11-12), pages 14849-14854, XP002225999 ISSN: 0027-8424
- FILLEUR STEPHANIE ET AL: "SiRNA-mediated inhibition of vascular endothelial growth factor severely limits tumor resistance to antiangiogenic thrombospondin-1 and slows tumor vascularization and growth." CANCER RESEARCH, vol. 63, no. 14, 15 juillet 2003 (2003-07-15), pages 3919-3922, XP002378581 ISSN: 0008-5472
- ELBASHIR SAYDA M ET AL: "RNA interference is mediated by 21- and 22-nucleotide RNAs." GENES & DEVELOPMENT, vol. 15, no. 2, 15 janvier 2001 (2001-01-15), pages 188-200, XP002206453 ISSN: 0890-9369
- ELBASHIR SAYDA M ET AL: "Duplexes of 21-nucleotide RNAs mediate RNA interference in cultured mammalian cells." NATURE (LONDON), vol. 411, no. 6836, 24 mai 2001 (2001-05-24), pages 494-498, XP002206451 ISSN: 0028-0836
- ELBASHIR SAYDA M ET AL: "Knockdown of mammalian gene expression using small interfering RNAs." BIOLOGY OF THE CELL (PARIS), vol. 93, no. 3-4, octobre 2001 (2001-10), page 259, XP002206454 ISSN: 0248-4900 & First Joint French-German Congress on Cell Biology;Strasbourg, France; November 07-09, 2001.
- ELBASHIR, S. ET AL.: "Functional anatomy of siRNAs for mediating efficient RNAi in Drosophila melanogaster embryo lysate." EMBO (EUROPEAN MOLECULAR BIOLOGY ORGANIZATION) JOURNAL, vol. 20, no. 23, 3 décembre 2001 (2001-12-03), pages 6877-6888, XP0002225998 ISSN: 0261-4189
- DATABASE MEDLINE [Online] US NATIONAL LIBRARY OF MEDICINE (NLM), BETHESDA, MD, US; 2002, FUTAMI TAKASHI ET AL: "Induction of apoptosis in HeLa cells with siRNA expression vector targeted against bcl-2." XP002264267 Database accession no. NLM12903200 & FUTAMI TAKASHI ET AL: "Induction of apoptosis in HeLa cells with siRNA expression vector targeted against bcl-2." NUCLEIC ACIDS RESEARCH. SUPPLEMENT (2001) 2002, no. 2, 2002, pages 251-252,

### LISTAGE DE SEQUENCES

<110> CENTRE NATIONAL DE LA RECERCHE SCIENTIFIQUE
<120> oligonucleotides inhibiteurs et leur utilisation pour reprimer specifiquement un gene
<130> 24240PCT Nov 2002
<140> pct/fr02/xxxxx
<141> 2002-11-08
<150> FR01/14549
<151> 2001-11-09
<150> FR02/04474
<151> 2002-04-10
<160> 77
<170> PatentIn version 3.1
<210> 1
   <211> 1182
   <212> DNA
   <213> Homo sapiens
<220>
   <221> misc_feature
   <222> (1)..(1182)
   <223> Séquence du gène p53
<400> 1
   atggaggagc cgcagtcaga tcctagcgtc gagccccctc tgagtcagga aacattttca      60
   gacctatgga aactacttcc tgaaaacaac gttctgtccc ccttgccgtc ccaagcaatg     120
   gatgatttga tgctgtcccc ggacgatatt gaacaatggt tcactgaaga cccaggtcca     180
   gatgaagctc ccagaatgcc agaggctgct ccccccgtgg cccctgcacc agcagctcct     240
   acaccggcgg cccctgcacc agccccctcc tggcccctgt catcttctgt cccttcccag     300
   aaaacctacc agggcagcta cggtttccgt ctgggcttct tgcattctgg gacagccaag     360
   tctgtgactt gcacgtactc ccctgccctc aacaagatgt tttgccaact ggccaagacc     420
   tgccctgtgc agctgtgggt tgattccaca cccccgcccg gcacccgcgt ccgcgccatg     480
   gccatctaca agcagtcaca gcacatgacg gaggttgtga ggcgctgccc ccaccatgag     540
   cgctgctcag atagcgatgg tctggcccct cctcagcatc ttatccgagt ggaaggaaat     600
   ttgcgtgtgg agtatttgga tgacagaaac acttttcgac atagtgtggt ggtgccctat     660
   gagccgcctg aggttggctc tgactgtacc accatccact acaactacat gtgtaacagt     720
   tcctgcatgg gcggcatgaa ccggaggccc atcctcacca tcatcacact ggaagactcc     780
   agtggtaatc tactgggacg gaacagcttt gaggtgcgtg tttgtgcctg tcctgggaga     840
   gaccggcgca cagaggaaga gaatctccgc aagaaagggg agcctcacca cgagctgccc     900
   ccagggagca ctaagcgagc actgcccaac aacaccagct cctctcccca gccaaagaag     960
   aaaccactgg atggagaata tttcaccctt cagatccgtg ggcgtgagcg cttcgagatg    1020
   ttccgagagc tgaatgaggc cttggaactc aaggatgccc aggctgggaa ggagccaggg    1080
   gggagcaggg ctcactccag ccacctgaag tccaaaaagg gtcagtctac ctcccgccat    1140
   aaaaaactca tgttcaagac agaagggcct gactcagact ga                       1182
<210> 2
   <211> 7904
   <212> DNA
   <213> Homo sapiens
<220>
   <221> misc_feature
   <222> (1)..(7904)
   <223> Variant HPV16
<400> 2
   actacaataa ttcatgtata aaactaaggg cgtaaccgaa atcggttgaa ccgaaaccgg     60
   ttagtataaa agcagacatt ttatgcacca aaagagaact gcaatgtttc aggacccaca    120
   ggagcgaccc agaaagttac cacagttatg cacagagctg caaacaacta tacatgatat    180
   aatattagaa tgtgtgtact gcaagcaaca gttactgcga cgtgaggtat atgactttgc    240
   ttttcgggat ttatgcatag tatatagaga tgggaatcca tatgctgtat gtgataaatg    300
   tttaaagttt tattctaaaa ttagtgagta tagacattat tgttatagtt tgtatggaac    360
   aacattagaa cagcaataca acaaaccgtt gtgtgatttg ttaattaggt gtattaactg    420
   tcaaaagcca ctgtgtcctg aagaaaagca aagacatctg gacaaaaagc aaagattcca    480
   taatataagg ggtcggtgga ccggtcgatg tatgtcttgt tgcagatcat caagaacacg    540
   tagagaaacc cagctgtaat catgcatgga gatacaccta cattgcatga atatatgtta    600
   gatttgcaac cagagacaac tgatctctac tgttatgagc aattaaatga cagctcagag    660
   gaggaggatg aaatagatgg tccagctgga caagcagaac cggacagagc ccattacaat    720
   attgtaacct tttgttgcaa gtgtgactct acgcttcggt tgtgcgtaca aagcacacac    780
   gtagacattc gtactttgga agacctgtta atgggcacac taggaattgt gtgccccatc    840
   tgttctcaga aaccataatc taccatggct gatcctgcag gtaccaatgg ggaagagggt    900
   acgggatgta atggatggtt ttatgtagag gctgtagtgg aaaaaaaaac aggggatgct    960
   atatcagatg acgagaacga aaatgacagt gatacaggtg aagatttggt agattttata   1020
   gtaaatgata atgattattt aacacaggca gaaacagaga cagcacatgc gttgtttact   1080
   gcacaggaag caaaacaaca tagagatgca gtacaggttc taaaacgaaa gtatttggta   1140
   gtccacttag tgatattagt ggatgtgtag acaataatat tagtcctaga ttaaaagcta   1200
   tatgtataga aaaacaaagt agagctgcaa aaaggagatt atttgaaagc gaagacagcg   1260
   ggtatggcaa tactgaagtg gaaactcagc agatgttaca ggtagaaggg cgccatgaga   1320
   ctgaaacacc atgtagtcag tatagtggtg gaagtggggg tggttgcagt cagtacagta   1380
   gtggaagtgg gggagagggt gttagtgaaa gacacactat atgccaaaca ccacttacaa   1440
   atattttaaa tgtactaaaa actagtaatg caaaggcagc aatgttagca aaatttaaag   1500
   agttatacgg ggtgagtttt tcagaattag taagaccatt taaaagtaat aaatcaacgt   1560
   gttgcgattg gtgtattgct gcatttggac ttacacccag tatagctgac agtataaaaa   1620
   cactattaca acaatattgt ttatatttac acattcaaag tttagcatgt tcatggggaa   1680
   tggttgtgtt actattagta agatataaat gtggaaaaaa tagagaaaca attgaaaaat   1740
   tgctgtctaa actattatgt gtgtctccaa tgtgtatgat gatagagcct ccaaaattgc   1800
   gtagtacagc agcagcatta tattggtata aaacaggtat atcaaatatt agtgaagtgt   1860
   atggagacac gccagaatgg atacaaagac aaacagtatt acaacatagt tttaatgatt   1920
   gtacatttga attatcacag atggtacaat gggcctacga taatgacata gtagacgata   1980
   gtgaaattgc atataaatat gcacaattgg cagacactaa tagtaatgca agtgcctttc   2040
   taaaaagtaa ttcacaggca aaaattgtaa aggattgtgc aacaatgtgt agacattata   2100
   aacgagcaga aaaaaaacaa atgagtatga gtcaatggat aaaatataga tgtgataggg   2160
   tagatgatgg aggtgattgg aagcaaattg ttatgttttt aaggtatcaa ggtgtagagt   2220
   ttatgtcatt tttaactgca ttaaaaagat ttttgcaagg catacctaaa aaaaattgca   2280
   tattactata tggtgcagct aacacaggta aatcattatt tggtatgagt ttaatgaaat   2340
   ttctgcaagg gtctgtaata tgttttgtaa attctaaaag ccatttttgg ttacaaccat   2400
   tagcagatgc caaaataggt atgttagatg atgctacagt gccctgttgg aactacatag   2460
   atgacaattt aagaaatgca ttggatggaa atttagtttc tatggatgta aagcatagac   2520
   cattggtaca actaaaatgc cctccattat taattacatc taacattaat gctggtacag   2580
   attctaggtg gccttattta cataatagat tggtggtgtt tacatttcct aatgagtttc   2640
   catttgacga aaacggaaat ccagtgtatg agcttaatga taagaactgg aaatcctttt   2700
   tctcaaggac gtggtccaga ttaagtttgc acgaggacga ggacaaggaa aacgatggag   2760
   actctttgcc aacgtttaaa tgtgtgtcag gacaaaatac taacacatta tgaaaatgat   2820
   agtacagacc tacgtgacca tatagactat tggaaacaca tgcgcctaga atgtgctatt   2880
   tattacaagg ccagagaaat gggatttaaa catattaacc accaagtggt gccaacactg   2940
   gctgtatcaa agaataaagc attacaagca attgaactgc aactaacgtt agaaacaata   3000
   tataactcac aatatagtaa tgaaaagtgg acattacaag acgttagcct tgaagtgtat   3060
   ttaactgcac caacaggatg tataaaaaaa catggatata cagtggaagt gcagtttgat   3120
   ggagacatat gcaatacaat gcattataca aactggacac atatatatat ttgtgaagaa   3180
   gcatcagtaa ctgtggtaga gggtcaagtt gactattatg gtttatatta tgttcatgaa   3240
   ggaatacgaa catattttgt gcagtttaaa gatgatgcag aaaaatatag taaaaataaa   3300
   gtatgggaag ttcatgcggg tggtcaggta atattatgtc ctacatctgt gtttagcagc   3360
   aacgaagtat cctctcctga aattattagg cagcacttgg ccaaccaccc cgccgcgacc   3420
   cataccaaag ccgtcgcctt gggcaccgaa gaaacacaga cgactatcca gcgaccaaga   3480
   tcagagccag acaccggaaa cccctgccac accactaagt tgttgcacag agactcagtg   3540
   gacagtgctc caatcctcac tgcatttaac agctcacaca aaggacggat taactgtaat   3600
   agtaacacta cacccatagt acatttaaaa ggtgatgcta atactttaaa atgtttaaga   3660
   tatagattta aaaagcattg tacattgtat actgcagtgt cgtctacatg gcattggaca   3720
   ggacataatg taaaacataa aagtgcaatt gttacactta catatgatag tgaatggcaa   3780
   cgtgaccaat ttttgtctca agttaaaata ccaaaaacta ttacagtgtc tactggattt   3840
   atgtctatat gacaaatctt gatactgcat ccacaacatt actggcgtgc tttttgcttt   3900
   gctttgtgtg cttttgtgtg tctgcctatt aatacgtccg ctgcttttgt ctgtgtctac   3960
   atacacatca ttaataatat tggtattact attgtggata acagcagcct ctgcgtttag   4020
   gtgttttatt gtatatatta tatttgttta tataccatta tttttaatac atacacatgc   4080
   acgcttttta attacataat gtatatgtac ataatgtaat tgttacatat aattgttgta   4140
   taccataact tactattttt tcttttttat tttcatatat aatttttttt tttgtttgtt   4200
   tgtttgtttt ttaataaact gttattactt aacaatgcga cacaaacgtt ctgcaaaacg   4260
   cacaaaacgt gcatcggcta cccaacttta taaaacatgc aaacaggcag gtacatgtcc   4320
   acctgacatt atacctaagg ttgaaggcaa aactattgct gaacaaatat tacaatatgg   4380
   aagtatgggt gtattttttg gtgggttagg aattggaaca gggtcgggta caggcggacg   4440
   cactgggtat attccattgg gaacaaggcc tcccacagct acagatacac ttgctcctgt   4500
   aagaccccct ttaacagtag atcctgtggg cccttctgat ccttctatag tttctttagt   4560
   ggaagaaact agttttattg atgctggtgc accaacatct gtaccttcca ttcccccaga   4620
   tgtatcagga tttagtatta ctacttcaac tgataccaca cctgctatat tagatattaa   4680
   taatactgtt actactgtta ctacacataa taatcccact ttcactgacc catctgtatt   4740
   gcagcctcca acacctgcag aaactggagg gcattttaca ctttcatcat ccactattag   4800
   tacacataat tatgaagaaa ttcctatgga tacatttatt gttagcacaa accctaacac   4860
   agtaactagt agcacaccca taccagggtc tcgcccagtg gcacgcctag gattatatag   4920
   tcgcacaaca caacaggtta aagttgtaga ccctgctttt gtaaccactc ccactaaact   4980
   tattacatat gataatcctg catatgaagg tatagatgtg gataatacat tatatttttc   5040
   tagtaatgat aatagtatta atatagctcc agatcctgac tttttggata tagttgcttt   5100
   acataggcca gcattaacct ctaggcgtac tggcattagg tacagtagaa ttggtaataa   5160
   acaaacacta cgtactcgta gtggaaaatc tataggtgct aaggtacatt attattatga   5220
   tttaagtact attgatcctg cagaagaaat agaattacaa actataacac cttctacata   5280
   tactaccact tcacatgcag cctcacctac ttctattaat aatggattat atgatattta   5340
   tgcagatgac tttattacag atacttctac aaccccggta ccatctgtac cctctacatc   5400
   tttatcaggt tatattcctg caaatacaac aattcctttt ggtggtgcat acaatattcc   5460
   tttagtatca ggtcctgata tacccattaa tataactgac caagctcctt cattaattcc   5520
   tatagttcca gggtctccac aatatacaat tattgctgat gcaggtgact tttatttaca   5580
   tcctagttat tacatgttac gaaaacgacg taaacgttta ccatattttt tttcagatgt   5640
   ctctttggct gcctagtgag gccactgtct acttgcctcc tgtcccagta tctaaggttg   5700
   taagcacgga tgaatatgtt gcacgcacaa acatatatta tcatgcagga acatccagac   5760
   tacttgcagt tggacatccc tattttccta ttaaaaaacc taacaataac aaaatattag   5820
   ttcctaaagt atcaggatta caatacaggg tatttagaat acatttacct gaccccaata   5880
   agtttggttt tcctgacacc tcattttata atccagatac acagcggctg gtttgggcct   5940
   gtgtaggtgt tgaggtaggt cgtggtcagc cattaggtgt gggcattagt ggccatcctt   6000
   tattaaataa attggatgac acagaaaatg ctagtgctta tgcagcaaat gcaggtgtgg   6060
   ataatagaga atgtatatct atggattaca aacaaacaca attgtgttta attggttgca   6120
   aaccacctat aggggaacac tggggcaaag gatccccatg taccaatgtt gcagtaaatc   6180
   caggtgattg tccaccatta gagttaataa acacagttat tcaggatggt gatatggttc   6240
   atactggctt tggtgctatg gactttacta cattacaggc taacaaaagt gaagttccac   6300
   tggatatttg tacatctatt tgcaaatatc cagattatat taaaatggtg tcagaaccat   6360
   atggcgacag cttatttttt tatttacgaa gggaacaaat gtttgttaga catttattta   6420
   atagggctgg tactgttggt gaaaatgtac cagacgattt atacattaaa ggctctgggt   6480
   ctactgcaaa tttagccagt tcaaattatt ttcctacacc tagtggttct atggttacct   6540
   ctgatgccca aatattcaat aaaccttatt ggttacaacg agcacagggc cacaataatg   6600
   gcatttgttg gggtaaccaa ctatttgtta ctgttgttga tactacacgc agtacaaata   6660
   tgtcattatg tgctgccata tctacttcag aaactacata taaaaatact aactttaagg   6720
   agtacctacg acatggggag gaatatgatt tacagtttat ttttcaactg tgcaaaataa   6780
   ccttaactgc agacgttatg acatacatac attctatgaa ttccactatt ttggaggact   6840
   ggaattttgg tctacaacct cccccaggag gcacactaga agatacttat aggtttgtaa   6900
   cccaggcaat tgcttgtcaa aaacatacac ctccagcacc taaagaagat gatcccctta   6960
   aaaaatacac tttttgggaa gtaaatttaa aggaaaagtt ttctgcagac ctagatcagt   7020
   ttcctttagg acgcaaattt ttactacaag caggattgaa ggccaaacca aaatttacat   7080
   taggaaaacg aaaagctaca cccaccacct catctacctc tacaactgct aaacgcaaaa   7140
   aacgtaagct gtaagtattg tatgtatgtt gaattagtgt tgtttgttgt gtatatgttt   7200
   gtatgtgctt gtatgtgctt gtaaatatta agttgtatgt gtgtttgtat gtatggtata   7260
   ataaacacgt gtgtatgtgt ttttaaatgc ttgtgtaact attgtgtcat gcaacataaa   7320
   taaacttatt gtttcaacac ctactaattg tgttgtggtt attcattgta tataaactat   7380
   atttgctaca tcctgttttt gttttatata tactatattt tgtagcgcca ggcccatttt   7440
   gtagcttcaa ccgaattcgg ttgcatgctt tttggcacaa aatgtgtttt tttaaatagt   7500
   tctatgtcag caactatggt ttaaacttgt acgtttcctg cttgccatgc gtgccaaatc   7560
   cctgttttcc tgacctgcac tgcttgccaa ccattccatt gttttttaca ctgcactatg   7620
   tgcaactact gaatcactat gtacattgtg tcatataaaa taaatcacta tgcgccaacg   7680
   ccttacatac cgctgttagg cacatatttt tggcttgttt taactaacct aattgcatat   7740
   ttggcataag gtttaaactt ctaaggccaa ctaaatgtca ccctagttca tacatgaact   7800
   gtgtaaaggt tagtcataca ttgttcattt gtaaaactgc acatgggtgt gtgcaaaccg   7860
   attttgggtt acacatttac aagcaactta tataataata ctaa                    7904
<210> 3
   <211> 22
   <212> DNA
   <213> Homo sapiens
<220>
   <221> misc_feature
   <222> (1)..(20)
   <223> brin sens de PML-rare
<220>
   <221> misc_feature
   <222> (21)..(22)
   <223> residus thymine ajoutés
<400> 3
   caugucaugu gucacaucuc tt 22
<210> 4
   <211> 22
   <212> DNA
   <213> Homo sapiens
<220>
   <221> misc_feature
   <222> (1)..(20)
   <223> brin anti-sens de PML-rare
<220>
   <221> misc_feature
   <222> (21)..(22)
   <223> residus thimine ajoutés
<400> 4
   gagaugugac acaugacaug tt 22
<210> 5
   <211> 22
   <212> DNA
   <213> Homo sapiens
<220>
   <221> misc_feature
   <222> (1)..(20)
   <223> brin sens PLM-rare
<220>
   <221> misc_feature
   <222> (21)..(22)
   <223> residus thimine ajoutés
<400> 5
   ggggaggcag ccauugagac tt 22
<210> 6
   <211> 22
   <212> DNA
   <213> Homo sapiens
<220>
   <221> misc_feature
   <222> (1)..(20)
   <223> brin anti-sens PML-rare
<220>
   <221> misc_feature
   <222> (21)..(22)
   <223> Résidus thimine ajoutés
<400> 6
   gucucaaugg cugccucccc tt 22
<210> 7
   <211> 23
   <212> DNA
   <213> Homo sapiens
<220>
   <221> misc_feature
   <222> (1)..(21)
   <223> séquence issue du VGEF humain
<220>
   <221> misc_feature
   <222> (22)..(23)
   <223> résidus thimine ajoutés
<400> 7
   augugaaugc agaccaaaga att 23
<210> 8
   <211> 23
   <212> DNA
   <213> Homo sapiens
<220>
   <221> misc_feature
   <222> (1)..(21)
   <223> séquence issue du VGEF humain
<220>
   <221> misc_feature <222> (22)..(23)
   <223> residus thimine ajoutés
<400> 8
   uucuuugguc ugcauucaca utt 23
<210> 9
   <211> 22
   <212> DNA
   <213> Homo sapiens
<220>
   <221> misc_feature
   <222> (1)..(20)
   <223> séquence issue du VEGF humain
<220>
   <221> misc_feature
   <222> (21)..(22)
   <223> résidus thimine ajoutés
<400> 9
   caugucaugu gucacaucuc tt 22
<210> 10
   <211> 22
   <212> DNA
   <213> Homo sapiens
<220>
   <221> misc_feature
   <222> (1)..(20)
   <223> séquence issue du VEGF humain
<220>
   <221> misc_feature
   <222> (21)..(22)
   <223> résidus thimine ajoutés
<400> 10
   gagaugugac acaugacaug tt 22
<210> 11
   <211> 23
   <212> DNA
   <213> Homo sapiens
<220>
   <221> misc_feature
   <222> (1)..(21)
   <223> séquence issue du HIF 1 alpha humain
<220>
   <221> misc_feature
   <222> (22)..(23)
   <223> résidus thimine ajoutés
<400> 11
   caugugacca ugaggaaaug att 23
<210> 12
   <211> 23
   <212> DNA
   <213> Homo sapiens
<220>
   <221> misc_feature
   <222> (1)..(21)
   <223> séquence issue du HIF 1 alpha humain
<220>
   <221> misc_feature
   <222> (22)..(23)
   <223> résidus Thimine ajoutés
<400> 12
   ucauuuccuc auggucacau gtt 23
<210> 13
   <211> 23
   <212> DNA
   <213> Homo sapiens
<220>
   <221> misc_feature
   <222> (1)..(21)
   <223> séquence issue du HIF 1 alpha humain
<220>
   <221> misc_feature
   <222> (22)..(23)
   <223> résidus thimine ajoutés
<400> 13
   gauagcaaug acgaaugcgu att 23
<210> 14
   <211> 23
   <212> DNA
   <213> Homo sapiens
<220>
   <221> misc_feature
   <222> (1)..(21)
   <223> Séquence issue du HIF 1 alpha humain
<220>
   <221> misc_feature
   <222> (22)..(23)
   <223> Séquence issue du HIF 1 alpha humain
<400> 14
   uacgcauucg ucauugcuau ctt 23
<210> 15
   <211> 23
   <212> DNA
   <213> Homo sapiens
<220>
   <221> misc_feature
   <222> (1)..(21)
   <223> séquence issue du récepteur aux androgènes humain
<220>
   <221> misc_feature
   <222> (22)..(23)
   <223> residus thimine ajoutés
<400> 15
   gacucagcug ccccauccac gtt 23
<210> 16
   <211> 23
   <212> DNA
   <213> Homo sapiens
<220>
   <221> misc_feature
   <222> (1)..(21)
   <223> séquence issue du HIF 1 alpha humain
<220>
   <221> misc_feature
   <222> (22)..(23)
   <223> résidus thimine ajoutés
<400> 16
   cguggauggg gcagcugagu ctt 23
<210> 17
   <211> 23
   <212> DNA
   <213> Homo sapiens
<220>
   <221> misc_feature
   <222> (1)..(21)
   <223> séquence issue du HIF 1 alpha humain
<220>
   <221> misc_feature
   <222> (22)..(23)
   <223> résidus thimine ajoutés
<400> 17
   gauagcaaug acgaaugcgu att 23
<210> 18
   <211> 23
   <212> DNA
   <213> Homo sapiens
<220>
   <221> misc_feature
   <222> (1)..(21)
   <223> séquence issue du HIF 1alpha humain
<220>
   <221> misc_feature
   <222> (22)..(23)
   <223> residus thimine ajoutés
<400> 18
   uacgcauucg ucauugcuau ctt 23
<210> 19
   <211> 21
   <212> DNA
   <213> Homo sapiens
<220>
   <221> misc_feature
   <222> (1)..(19)
   <223> Séquence issue du récepteur aux androgènes portant la mutation T8 77A
<220>
   <221> misc_feature
   <222> (20)..(21)
   <223> residus thimine ajoutés
<400> 19
   gcaucaguuc gcuuuugact t 21
<210> 20
   <211> 21
   <212> DNA
   <213> Homo sapiens
<220>
   <221> misc_feature
   <222> (1)..(19)
   <223> séquence issue du récepteur aux androgènes portant la mutation T8 77A
<220>
   <221> misc_feature
   <222> (20)..(21)
   <223> résidus thimine ajoutés
<400> 20
   gucaaaagcg aacugaugct t 21
<210> 21
   <211> 21
   <212> DNA
   <213> Homo sapiens
<220>
   <221> misc_feature
   <222> (1)..(19)
   <223> séquence issue du p53 humain sauvage (sens)
<220>
   <221> misc_feature
   <222> (20)..(21)
   <223> residus thimine ajoutés
<400> 21
   gcaugaaccg gaggcccaut t 21
<210> 22
   <211> 21
   <212> DNA
   <213> Homo sapiens
<220>
   <221> misc_feature
   <222> (1)..(19)
   <223> séquence issue du gène p53 humain sauvage (antisens)
<220>
   <221> misc_feature
   <222> (20)..(21)
   <223> résidus thimine ajoutés
<400> 22
   augggccucc gguucaugct t 21
<210> 23
   <211> 21
   <212> DNA
   <213> Homo sapiens
<220>
   <221> misc_feature
   <222> (1)..(19)
   <223> séquence issue du p53 humain muté portant la mutation MT1 (r248w) (sens)
<220>
   <221> misc_feature
   <222> (20)..(21)
   <223> residus thimine ajoutés
<400> 23
   gcaugaacug gaggcccaut t 21
<210> 24
   <211> 21
   <212> DNA
   <213> Homo sapiens
<220>
   <221> misc_feature
   <222> (1)..(19)
   <223> séquence issue du p53 humain muté portant la mutation MT1 (r248w) (antisens)
<220>
   <221> misc_feature <222> (20)..(21)
   <223> résidus thimine ajoutés
<400> 24
   augggccucc aguucaugct t 21
<210> 25
   <211> 21
   <212> DNA
   <213> Homo sapiens
<220>
   <221> misc_feature <222> (1)..(19)
   <223> séquence issue du p53 humain muté portant la mutation MT2 (r248w) (sens)
<220>
   <221> misc_feature
   <222> (20)..(21)
   <223> résidus thimine ajoutés
<400> 25
   ucaugaacug gaggcccaut t 21
<210> 26
   <211> 21
   <212> DNA
   <213> Homo sapiens
<220>
   <221> misc_feature <222> (1)..(19)
   <223> séquence issue du p53 humain muté portant la mutation MT2 (r248w) (antisens)
<220>
   <221> misc_feature
   <222> (20)..(21)
   <223> résidus thimine ajoutés
<400> 26
   augggccucc aguucaugat t 21
<210> 27
   <211> 21
   <212> DNA
   <213> Homo sapiens
<220>
   <221> misc_feature
   <222> (1)..(19)
   <223> séquence issue du E6 de HPV (sens)
<220>
   <221> misc_feature
   <222> (20)..(21)
   <223> réidus thimine ajoutés
<400> 27
   ccacaguuau gcacagagct t 21
<210> 28
   <211> 20
   <212> DNA
   <213> Homo sapiens
<220>
   <221> misc_feature
   <222> (1)..(18)
   <223> séquence issue du E6 de HPV (antisens)
<220>
   <221> misc_feature
   <222> (19)..(20)
   <223> résidus thimine ajoutés
<400> 28
   gcucugugca uaacuuggtt 20
<210> 29
   <211> 22
   <212> DNA
   <213> Homo sapiens
<220>
   <221> misc_feature
   <222> (1)..(22)
   <223> séquence issue du gène codant la GFP (brin sens)
<400> 29
   gcaagctgac cctgaagttc at 22
<210> 30
   <211> 22
   <212> DNA
   <213> Homo sapiens
<220>
   <221> misc_feature
   <222> (1)..(22)
   <223> séquence issue du gène codant la GFP (brin anti-sens)
<400> 30
   gaacuucagg gucagcuugc cg 22
<210> 31
   <211> 22
   <212> DNA
   <213> Homo sapiens
<220>
   <221> misc_feature
   <222> (1)..(20)
   <223> séquence issue du gène codant la GFP (brin sens)
<220>
   <221> misc_feature
   <222> (21)..(22)
   <223> résidus thimine ajoutés
<400> 31
   caugucaugu gucacaucuc tt 22
<210> 32
   <211> 22
   <212> DNA
   <213> Homo sapiens
<220>
   <221> misc_feature
   <222> (1)..(20)
   <223> séquence issue du gène codant la GFP (brin antisens)
<220>
   <221> misc_feature
   <222> (21)..(22)
   <223> résidus thimine ajoutés
<400> 32
   gagaugugac acaugacaug tt 22
<210> 33
   <211> 21
   <212> DNA
   <213> Homo sapiens
<220>
   <221> misc_feature
   <222> (1)..(21)
   <223> séquence issue du récepteur aux androgènes humain muté (brin sen s)
<400> 33
   gcatcagttc gcttttgact t 21
<210> 34
   <211> 21
   <212> DNA
   <213> Homo sapiens
<220>
   <221> misc_feature
   <222> (1)..(19)
   <223> séquence issue du récepteur aux androgènes humain muté (brin sen s)
<220>
   <221> misc_feature
   <222> (20)..(21)
   <223> résidus thimine ajoutés
<400> 34
   gcaucaguuc gcuuuugact t 21
<210> 35
   <211> 21
   <212> DNA
   <213> Homo sapiens
<220>
   <221> misc_feature
   <222> (1)..(21)
   <223> séquence issue du récepteur aux androgènes humain muté (brin sen s)
<400> 35
   gtcaaaagcg aactgatgct t 21
<210> 36
   <211> 21
   <212> DNA
   <213> Homo sapiens
<220>
   <221> misc_feature
   <222> (1)..(19)
   <223> séquence issue du récepteur aux androgènes humain muté (brin sen s)
<220>
   <221> misc_feature <222> (20)..(21)
   <223> residus thimine ajoutés
<400> 36
   gucaaaagcg aacugaugct t 21
<210> 37
   <211> 21
   <212> DNA
   <213> Homo sapiens
<220>
   <221> misc_feature
   <222> (1)..(19)
   <223> séquence issue du récepteur aux androgènes humain muté (brin sen s)
<220>
   <221> misc_feature
   <222> (20)..(21)
   <223> résidus thimine ajoutés
<400> 37
   guucggucug cuuacacuat t 21
<210> 38
   <211> 21
   <212> DNA
   <213> Homo sapiens
<220>
   <221> misc_feature
   <222> (1)..(19)
   <223> séquence issue du récepteur aux androgènes humain muté (brin ant isens)
<220>
   <221> misc_feature
   <222> (20)..(21)
   <223> résidus thimine ajoutés
<400> 38
   uaguguaagc agaccgaact t 21
<210> 39
   <211> 21
   <212> DNA
   <213> Homo sapiens
<220>
   <221> misc_feature
   <222> (1)..(19)
   <223> séquence issue du gène p53 humain sauvage (brin sens)
<220>
   <221> misc_feature
   <222> (20)..(21)
   <223> residus thimine ajoutés
<400> 39
   gcaugaaccg gaggcccaut t 21
<210> 40
   <211> 21
   <212> DNA
   <213> Homo sapiens
<220>
   <221> misc_feature
   <222> (1)..(19)
   <223> séquence issue du gène p53 humain sauvage (brin antisens)
<220>
   <221> misc_feature
   <222> (20)..(21)
   <223> résidus thimine ajoutés
<400> 40
   augggccucc gguucaugct t 21
<210> 41
   <211> 21
   <212> DNA
   <213> Homo sapiens
<220>
   <221> misc_feature
   <222> (1)..(19)
   <223> séquence issue du gène p53 humain muté (brin sens)
<220>
   <221> misc_feature
   <222> (20)..(21)
   <223> résidus thimine ajoutés
<400> 41
   gcaugaaccg gaggcccaut t 21
<210> 42
   <211> 21
   <212> DNA
   <213> Homo sapiens
<220>
   <221> misc_feature
   <222> (1)..(19)
   <223> séquence issue du gène p53 humain sauvage (brin antisens)
<220>
   <221> misc_feature
   <222> (20)..(21)
   <223> résidus thimine ajoutés
<400> 42
   augggccucc gguucaugct t 21
<210> 43
   <211> 21
   <212> DNA
   <213> Homo sapiens
<220>
   <221> misc_feature
   <222> (1)..(19)
   <223> séquence issue du gène p53 muté (brin sens)
<220>
   <221> misc_feature
   <222> (20)..(21)
   <223> résidus thimine ajoutés
<400> 43
   gcaugaaccg gaggcccaut t 21
<210> 44
   <211> 21
   <212> DNA
   <213> Homo sapiens
<220>
   <221> misc_feature
   <222> (1)..(19)
   <223> séquence issu du gène p53 humain muté (brin antisens)
<220>
   <221> misc_feature
   <222> (20)..(21)
   <223> résidus thimine ajoutés
<400> 44
   augggccucc gguucaugct t 21
<210> 45
   <211> 21
   <212> DNA
   <213> Homo sapiens
<220>
   <221> misc_feature
   <222> (1)..(19)
   <223> séquence issue du gène p53 muté (brin sens)
<220>
   <221> misc_feature
   <222> (20)..(21)
   <223> résidus thimine ajoutés
<400> 45
   gcatgaaccg gaggcccatt t 21
<210> 46
   <211> 21
   <212> DNA
   <213> Homo sapiens
<220>
   <221> misc_feature
   <222> (1)..(19)
   <223> séquence issue du gène p53 humain muté (brin antisens)
<220>
   <221> misc_feature
   <222> (20)..(21)
   <223> résidus thimine ajoutés
<400> 46
   augggccucc gguucaugct t 21
<210> 47
   <211> 21
   <212> DNA
   <213> Homo sapiens
<220>
   <221> misc_feature
   <222> (1)..(19)
   <223> séquence issue du gène p53 humain muté (brin sens)
<220>
   <221> misc_feature
   <222> (20)..(21)
   <223> résidus thimine ajoutés
<400> 47
   gcaugaaccg gaggcccaut t 21
<210> 48
   <211> 22
   <212> DNA
   <213> Homo sapiens
<220>
   <221> misc_feature
   <222> (1)..(20)
   <223> séquence issue du gène p53 humain muté (brin antisens)
<220>
   <221> misc_feature
   <222> (21)..(22)
   <223> résidus thimine ajoutés
<400> 48
   atgggccutc cggttcatgc tt 22
<210> 49
   <211> 21
   <212> DNA
   <213> Homo sapiens
<220>
   <221> misc_feature
   <222> (1)..(19)
   <223> séquence issue du gène p53 humain muté (brin sens)
<220>
   <221> misc_feature
   <222> (20)..(21)
   <223> résidus thimine ajoutés
<400> 49
   gcaugaacug gaggcccaut t 21
<210> 50
   <211> 21
   <212> DNA
   <213> Homo sapiens
<220>
   <221> misc_feature
   <222> (1)..(19)
   <223> séquence issue du gène p53 humain muté (brin antisens)
<220>
   <221> misc_feature
   <222> (20)..(21)
   <223> résidus thimine ajoutés
<400> 50
   augggccucc aguucaugct t 21
<210> 51
   <211> 21
   <212> DNA
   <213> Homo sapiens
<220>
   <221> misc_feature
   <222> (1)..(19)
   <223> séquence issue du gène p53 humain muté (brin sens)
<220>
   <221> misc_feature
   <222> (20)..(21)
   <223> résidus thimine ajoutés
<400> 51
   gcaugaacug gaggcccaut t 21
<210> 52
   <211> 21
   <212> DNA
   <213> Homo sapiens
<220>
   <221> misc_feature
   <222> (1)..(19)
   <223> séquence issue du gène p53 humain muté (brin antisens)
<220>
   <221> misc_feature
   <222> (20)..(21)
   <223> résidus thimine ajoutés
<400> 52
   augggccucc aguucaugct t 21
<210> 53
   <211> 21
   <212> DNA
   <213> Homo sapiens
<220>
   <221> misc_feature
   <222> (1)..(19)
   <223> séquence issue du gène p53 humain muté (brin isens)
<220>
   <221> misc_feature
   <222> (20)..(21)
   <223> résidus thimine ajoutés
<400> 53
   gcaugaacug gaggcccaut t 21
<210> 54
   <211> 21
   <212> DNA
   <213> Homo sapiens
<220>
   <221> misc_feature
   <222> (1)..(19)
   <223> séquence issue du gène p53 humain muté (brin antisens)
<220>
   <221> misc_feature
   <222> (20)..(21)
   <223> résidus thimine ajoutés
<400> 54
   augggccucc aguucaugct t 21
<210> 55
   <211> 21
   <212> DNA
   <213> Homo sapiens
<220>
   <221> misc_feature
   <222> (1)..(19)
   <223> séquence issue du gène p53 humain muté (brin sens)
<220>
   <221> misc_feature
   <222> (20)..(21)
   <223> résidus thimine ajoutés
<400> 55
   gcatgaactg gaggcccatt t 21
<210> 56
   <211> 21
   <212> DNA
   <213> Homo sapiens
<220>
   <221> misc_feature
   <222> (1)..(19)
   <223> séquence issue du gène p53 humain muté (brin antisens)
<220>
   <221> misc_feature
   <222> (20)..(21)
   <223> résidus thimine ajoutés
<400> 56
   augggccucc aguucaugct t 21
<210> 57
   <211> 21
   <212> DNA
   <213> Homo sapiens
<220>
   <221> misc feature
   <222> (1)..(19)
   <223> séquence issue du gène p53 humain muté (brin sens)
<220>
   <221> misc_feature
   <222> (20)..(21)
   <223> résidus thimine ajoutés
<400> 57
   gcatgaactg gaggcccatt t 21
<210> 58
   <211> 21
   <212> DNA
   <213> Homo sapiens
<220>
   <221> misc_feature
   <222> (1)..(19)
   <223> séquence issue du gène p53 humain muté (brin antisens)
<220>
   <221> misc_feature
   <222> (20)..(21)
   <223> résidus thimine ajoutés
<400> 58
   augggccucc aguucaugct t 21
<210> 59
   <211> 3933
   <212> DNA
   <213> Homo sapiens
<220>
   <221> misc_feature
   <222> (1)..(3933)
   <223> Homo sapiens hypoxia-inducible factor 1 sous-unité alpha. (HIF-1 alpha)
<400> 59
   cacgaggcag cactctcttc gtcgcttcgg ccagtgtgtc gggctgggcc ctgacaagcc     60
   acctgaggag aggctcggag ccgggcccgg accccggcga ttgccgcccg cttctctcta    120
   gtctcacgag gggtttcccg cctcgcaccc ccacctctgg acttgccttt ccttctcttc    180
   tccgcgtgtg gagggagcca gcgcttaggc cggagcgagc ctgggggccg cccgccgtga    240
   agacatcgcg gggaccgatt caccatggag ggcgccggcg gcgcgaacga caagaaaaag    300
   ataagttctg aacgtcgaaa agaaaagtct cgagatgcag ccagatctcg gcgaagtaaa    360
   gaatctgaag ttttttatga gcttgctcat cagttgccac ttccacataa tgtgagttcg    420
   catcttgata aggcctctgt gatgaggctt accatcagct atttgcgtgt gaggaaactt    480
   ctggatgctg gtgatttgga tattgaagat gacatgaaag cacagatgaa ttgcttttat    540
   ttgaaagcct tggatggttt tgttatggtt ctcacagatg atggtgacat gatttacatt    600
   tctgataatg tgaacaaata catgggatta actcagtttg aactaactgg acacagtgtg    660
   tttgatttta ctcatccatg tgaccatgag gaaatgagag aaatgcttac acacagaaat    720
   ggccttgtga aaaagggtaa agaacaaaac acacagcgaa gcttttttct cagaatgaag    780
   tgtaccctaa ctagccgagg aagaactatg aacataaagt ctgcaacatg gaaggtattg    840
   cactgcacag gccacattca cgtatatgat accaacagta accaacctca gtgtgggtat    900
   aagaaaccac ctatgacctg cttggtgctg atttgtgaac ccattcctca cccatcaaat    960
   attgaaattc ctttagatag caagactttc ctcagtcgac acagcctgga tatgaaattt   1020
   tcttattgtg atgaaagaat taccgaattg atgggatatg agccagaaga acttttaggc   1080
   cgctcaattt atgaatatta tcatgctttg gactctgatc atctgaccaa aactcatcat   1140
   gatatgttta ctaaaggaca agtcaccaca ggacagtaca ggatgcttgc caaaagaggt   1200
   ggatatgtct gggttgaaac tcaagcaact gtcatatata acaccaagaa ttctcaacca   1260
   cagtgcattg tatgtgtgaa ttacgttgtg agtggtatta ttcagcacga cttgattttc   1320
   tcccttcaac aaacagaatg tgtccttaaa ccggttgaat cttcagatat gaaaatgact   1380
   cagctattca ccaaagttga atcagaagat acaagtagcc tctttgacaa acttaagaag   1440
   gaacctgatg ctttaacttt gctggcccca gccgctggag acacaatcat atctttagat   1500
   tttggcagca acgacacaga aactgatgac cagcaacttg aggaagtacc attatataat   1560
   gatgtaatgc tcccctcacc caacgaaaaa ttacagaata taaatttggc aatgtctcca   1620
   ttacccaccg ctgaaacgcc aaagccactt cgaagtagtg ctgaccctgc actcaatcaa   1680
   gaagttgcat taaaattaga accaaatcca gagtcactgg aactttcttt taccatgccc   1740
   cagattcagg atcagacacc tagtccttcc gatggaagca ctagacaaag ttcacctgag   1800
   cctaatagtc ccagtgaata ttgtttttat gtggatagtg atatggtcaa tgaattcaag   1860
   ttggaattgg tagaaaaact ttttgctgaa gacacagaag caaagaaccc attttctact   1920
   caggacacag atttagactt ggagatgtta gctccctata tcccaatgga tgatgacttc   1980
   cagttacgtt ccttcgatca gttgtcacca ttagaaagca gttccgcaag ccctgaaagc   2040
   gcaagtcctc aaagcacagt tacagtattc cagcagactc aaatacaaga acctactgct   2100
   aatgccacca ctaccactgc caccactgat gaattaaaaa cagtgacaaa agaccgtatg   2160
   gaagacatta aaatattgat tgcatctcca tctcctaccc acatacataa agaaactact   2220
   agtgccacat catcaccata tagagatact caaagtcgga cagcctcacc aaacagagca   2280
   ggaaaaggag tcatagaaca gacagaaaaa tctcatccaa gaagccctaa cgtgttatct   2340
   gtcgctttga gtcaaagaac tacagttcct gaggaagaac taaatccaaa gatactagct   2400
   ttgcagaatg ctcagagaaa gcgaaaaatg gaacatgatg gttcactttt tcaagcagta   2460
   ggaattggaa cattattaca gcagccagac gatcatgcag ctactacatc actttcttgg   2520
   aaacgtgtaa aaggatgcaa atctagtgaa cagaatggaa tggagcaaaa gacaattatt   2580
   ttaataccct ctgatttagc atgtagactg ctggggcaat caatggatga aagtggatta   2640
   ccacagctga ccagttatga ttgtgaagtt aatgctccta tacaaggcag cagaaaccta   2700
   ctgcagggtg aagaattact cagagctttg gatcaagtta actgagcttt ttcttaattt   2760
   cattcctttt tttggacact ggtggctcac tacctaaagc agtctattta tattttctac   2820
   atctaatttt agaagcctgg ctacaatact gcacaaactt ggttagttca atttttgatc   2880
   ccctttctac ttaatttaca ttaatgctct tttttagtat gttctttaat gctggatcac   2940
   agacagctca ttttctcagt tttttggtat ttaaaccatt gcattgcagt agcatcattt   3000
   taaaaaatgc acctttttat ttatttattt ttggctaggg agtttatccc tttttcgaat   3060
   tatttttaag aagatgccaa tataattttt gtaagaaggc agtaaccttt catcatgatc   3120
   ataggcagtt gaaaaatttt tacacctttt ttttcacatt ttacataaat aataatgctt   3180
   tgccagcagt acgtggtagc cacaattgca caatatattt tcttaaaaaa taccagcagt   3240
   tactcatgga atatattctg cgtttataaa actagttttt aagaagaaat tttttttggc   3300
   ctatgaaatt gttaaacctg gaacatgaca ttgttaatca tataataatg attcttaaat   3360
   gctgtatggt ttattattta aatgggtaaa gccatttaca taatatagaa agatatgcat   3420
   atatctagaa ggtatgtggc atttatttgg ataaaattct caattcagag aaatcatctg   3480
   atgtttctat agtcactttg ccagctcaaa agaaaacaat accctatgta gttgtggaag   3540
   tttatgctaa tattgtgtaa ctgatattaa acctaaatgt tctgcctacc ctgttggtat   3600
   aaagatattt tgagcagact gtaaacaaga aaaaaaaaat catgcattct tagcaaaatt   3660
   gcctagtatg ttaatttgct caaaatacaa tgtttgattt tatgcacttt gtcgctatta   3720
   acatcctttt tttcatgtag atttcaataa ttgagtaatt ttagaagcat tattttagga   3780
   atatatagtt gtcacagtaa atatcttgtt ttttctatgt acattgtaca aatttttcat   3840
   tccttttgct ctttgtggtt ggatctaaca ctaactgtat tgttttgtta catcaaataa   3900
   acatcttctg tggaaaaaaa aaaaaaaaaa aaa                                3933
<210> 60
   <211> 3166
   <212> DNA
   <213> Homo sapiens
<220>
   <221> misc_feature
   <222> (1)..(3166)
   <223> VEGF A humain
<400> 60
   aagagctcca gagagaagtc gaggaagaga gagacggggt cagagagagc gcgcgggcgt     60
   gcgagcagcg aaagcgacag gggcaaagtg agtgacctgc ttttgggggt gaccgccgga    120
   gcgcggcgtg agccctcccc cttgggatcc cgcagctgac cagtcgcgct gacggacaga    180
   cagacagaca ccgcccccag ccccagttac cacctcctcc ccggccggcg gcggacagtg    240
   gacgcggcgg cgagccgcgg gcaggggccg gagcccgccc ccggaggcgg ggtggagggg    300
   gtcggagctc gcggcgtcgc actgaaactt ttcgtccaac ttctgggctg ttctcgcttc    360
   ggaggagccg tggtccgcgc gggggaagcc gagccgagcg gagccgcgag aagtgctagc    420
   tcgggccggg aggagccgca gccggaggag ggggaggagg aagaagagaa ggaagaggag    480
   agggggccgc agtggcgact cggcgctcgg aagccgggct catggacggg tgaggcggcg    540
   gtgtgcgcag acagtgctcc agcgcgcgcg ctccccagcc ctggcccggc ctcgggccgg    600
   gaggaagagt agctcgccga ggcgccgagg agagcgggcc gccccacagc ccgagccgga    660
   gagggacgcg agccgcgcgc cccggtcggg cctccgaaac catgaacttt ctgctgtctt    720
   gggtgcattg gagccttgcc ttgctgctct acctccacca tgccaagtgg tcccaggctg    780
   cacccatggc agaaggagga gggcagaatc atcacgaagt ggtgaagttc atggatgtct    840
   atcagcgcag ctactgccat ccaatcgaga ccctggtgga catcttccag gagtaccctg    900
   atgagatcga gtacatcttc aagccatcct gtgtgcccct gatgcgatgc gggggctgct    960
   ccaatgacga gggcctggag tgtgtgccca ctgaggagtc caacatcacc atgcagatta   1020
   tgcggatcaa acctcaccaa ggccagcaca taggagagat gagcttccta cagcacaaca   1080
   aatgtgaatg cagaccaaag aaagatagag caagacaaga aaatccctgt gggccttgct   1140
   cagagcggag aaagcatttg tttgtacaag atccgcagac gtgtaaatgt tcctgcaaaa   1200
   acacacactc gcgttgcaag gcgaggcagc ttgagttaaa cgaacgtact tgcagatgtg   1260
   acaagccgag gcggtgagcc gggcaggagg aaggagcctc cctcagggtt tcgggaacca   1320
   gatctctctc caggaaagac tgatacagaa cgatcgatac agaaaccacg ctgccgccac   1380
   cacaccatca ccatcgacag aacagtcctt aatccagaaa cctgaaatga aggaagagga   1440
   gactctgcgc agagcacttt gggtccggag ggcgagactc cggcggaagc attcccgggc   1500
   gggtgaccca gcacggtccc tcttggaatt ggattcgcca ttttattttt cttgctgcta   1560
   aatcaccgag cccggaagat tagagagttt tatttctggg attcctgtag acacacccac   1620
   ccacatacat acatttatat atatatatat tatatatata taaaaataaa tatctctatt   1680
   ttatatatat aaaatatata tattcttttt ttaaattaac agtgctaatg ttattggtgt   1740
   cttcactgga tgtatttgac tgctgtggac ttgagttggg aggggaatgt tcccactcag   1800
   atcctgacag ggaagaggag gagatgagag actctggcat gatctttttt ttgtcccact   1860
   tggtggggcc agggtcctct cccctgccca agaatgtgca aggccagggc atgggggcaa   1920
   atatgaccca gttttgggaa caccgacaaa cccagccctg gcgctgagcc tctctacccc   1980
   aggtcagacg gacagaaaga caaatcacag gttccgggat gaggacaccg gctctgacca   2040
   ggagtttggg gagcttcagg acattgctgt gctttgggga ttccctccac atgctgcacg   2100
   cgcatctcgc ccccaggggc actgcctgga agattcagga gcctgggcgg ccttcgctta   2160
   ctctcacctg cttctgagtt gcccaggagg ccactggcag atgtcccggc gaagagaaga   2220
   gacacattgt tggaagaagc agcccatgac agcgcccctt cctgggactc gccctcatcc   2280
   tcttcctgct ccccttcctg gggtgcagcc taaaaggacc tatgtcctca caccattgaa   2340
   accactagtt ctgtcccccc aggaaacctg gttgtgtgtg tgtgagtggt tgaccttcct   2400
   ccatcccctg gtccttccct tcccttcccg aggcacagag agacagggca ggatccacgt   2460
   gcccattgtg gaggcagaga aaagagaaag tgttttatat acggtactta tttaatatcc   2520
   ctttttaatt agaaattaga acagttaatt taattaaaga gtagggtttt ttttcagtat   2580
   tcttggttaa tatttaattt caactattta tgagatgtat cttttgctct ctcttgctct   2640
   cttatttgta ccggtttttg tatataaaat tcatgtttcc aatctctctc tccctgatcg   2700
   gtgacagtca ctagcttatc ttgaacagat atttaatttt gctaacactc agctctgccc   2760
   tccccgatcc cctggctccc cagcacacat tcctttgaaa gagggtttca atatacatct   2820
   acatactata tatatattgg gcaacttgta tttgtgtgta tatatatata tatatgttta   2880
   tgtatatatg tgatcctgaa aaaataaaca tcgctattct gttttttata tgttcaaacc   2940
   aaacaagaaa aaatagagaa ttctacatac taaatctctc tcctttttta attttaatat   3000
   ttgttatcat ttatttattg gtgctactgt ttatccgtaa taattgtggg gaaaagatat   3060
   taacatcacg tctttgtctc tagtgcagtt tttcgagata ttccgtagta catatttatt   3120
   tttaaacaac gacaaagaaa tacagatata tcttaaaaaa aaaaaa                  3166
<210> 61
   <211> 17
   <212> DNA
   <213> Homo sapiens
<220>
   <221> misc_feature
   <222> (1)..(17)
   <223> séquence issue du gène p53 humain sauvage
<400> 61
   gaggtgcgtg tttgtgc 17
<210> 62
   <211> 19
   <212> DNA
   <213> Homo sapiens
<220>
   <221> misc_feature
   <222> (1)..(19)
   <223> séquence issue du gène p53 humain sauvage
<400> 62
   gcatgaaccg gaggcccat 19
<210> 63
   <211> 19
   <212> DNA
   <213> Homo sapiens
<220>
   <221> misc_feature
   <222> (1)..(19)
   <223> séquence issue du gène p53 humain sauvage
<400> 63
   gcatgaaccg gaggcccat 19
<210> 64
   <211> 19
   <212> DNA
   <213> Homo sapiens
<220>
   <221> misc_feature
   <222> (1)..(19)
   <223> séquence issue du gène p53 humain sauvage
<400> 64
   gcatgaaccg gaggcccat 19
<210> 65
   <211> 19
   <212> DNA
   <213> Homo sapiens
<220>
   <221> misc_feature
   <222> (1)..(19)
   <223> séquence issue du gène p53 humain sauvage
<400> 65
   ctgcatgggc ggcatgaac 19
<210> 66
   <211> 19
   <212> DNA
   <213> Homo sapiens
<220>
   <221> misc_feature
   <222> (1)..(19)
   <223> séquence issue du gène p53 humain sauvage
<400> 66
   tgggagagac cggcgcaca 19
<210> 67
   <211> 19
   <212> DNA
   <213> Homo sapiens
<220>
   <221> misc_feature
   <222> (1)..(19)
   <223> séquence issue du gène p53 humain sauvage
<400> 67
   tgtgaggcac tgcccccac 19
<210> 68
   <211> 20
   <212> DNA
   <213> Homo sapiens
<220>
   <221> misc_feature
   <222> (1)..(20)
   <223> séquence issue du gène p53 humain sauvage
<400> 68
   taacagttcc tgcatgggcg 20
<210> 69
   <211> 17
   <212> DNA
   <213> Homo sapiens
<220>
   <221> misc_feature
   <222> (1)..(17)
   <223> séquence issue du gène p53 humain muté, portant la mutation r273h
<400> 69
   gaggtgcatg tttgtgc 17
<210> 70
   <211> 19
   <212> DNA
   <213> Homo sapiens
<220>
   <221> misc_feature
   <222> (1)..(19)
   <223> séquence issue du gène p53 humain muté, portant la mutation r248q
<400> 70
   gcatgaacca gaggcccat 19
<210> 71
   <211> 18
   <212> DNA
   <213> Homo sapiens
<220>
   <221> misc_feature
   <222> (1)..(18)
   <223> séquence issue du gène p53 humain muté, portant la mutation r248w <400> 71 gcatgaactg gaggccat 18
<210> 72
   <211> 19
   <212> DNA
   <213> Homo sapiens
<220>
   <221> misc_feature
   <222> (1)..(19)
   <223> séquence issue du gène p53 humain muté, portant la mutation r249s <400> 72 gcatgaaccg gagtcccat 19
<210> 73
   <211> 19
   <212> DNA
   <213> Homo sapiens
<220>
   <221> misc_feature
   <222> (1)..(19)
   <223> séquence issue du gène p53 humain muté, portant la mutation g245s <400> 73 ctgcatgggc agcatgaac 19
<210> 74
   <211> 19
   <212> DNA
   <213> Homo sapiens
<220>
   <221> misc_feature
   <222> (1)..(19)
   <223> séquence issue du gène p53 humain muté, portant la mutation r282w <400> 74 tgggagagac tggcgcaca 19
<210> 75
   <211> 19
   <212> DNA
   <213> Homo sapiens
<220>
   <221> misc_feature
   <222> (1)..(19)
   <223> séquence issue du gène p53 humain muté, portant la mutation r175h
<400> 75
   tgtgaggcgc tgcccccac 19
<210> 76
   <211> 20
   <212> DNA
   <213> Homo sapiens
<220>
   <221> misc_feature
   <222> (1)..(20)
   <223> séquence issue du gène p53 humain muté, portant la mutation c242s
<400> 76
   taacagttcc tccatgggcg 20
<210> 77
   <211> 3231
   <212> DNA
   <213> Homo sapiens
<220>
   <221> misc_feature
   <222> (1)..(3231)
   <223> séquence codant pour le récepteur aux androgènes humain.
<400> 77
   agctagctgc agcgactacc gcatcatcac agcctgttga actcttctga gcaagagaag     60
   gggaggcggg gtaagggaag taggtggaag attcagccaa gctcaaggat ggaagtgcag    120
   ttagggctgg gaagggtcta ccctcggccg ccgtccaaga cctaccgagg agctttccag    180
   aatctgttcc agagcgtccg cgaagtgatc cagaacccgg gccccaggca cccagaggcc    240
   gcgagcgcag cacctcccgg cgccagtttg ctgctgctgc agcagcagca gcagcagcag    300
   cagcagcagc agcagcagca gcagcaagag actagcccca ggcagcagca gcagcagcag    360
   ggtgaggatg gttctcccca agcccatcgt agaggcccca caggctacct ggtcctggat    420
   gaggaacagc aaccttcaca gccgcagtcg gccctggagt gccaccccga gagaggttgc    480
   gtcccagagc ctggagccgc cgtggccgcc agcaaggggc tgccgcagca gctgccagca    540
   cctccggacg aggatgactc agctgcccca tccacgttgt ccctgctggc ccccactttc    600
   cccggcttaa gcagctgctc cgctgacctt aaagacatcc tgagcgaggc cagcaccatg    660
   caactccttc agcaacagca gcaggaagca gtatccgaag gcagcagcag cgggagagcg    720
   agggaggcct cgggggctcc cacttcctcc aaggacaatt acttaggggg cacttcgacc    780
   atttctgaca acgccaagga gttgtgtaag gcagtgtcgg tgtccatggg cctgggtgtg    840
   gaggcgttgg agcatctgag tccaggggaa cagcttcggg gggattgcat gtacgcccca    900
   cttttgggag ttccacccgc tgtgcgtccc actccttgtg ccccattggc cgaatgcaaa    960
   ggttctctgc tagacgacag cgcaggcaag agcactgaag atactgctga gtattcccct   1020
   ttcaagggag gttacaccaa agggctagaa ggcgagagcc taggctgctc tggcagcgct   1080
   gcagcaggga gctccgggac acttgaactg ccgtctaccc tgtctctcta caagtccgga   1140
   gcactggacg aggcagctgc gtaccagagt cgcgactact acaactttcc actggctctg   1200
   gccggaccgc cgccccctcc gccgcctccc catccccacg ctcgcatcaa gctggagaac   1260
   ccgctggact acggcagcgc ctgggcggct gcggcggcgc agtgccgcta tggggacctg   1320
   gcgagcctgc atggcgcggg tgcagcggga cccggttctg ggtcaccctc agccgccgct   1380
   tcctcatcct ggcacactct cttcacagcc gaagaaggcc agttgtatgg accgtgtggt   1440
   ggtggtgggg gtggtggcgg cggcggcggc ggcggcggcg gcggcgaggc gggagctgta   1500
   gccccctacg gctacactcg gccccctcag gggctggcgg gccaggaaag cgacttcacc   1560
   gcacctgatg tgtggtaccc tggcggcatg gtgagcagag tgccctatcc cagtcccact   1620
   tgtgtcaaaa gcgaaatggg cccctggatg gatagctact ccggacctta cggggacatg   1680
   cgtttggaga ctgccaggga ccatgttttg cccattgact attactttcc accccagaag   1740
   acctgcctga tctgtggaga tgaagcttct gggtgtcact atggagctct cacatgtgga   1800
   agctgcaagg tcttcttcaa aagagccgct gaagggaaac agaagtacct gtgcgccagc   1860
   agaaatgatt gcactattga taaattccga aggaaaaatt gtccatcttg tcgtcttcgg   1920
   aaatgttatg aagcagggat gactctggga gcccggaagc tgaagaaact tggtaatctg   1980
   aaactacagg aggaaggaga ggcttccagc accaccagcc ccactgagga gacaacccag   2040
   aagctgacag tgtcacacat tgaaggctat gaatgtcagc ccatctttct gaatgtcctg   2100
   gaagccattg agccaggtgt agtgtgtgct ggacacgaca acaaccagcc cgactccttt   2160
   gcagccttgc tctctagcct caatgaactg ggagagagac agcttgtaca cgtggtcaag   2220
   tgggccaagg ccttgcctgg cctccgcaac ttacacgtgg acgaccagat ggctgtcatt   2280
   cagtactcct ggatggggct catggtgttt gccatgggct ggcgatcctt caccaatgtc   2340
   aactccagga tgctctactt cgcccctgat ctggttttca atgagtaccg catgcacaag   2400
   tcccggatgt acagccagtg tgtccgaatg aggcacctct ctcaagagtt tggatggctc   2460
   caaatcaccc cccaggaatt cctgtgcatg aaagccatgc tactcttcag cattattcca   2520
   gtggatgggc tgaaaaatca aaaattcttt gatgaacttc gaatgaacta catcaaggaa   2580
   ctcgatcgta tcattgcatg caaaagaaaa aatcccacat cctgctcaag acgcttctac   2640
   cagctcacca agctcctgga ctccgtgcag cctattgcga gagagctgca tcagttcact   2700
   tttgacctgc taatcaagtc acacatggtg agcgtggact ttccggaaat gatggcagag   2760
   atcatctctg tgcaagtgcc caagatcctt tctgggaaag tcaagcccat ctatttccac   2820
   acccagtgaa gcattggaaa ccctatttcc ccaccccagc tcatgccccc tttcagatgt   2880
   cttctgcctg ttataactct gcactactcc tctgcagtgc cttggggaat ttcctctatt   2940
   gatgtacagt ctgtcatgaa catgttcctg aattctatct gctgggcttt ttttttctct   3000
   ttctctcctt tctttttctt cttccctccc tatctaaccc tcccatggca ccttcagact   3060
   ttgcttccca ttgtggctcc tatctgtgtt ttgaatggtg ttgtatgcct taaatctgtg   3120
   atgatcctca tatggcccag tgtcaagttg tgcttgttta cacgcatctc tgtgccagcc   3180
   acacaaaccg tttacttact taccgcaagg gaacttagag agctagaatt c            3231

## Description

La présente invention concerne le domaine de l'investigation et du traitement génétiques de pathologies humaines, notamment les cancers ou les maladies infectieuses. Plus particulièrement, l'invention vise à offrir des moyens pour déterminer la fonction d'un gène ou d'une famille de gènes impliqués dans un processus cellulaire, et pour réprimer un gène nocif responsable d'une pathologie chez l'homme ou l'animal. L'invention se rapporte aux agents actifs pour la mise en oeuvre de ces méthodes et les compositions les contenant.

On connaît dans l'art antérieur, des techniques d'oligonucléotides anti-sens permettant d'inhiber spécifiquement un gène dans les cellules de mammifères. Ces techniques sont basées sur l'introduction dans les cellules d'un court oligonucléotide d'ADN complémentaire du gène cible. Cet oligonucléotide induit la dégradation de l'ARN messager transcrit par le gène cible. Un autre mode d'action des anti-sens consiste à introduire dans la cellule un oligonucléotide d'ADN qui va former une triple hélice avec le gène cible. La formation de cette triple hélice réprime le gène soit en bloquant l'accès pour des protéines activatrices, soit dans des approches plus sophistiquées, en induisant la dégradation du gène. Aucune de ces approches ne semble s'appuyer sur un mécanisme cellulaire existant dans les cellules de mammifères, et elles se sont avérés peu efficaces. En effet, l'utilisation des anti-sens en clinique est réduite à quelques cas très rares, et il n'y a aucune utilisation possible des oligonucléotides formant triple hélice.

La méthode de l'invention est basée sur l'interférence ARN désigné aussi « RNA'inh » ou « RNAi » ou encore co-suppression, qui a été mise en évidence dans les plantes. Chez les plantes, il a été observé que l'introduction d'un long ARN double brin, correspondant à un gène, induit la répression spécifique et efficace du gène ciblé. Le mécanisme de cette interférence comporte la dégradation de l'ARN double brin en courts duplex d'oligonucléotides de 20 à 22 nucléotides.

Les Inventeurs ont maintenant montré que ce principe peut s'appliquer à des gènes de mammifères qui jouent un rôle important dans le contrôle du destin cellulaire.

L'approche « RNA'inh » plus généralement dénommée selon l'invention oligonucléotides inhibiteurs ou ARNi s'appuie sur un mécanisme cellulaire dont l'importance est soulignée par son grand degré de conservation puisque ce mécanisme est conservé à travers les règnes et les espèces et a été montré non seulement chez la plante, mais aussi chez le vers Caenorhabditis Elegans et la levure et les mammifères, homme et souris.

Les travaux de recherche réalisés dans le cadre de l'invention ont montré que cette approche est beaucoup plus efficace pour réprimer spécifiquement les gènes que les techniques envisagées dans l'art antérieur. En outre, elle réunit potentiellement les avantages des anti-sens et des anti-gènes. En effet, chez la plante, la co-suppression s'effectue au niveau post-transcriptionnel, sur l'ARN mature, mais aussi au niveau transcriptionnel, donc sur le gène lui-même. En effet, la répression se transmet de génération en génération et permettrait donc de réprimer un gène de façon prolongée voire définitive.

L'invention a donc pour objet un oligonucléotide double brin constitué de deux séquences oligonucléotidiques complémentaires formant un hybride comprenant chacune à l'une de leurs extrémités 3' ou 5' un à cinq nucléotides non appariés formant des bouts simples brins débordant de l'hybride, l'une desdites séquences oligonucléotidiques étant complémentaire d'une séquence cible appartenant à une molécule d'ADN ou d'ARN d'un gène codant un récepteur aux androgènes muté ou non muté, l'une desdites séquences oligonucléotidiques étant représentée dans la liste de séquence en annexe sous le numéro SEQ ID NO. 15 ou SEQ ID NO. 16.

chacune des deux séquences oligonucléotidiques complémentaires comprend à l'une des extrémités 3' ou 5' un à cinq nucléotides non appariés formant des bouts simples brins débordant de l'hybride.

Avantageusement les deux séquences oligonucléotidiques ont la même taille.

Du fait de la loi d'appariement des bases, on désignera aussi indistinctement ci-après par oligonucléotide de l'invention, l'une ou l'autre des séquences de l'oligonucléotide double brin de l'invention qui est complémentaire d'une séquence cible appartenant à une molécule d'ADN ou d'ARN d'un gène codant un récepteur aux androgènes muté ou non muté, l'une desdites séquences oligonucléotidiques étant représenté dans la liste de séquences en annexe sous le numéro SEQ ID NO. 15 ou SEQ ID NO. 16.

Les oligonucléotides peuvent être de nature ribonucléotidique, désoxy ribonucléotidique ou mixte. On décrit également l'oligonucléotide complémentaire de la séquence cible, aussi désigné brin antisens, majoritairement de nature ribonucléotidique. Le brin sens peut être de nature ribonucléotidique désoxy ribonucléotidique ou mixte. Des exemples d'oligonucléotides de type ARN/ARN ou ADN/ARN sont donnés dans la partie expérimentale ci-après.

En effet, les hybrides ARN/ARN sont plus stables que les hybrides ADN/ADN ou ADN/ARN et beaucoup plus stables que les acides nucléiques simples brins utilisés dans des stratégies anti-sens.

On entend aussi par oligonucléotide, un polynucléotide de 2 à 100, et plus généralement de 5 à 50, nucléotides de type ribo-, désoxyribo- ou mixte.

La partie de la séquence oligonucléotidique qui est hybridée et complémentaire de la séquence cible ayant une taille comprise entre 15 et 25 nucléotides et tout préférentiellement de 20 à 23 nucléotides est decrite.

Les oligonucléotides doubles brins de l'invention comprennent à l'une de leurs l'extrémités 3' à 5' de chaque brin, de 1 à 5 nucléotides de préférence de 2 à 3 et tout préférentiellement 2 nucléotides débordant de l'hybride. Ces nucléotides débordant de l'hybride peuvent être ou non complémentaire de la séquence cible. Ainsi, l'invention, les nucléotides débordant de l'hybride sont des nucléotides quelconque par exemple des Thymines.

On peut représenter un oligonucléotide double brin de l'invention de la façon suivante, où chaque tiret correspond à un nucléotide et où chaque brin comprend à son extrémité 3' deux thymines débordant de l'hydride :

La séquence des oligonucléotides décrits est substantiellement complémentaire d'une séquence cible appartenant à une molécule d'ADN ou d'ARN messager d'un gène que l'on souhaite réprimer spécifiquement. Bien que l'on préfère des oligonucléotides parfaitement complémentaires de la séquence cible, on entend par substantiellement complémentaire, le fait que la séquence oligonucléotidique peut comprendre quelques nucléotides mutés par rapport à la séquence cible dès lors que les propriétés de répression du gène visé ne sont pas altérées. Ainsi, une séquence oligonucléotidique décrite peut comprendre de 1 à 3 nucléotides mutés.

Ces nucléotides mutés decrits peuvent donc être ceux débordant de l'hybride ou des nucléotides à l'intérieur de la séquence oligonucléotide.

un oligonucléotide de l'invention est un hybride parfait. la partie de la séquence oligonucléotidique qui est hybridée parfaitement complémentaire de la séquence cible alors que les nucléotides débordant de l'hybride peuvent être quelconques et notamment des thymines. On entend ainsi également par parfaitement complémentaire le fait que l'oligonucléotide de l'invention soit complémentaire d'une séquence qui appartient à un ADN ou ARN d'un gène codant un récepteur aux androgènes muté ou non mutés. Les oligonucléotides de l'invention peuvent permettre ainsi de discriminer entre la séquence du gène sauvage et du gène muté ce qui peut présenter un intérêt particulier tant dans l'analyse des gènes que dans les utilisations thérapeutiques des oligonucléotides de l'invention.

Les oligonucléotides de l'invention sont généralement constitués de bases nucléotidiques naturelles (A, T, G, C, U), mais peuvent aussi comprendre des nucléotides modifiés ou des nucléotides portant des groupements réactifs ou des agents de pontage ou agents intercalant pouvant réagir avec la séquence cible complémentaire à l'oligonucléotide.

Les oligonucléotides de l'invention peuvent être préparés par les méthodes conventionnelles de synthèse chimique ou biologique des oligonucléotides.

L'invention envisage aussi les oligonucléotides couplés à des substances favorisant ou permettant leur pénétration, le ciblage ou l'adressage dans les cellules, il peut s'agit de lipides, de protéines, polypeptides ou peptides ou de toute autre substance naturelle ou synthétique. En effet, les oligonucléotides de l'invention sont destinés à être internalisés dans les cellules et avantageusement dans certains cas, jusque dans le noyau des cellules, où ils vont interagir avec des molécules d'acide nucléiques portant la séquence cible de l'oligonucléotide. De même, il peut être intéressant de favoriser leur pénétration dans un tissu particulier comme une tumeur, l'os, etc.

Les oligonucléotides décrit sont utiles pour réprimer de manière très efficace et très spécifique un gène ou un ensemble de gènes et donc pour le traitement de nombreuses pathologies humaines. Ils constituent aussi un outil de recherche pour l'investigation et la compréhension de la fonction de gènes. L'invention à donc pour objet.

Composition notamment pharmaceutique pour être utilisée dans la recherche de la fonction de gène ou à des fins thérapeutiques ou diagnostiques comprenant à titre d'agent actif au moins un oligonucléotide double brin constitué de deux séquences oligonucléotidiques complémentaires formant un hybride comprenant chacune à l'une de leurs extrémités 3' ou 5' un à cinq nucléotides non appariés formant des bouts simples brins débordant de l'hybride, l'une desdites séquences oligonucléotidiques étant complémentaire d'une séquence cible appartenant à une molécule d'ADN ou d'ARN d'un gène codant un récepteur aux androgènes muté ou non muté, l'une desdites séquences oligonucléotidiques étant représentée dans la liste de séquence en annexe sous le numéro SEQ ID NO. 15 ou SEQ ID NO. 16. ou un oligonucléotide tel que défini précédemment couplé à des substances favorisant ou permettant leur pénétration, ciblage ou adressage dans les cellules.

Les oligonucléotides de l'invention peuvent être mis en oeuvre dans des applications ex vivo par exemple lors de greffe. Ainsi, les oligonucléotides peuvent être soit transfectés dans des cellules, notamment tumorales, qui seront ensuite injectées soit injectés dans les tissus par exemple des tumeurs déjà développées, par exemple par voie locale, systémique ou aérosols etc, avec agents de vectorisation éventuellement nécessaires.

Les oligonucléotides seront utilisés à des concentrations suffisantes en fonction de l'application et de la forme d'administration utilisée avec des excipients pharmaceutiques appropriés. Suivant la nature des oligonucléotides (ADN/ARN ou ARN/ARN) des doses différentes pourront être utilisées pour obtenir l'effet biologique recherché.

Les oligonucléotides décrits dans la présente invention sont également utiles comme outils de diagnostic permettant d'établir in vitro le profil génétique d'un patient à partir d'un échantillon cellulaire de celui-ci. La mise en oeuvre des oligonucléotides décrits dans un tel procédé d'analyse permet de connaître ou d'anticiper la réponse des cellules cancéreuses de ce patient et d'établir un traitement personnalisé ou encore d'ajuster le traitement d'un patient.

Les oligonucléotides de l'invention présentent plusieurs avantages par rapport aux agents chimiothérapeutiques classiques :
- Les hybrides ARN-ARN sont plus stables que les hybrides ADN-ADN ou ADN-ARN et beaucoup plus stables que les acides nucléiques simples brins utilisés dans des stratégies anti-sens.
- Ils constituent des composés naturels, aucune réaction immunologique ou d'intolérance médicamenteuse n'est a priori à craindre.
- Les expériences de transfections réalisées dans le cadre de l'invention montrent une bien meilleure pénétration des RNAi dans les cellules tumorales que celle obtenue avec des plasmides. Ce point est essentiel dans le cas de cellules tumorales qui sont généralement très difficiles à transfecter.
- Les expériences d'injection systémique de siRNA in vivo montrent une très bonne pénétration de ces molécules dans les tissus.
- Il est aisé de mélanger plusieurs RNAi entre eux afin de prendre pour cibles plusieurs gènes cellulaires en même temps.

Les oligonucléotides décrits et les compositions les contenant sont utiles pour le traitement ou la prévention des maladies infectieuses ou virales, en particulier le SIDA, les maladies infectieuses non conventionnelles, en particulier ESB et Kreutzfeld Jacob. Ils sont tout particulièrement indiqués pour traiter des maladies virales à l'origine de cancers. Le tableau ci-dessous rapporte des exemples de virus impliqués dans des pathologies cancéreuses chez l'homme.

**Tableau 1**

| Virus | Type de cancer humain associé |
|---|---|
| Virus de l'hépatite B (VHB) | Carcinome du foie |
| Virus d'Epstein-Barr (EBV) | Lymphome de Burkitt, cancer nasopharyngé, maladie de Hodgkin, lymphomes non hodgkiniens, cancer gastrique, cancer du sein. |
| Herpèsvirus humain 8 ou HHV-8/KSHV | Sarcome de Kaposi (SK), lymphomes primitif des séreuses (PEL), maladie de Castelman multifocale (MCD) |
| VPH | Col de l'utérus, tête, cou, peau, nasopharynx |
| Virus des lymphocytes T (HTLV) | Leucémie de type T |
| Virus de l'hépatite C (VHC) | Carcinome du foie |

Les oligonucléotides décrits et les compositions les contenant sont encore utiles pour le traitement ou la prévention des maladies liées à une hypervascularisation comme la dégénérescence maculaire liée à l'age, l'angiogénèse tumorale, les rétinopathies diabétiques, le psoriasis, l'arthrite rhumatoide.

Les travaux de recherche réalisés ont permis de montrer que ces oligonucléotides sont particulièrement adaptés pour réprimer des gènes nocifs impliqués dans la cancérisation et sont donc tout particulièrement utiles pour le traitement ou la prévention des cancers et plus généralement des maladies oncologiques.

Les oligonucléotides de l'invention sont utiles pour la préparation d'une composition pharmaceutique utile pour la prévention ou le traitement du cancer.

De manière préférée, les oligonucléotides de l'invention sont utiles pour la préparation d'une composition pharmaceutique utile pour la prévention ou le traitement du cancer de la prostate.

Un traitement anti-cancéreux idéal doit entraîner la mort de la cellule tumorale tout en évitant les phénomènes de résistance. La mort cellulaire peut être obtenue par :
- Inhibition de la division cellulaire, blocage du cycle cellulaire,
- Induction de l'apoptose des cellules tumorales,
- Induction de la sénescence,
- Induction de la nécrose,
- Induction de la différenciation. Dans ce cas, les traitements conduisent la cellule à redevenir normale.

Ainsi, dans la presente, un oligonucléotide ou un ensemble d'oligonucléotides différents, comportant chacun une séquence oligonucléotidique complémentaire d'une séquence cible appartenant à une molécule d'ADN ou d'ARN messager d'un gène dont la répression induit l'apoptose, ou la sénescence, ou la nécrose, ou la différenciation des cellules tumorales ou empêche leur division ou plusieurs de ces phénomènes sont décrits.

L'induction de l'apoptose des cellules tumorales est basée sur le fait que la fonction de nombreux gènes cellulaires (par exemple membres de la famille BCL2, BCL XL) est de protéger les cellules de l'apoptose. La perte d'expression de ces gènes induite par RNAi permet donc le passage en apoptose.

La mort cellulaire peut également être provoquée par la perte d'adhésion des cellules à la matrice (anoïkis). Cet effet peut être obtenue en perturbant la balance entre protéases et inhibiteurs de protéases dans les tumeurs et leur environnement stromal. Cette perturbation a par ailleurs pour effet de diminuer les capacités des cellules tumorales à envahir les tissus sains et à se métastaser. Les siRNA peuvent donc être utilisés pour empêcher la synthèse de protéines des familles des métallo protéases matricielles (MMP), des métallo protéases matricielles membranaires, de leurs inhibiteurs (TIMPs), ainsi que celle des activateurs des inhibiteurs des protéases comme par exemple PAI-1 et des protéases elles-mêmes comme par exemple l'urokinase.

L'induction de la sénescence repose sur le fait que les cellules normales ne peuvent se diviser qu'un nombre limité de fois. Ce nombre est programmé, environ 50 divisions par exemple pour des fibroblastes embryonnaires, et "mesuré" par la longueur des télomères qui se raccourcit au fur et à mesure des divisions cellulaires. En deçà d'une certaine taille, les télomères ne sont plus fonctionnels et la cellule, incapable de se diviser, entre en sénescence. Dans les cellules germinales cependant, cette longueur est maintenue constante par l'action d'une enzyme, la télomérase. La télomérase est ré-exprimée dans de nombreux cancers, ce qui permet aux cellules tumorales de se multiplier indéfiniment. Un RNAi bloquant l'expression de la télomérase serait sans conséquence sur les cellules somatiques normales et devrait conduire les cellules tumorales vers la sénescence.

Le blocage de la division cellulaire conduit également les cellules à la sénescence. Ce blocage peut être obtenu en inhibant des récepteurs cellulaires essentiels. Ces récepteurs peuvent appartenir suivant la nature de la cellule soit à la classe des récepteurs des facteurs de croissance (EGF, SST2, PDGF, FGF notamment), que ceux-ci soient ou non mutés, soit à celle des récepteurs nucléaires d'hormones (androgènes, oestrogènes, glucocorticoïdes notamment).

Les récepteurs d'hormones sont fréquemment mutés dans les cancers, et l'invention concerne dans ce cas l'utilisation d'oligonucléotides reconnaissant les formes mutées de ces récepteurs et qui n'inhibent pas la synthèse des formes sauvages. Ceci permet par exemple dans le cas des carcinomes prostatiques devenus résistants par mutation du récepteur des androgènes de traiter par voie systémique les patients avec des siRNA qui bloquent la synthèse du récepteur muté sans induire d'effets de castration liés à l'inhibition des formes sauvage du récepteur dans d'autres organes. Un exemple d'utilisation d'oligonucléotides reconnaissant des formes mutées du récepteur est présenté.

Le cycle cellulaire peut également être arrêté en inhibant la synthèse de protéines indispensables à son déroulement comme par exemple les cyclines, kinases dépendantes des cyclines, enzymes de réplication de l'ADN, facteurs de transcription tels que E2F.

L'induction de la nécrose résulte du besoin des cellules tumorales en oxygène et en nutriments. Initialement une tumeur assure son développement à partir des vaisseaux préexistants de l'hôte. Au-delà de 1 à 2 mm de diamètre, les cellules situées au centre de la tumeur se trouvent en hypoxie. Cette hypoxie, par l'intermédiaire d'une proline hydroxylase, entraîne la stabilisation du facteur de transcription Hif1α, dont la séquence SEQ ID NO. 59 est donnée en annexe, qui, en se fixant sur des séquences HRE dans les promoteurs de ses gènes cibles, déclenche la réaction hypoxique. Cette réaction conduit à l'activation d'une centaine de gènes permettant d'activer notamment la voie de la glycolyse anaérobie, la réponse au stress et l'angiogénèse. Ce dernier mécanisme active en particulier le gène du VEGF, dont la séquence SEQ ID NO. 60 est donnée en annexe, principal facteur angiogénique tumoral.

Ainsi des oligonucléotides bloquant par exemple l'expression du facteur de transcription Hif1α ou par exemple celle du VEGF mettent les cellules tumorales dans l'incapacité à monter une réponse hypoxique ou angiogénique. L'angiogénèse est un mécanisme normalement réprimé chez l'adulte à l'exception du cycle menstruel (utérus ovaires). L'inhibition de ce mécanisme a donc peu de conséquences pour les tissus normaux.

Dans la présente, il est décrit un oligonucléotide dont l'une desdites séquences oligonucléotidiques est substantiellement complémentaire d'une séquence cible appartenant à une molécule d'ADN ou d'ARN messager du gène codant :
- le facteur de transcription Hif1α;
- une ou plusieurs des isoformes du VEGF A ou d'un membre de la famille de ce facteur de croissance.

Dans certains cancers, le phénotype tumoral résulte de, ou est maintenu par, l'expression d'une protéine normalement absente des cellules normales. Cette protéine peut résulter de l'expression actuelle ou ancienne d'un génome viral dans la cellule comme celui du virus du papillome, HPV, ou du virus de l'hépatite B. Cette protéine peut également résulter de la mutation (ponctuelle, délétion, insertion) d'un gène cellulaire normal. Dans ce cas, il est fréquent que la protéine mutée ainsi produite possède des propriétés transdominantes négatives par rapport à la protéine normale. La spécificité des siRNA permet d'inhiber la synthèse de la protéine mutante sans bloquer la synthèse des protéines sauvages. Deux exemples concernant des formes mutées de la protéine p53 et du récepteur des androgènes sont rapportés dans la partie expérimentale ci-après.

Les travaux de recherche réalisés dans le cadre de l'invention ont permis de montrer que ces oligonucléotides sont particulièrement adaptés pour réprimer des gènes nocifs impliqués dans la cancérisation et tout particulièrement à ceux conduisant à la formation de protéine de fusion dans les cellules cancéreuses, comme la protéine de fusion PML-RAR alfa.

Dans la présente, il est décrit des oligonucléotides dont la séquence est complémentaire d'une séquence cible appartenant à un gène résultant d'une translocation chromosomique de façon à inhiber les effets de la protéine de fusion exprimée par ce gène. Ainsi, la séquence cible est celle correspondant à la séquence de la jonction de la protéine de fusion.

Le tableau 2 de l'annexe A à la fin de la présente description est une liste non exhaustive des protéines de fusion représentant des cibles thérapeutiques ou diagnostiques pour les oligonucléotides décrits.

Le fait de cibler avec un oligonucléotide décrit , la jonction entre deux gènes, par exemple les deux gènes pml et rarα, permet d'aboutir à l'inhibition spécifique de la protéine de fusion sans affecter le rôle biologique des protéines naturelles qui peuvent être codées par le second allèle. Cette forme de mise en oeuvre englobe donc toutes les protéines de fusion impliquées dans la cancérogénèse, particulièrement les leucémies. Les formes réciproques si elles existent, ainsi que tous les variants des protéines de fusion citées en annexe constituent également des cibles. L'invention concerne donc tout particulièrement, l'utilisation des oligonucléotides comme défini ci-dessus pour la préparation d'une composition pharmaceutique destinée au traitement des cancers.

Les thérapies anti-cancéreuses actuelles prennent pour cible les cellules cancéreuses, par différentes approches, prises isolément ou combinées entre elles (chimiothérapie, chirurgie, radiothérapie, immunothérapie). Les échecs thérapeutiques sont massivement dus soit à des cellules n'ayant pas été atteintes par le traitement soit, et majoritairement, à des cellules ayant muté en réponse au traitement. Cette capacité de mutation est grandement facilitée par l'instabilité génétique des cellules tumorales. L'inhibition de la vascularisation tumorale, privant les cellules d'oxygène et de nutriments, a depuis quelques années ouvert de nouvelles perspectives thérapeutiques en cancérologie. Cette stratégie, complémentaire des précédentes, prend pour cible la cellule endothéliale normale de l'hôte, génétiquement stable, et donc théoriquement peu susceptible de muter. De nombreux essais cliniques visant à inhiber l'angiogénèse tumorale par différentes approches sont en cours dans le monde. Cependant, les premiers résultats rapportés semblent assez décevants.

Les Inventeurs ont démontré que des tumeurs sont capables de compenser les effets d'inhibiteurs de l'angiogénèse, en sélectionnant des sous-populations de cellules sécrétant de fortes concentrations de facteurs pro angiogéniques.

Les tumeurs ne sont pas constituées de cellules homogènes quant à leur expression génique. Ceci est attesté par de très nombreuses études dans lesquelles des immunomarquages ont été réalisés pour une grande variété d'antigènes dans les tumeurs. Macroscopiquement, une tumeur est fréquemment composée de régions hautement vascularisées côtoyant des zones de nécrose ou au contraire avasculaires.

Cette hétérogénéité tumorale favorise l'échappement des tumeurs aux traitements appliqués, quelle qu'en soit la nature. Plus la diversité de l'expression génique dans une tumeur est grande plus la probabilité qu'il existe au moins une cellule capable de résister à un agent anti-tumoral est en effet élevée. Il apparaît dès lors essentiel d'associer différentes stratégies afin de réduire tout d'abord l'hétérogénéité tumorale et d'éviter les phénomènes d'échappement.

Dans la présente, il est décrit des siRNAs inhibiteurs de l'expression de gènes responsables de l'inactivation de la p53 et à leur utilisation dans le traitement des cancers. La p53 est le produit d'un gène suppresseur de tumeurs ou anti-oncogène, muté dans plus de 50% des tumeurs chez l'homme. La p53 est ainsi considérée comme un « gardien du génome ». Elle est activée dans les cellules en cas de stress génotoxique et participe à divers processus dont l'induction du processus de mort programmée.

Dans 74% des cas de mutation monoallélique, l'inactivation de la p53 est due à une mutation ponctuelle aboutissant à l'expression d'une protéine de taille normale, mais mutée. On considère généralement que la forme mutée forme des hétéromères avec le produit de l'allèle sauvage sur lequel elle agit comme un « transdominant négatif » et bloque son activité. La forme mutante semble également avoir une activité oncogénique en elle-même. Ainsi, des formes mutées de la p53 sont capables d'activer le gène MDR, qui facilite la résistance des cellules cancéreuses aux chimiothérapies. De plus, l'expression de mutants de la p53 est associée à une plus forte angiogenèse tumorale, sans doute en raison du fait que les formes mutantes de la p53 ne sont plus capables de stimuler la transcription du gène de la thrombospondine, l'un des plus puissants répresseurs de l'angiogénèse, et activent le VEGF et le bFGF, deux puissants activateurs de l'angiogénèse. De plus, les cellules dans lesquelles une forme mutée de la p53 s'exprime perdent divers niveaux de régulation. En particulier elles ne sont plus capables d'entamer un processus de mort programmée, qui constitue l'un des processus majeurs de protection contre la tumorigenèse. La restauration d'une activité p53 sauvage entraîne, dans des cellules tumorales en culture, la restauration de cette réponse cellulaire. Ainsi, l'inhibition de l'expression des formes mutées de la p53 représente potentiellement un outil puissant en thérapie anti-cancéreuse.

Il n'y a, à l'heure actuelle, aucun moyen efficace de restaurer une activité p53 dans les cellules cancéreuses humaines. En ce qui concerne les cancers dans lesquels les deux allèles sont inactivés, des tentatives de restauration de l'activité p53 par thérapie génique sont envisagées. Ces approches sont compliquées par l'utilisation de vecteurs viraux et se montrent pour le moment peu efficaces.

Par ailleurs, il a été observé spécifiquement dans les cancers cervicaux liés à l'infection par le virus HPV des cellules du col de l'utérus, que la p53 peut-être inactivée par la surexpression d'une protéine virale. En effet, ce virus code pour une protéine, la protéine E6, qui inactive la p53. Dans ce type de cancers, c'est l'inhibition de la protéine E6 qui pourra restaurer une activité p53 sauvage.

Dans la présente, il est décrit de nouveaux moyens permettant d'activer la p53 en inhibant l'expression de gènes responsables de son inactivation. Les travaux de recherche réalisés dans le cadre de la présente invention ont permis de mettre en évidence qu'il était ainsi possible de réprimer de manière très efficace et très spécifique l'expression d'une forme mutante de la p53.

Dans la présente, il est décrit des oligonucléotides présentant une séquence complémentaire d'une séquence polynucléotidique spécifique du gène de la p53 mutée. Il s'agit donc d'oligonucléotides dont la séquence porte une mutation par rapport à la séquence de la p53 sauvage. La séquence du gène sauvage de la p53 est indiquée dans la liste de séquences en annexe sous le numéro SEQ ID NO.1. Les différentes mutations pouvant intervenir sur la séquence de la p53 sont indiquées dans le tableau 3 de l'annexe B à la fin de la présente description.

Les mutations les plus fréquemment observées dans les pathologies cancéreuses sont reportées dans le tableau 4 ci-après.

**Tableau 4**

| Position | P53 sauvage | SEQ ID No. |
|---|---|---|
| R273H | GAGGTGCGTGTTTGTGC | SEQ ID No. 61 |
| R248Q | gcaTgaaccggaggcccaT | SEQ ID No. 62 |
| R248W | gcaTgaaccggaggcccaT | SEQ ID No. 63 |
| R249S | gcaTgaaccggaggcccaT | SEQ ID No. 64 |
| G245S | CTGCATGGGCGGCATGAAC | SEQ ID No. 65 |
| R282W | TGGGAGAGACCGGCGCACA | SEQ ID No. 66 |
| R175H | TGTGAGGCACTGCCCCCAC | SEQ ID No. 67 |
| C242S | TAACAGTTCCTGCATGGGCG | SEQ ID No. 68 |
| Position | P53 mutée | |
| R273H | GAGGTGCATGTTTGTGC | SEQ ID No. 69 |
| R248Q | gcaTgaacCAgaggcccaT | SEQ ID No. 70 |
| R248W | GCATGAACTGGAGGC CAT | SEQ ID No. 71 |
| R249S | gcaTgaaccggagTcccaT | SEQ ID No. 72 |
| G245S | CTGCATGC,GCAGCATGAAC | SEQ ID No. 73 |
| R282W | TGGGAGAGACTGGCGCACA | SEQ ID No. 74 |
| R175H | TGTGAGGCGCTGCCCCCAC | SEQ ID No. 75 |
| C242S | TAACAGTTCCTCCATGGGCG | SEQ ID No. 76 |

Ainsi, des oligonucléotides décrits sont complémentaires d'une séquence cible appartenant au gène de la p53 muté portant l'une au moins des mutations données dans le tableau 3 et tout particulièrement l'une au moins des mutations du tableau 4 ci-dessus.

Ces oligonucléotides sont capables de discriminer de manière efficace entre la forme sauvage et la forme mutée de la p53. En effet, la stratégie est de bloquer l'expression de la forme mutée pour réactiver la forme sauvage et induire dans les cellules un processus de mort programmée pour lequel la forme sauvage est indispensable, et/ou bloquer tout autre processus induit par la forme mutée de la p53. En outre, cette capacité de discrimination des oligonucléotides de l'invention permet de ne toucher que les cellules cancéreuses et d'épargner les cellules normales, qui n'expriment pas cette forme mutée de la p53.

Dans la présente, il est décrit le traitement ou la prévention des maladies induites par une inactivation de la protéine p53 et tout particulièrement les cancers résultant de l'expression de la protéine p53 mutée et les cancers résultant de l'expression de gènes inhibiteurs de la p53. Dans la présente, il est décrit la prévention de l'apparition de cancers chez les sujets exprimant une forme mutée de la p53, comme dans le cas du syndrome de Li Fraumeni.

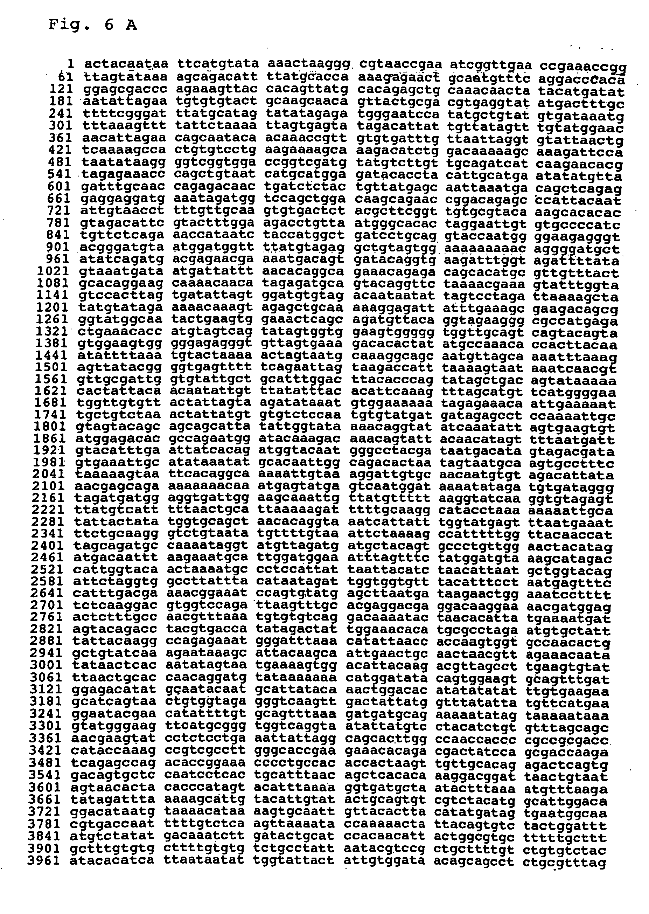

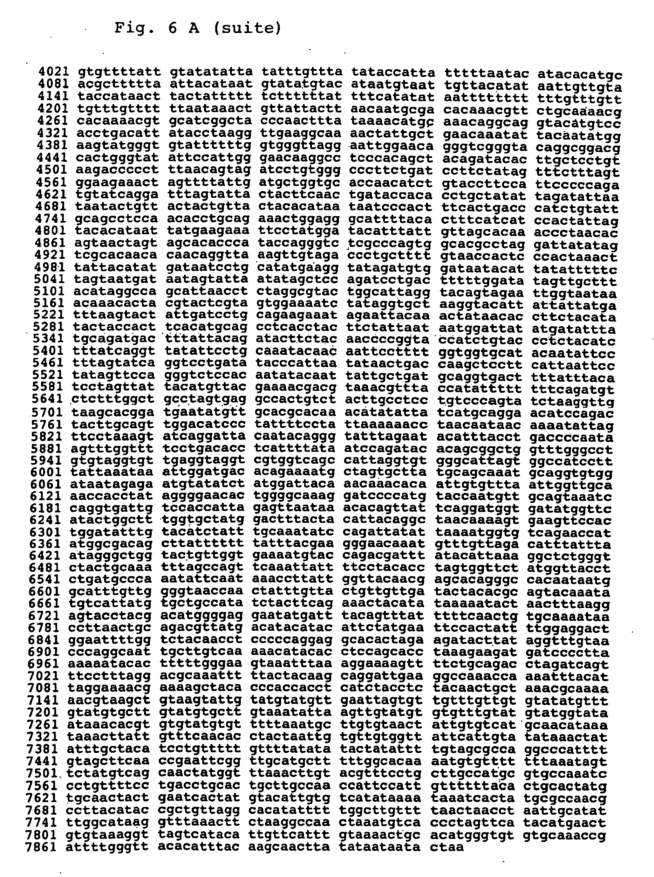

La P53 peut être inactivée à travers plusieurs mécanismes distincts. En particulier, dans la majorité des cancers cervicaux, la P53 est inactivée par une protéine codée par le virus du papillome humain, la protéine E6. E6 entraîne l'ubiquitinylation de la P53 ce qui conduit à sa dégradation par le protéasome. Dans ce cas, l'expression de la P53 peut être restaurée par l'inhibition de l'expression de la protéine E6. Dans la présente, il est décrit des oligonucléotides présentant une séquence complémentaire d'une séquence polynucléotidique spécifique du gène de la protéine E6 de HPV. La séquence du gène de la protéine E6 de HPV est donnée à la figure 6A ainsi que dans la liste de séquences en annexe sous le numéro SEQ ID NO 2.

Comme indiqué précédemment, une stratégie selon l'invention a pour but de bloquer à l'aide de RNAi l'expression du récepteur des androgènes dans les carcinomes. La séquence du récepteur des androgènes est donnée dans la liste de séquence en annexe sous le numéro SEQ ID NO. 77. Pour traiter les carcinomes avant qu'ils ne soient devenus résistants ou ceux qui le sont devenus par amplification du récepteur sans mutation, des siRNA homologues d'une région pour laquelle aucune mutation n'a été décrite dans les banques de données des mutations du récepteur des androgènes (notés siRNA AR) ont été utilisés. Pour traiter spécifiquement les carcinomes prostatiques devenus androgéno résistants par mutation, un séquençage de l'ARNm codant pour le récepteur sera effectué dans les cellules du patient afin de concevoir une séquence spécifique de la mutation, permettant de traiter le patient sans conséquence pour les cellules normales. Un exemple est présenté par l'utilisation de siRNA reconnaissant spécifiquement la mutation du récepteur des androgènes présente dans la lignée cellulaire LNCaP (siRNA LNCaP).

En conséquence, l'invention se rapporte à des oligonucléotides double brin constitué de deux séquences oligonucléotidiques complémentaires formant un hybride comprenant chacune à l'une de leurs extrémités 3' ou 5' un à cinq nucléotides non appariés formant des bouts simples brins débordant de l'hybride, l'une desdites séquences oligonucléotidiques étant complémentaire d'une séquence cible appartenant à une molécule d'ADN ou d'ARN d'un gène codant un récepteur aux androgènes muté ou non muté, l'une desdites séquences oligonucléotidiques étant représentée dans la liste de séquence en annexe sous le numéro SEQ ID NO. 15 ou SEQ ID NO. 16. Il s'agit par exemple du récepteur aux androgènes portant l'une au moins des mutations données dans le tableau 5 de l'annexe C. Ces oligonucléotides de l'invention spécifiques du récepteur aux androgènes sont utiles pour traiter ou prévenir les maladies androgéno-dépendantes, comme par exemple le cancer de la prostate.

D'autres avantages et caractéristiques de l'invention apparaîtront des exemples qui suivent concernant :
- Exemple 1 : Inhibition de la protéine PML-RARα associée à la leucémie aiguë promyélocytaire (APL).
- Exemple 2 : Inhibition de l'angiogénèse tumorale induite par le VEGF.
- Exemple 3 : Inhibition de la réponse hypoxique induite par HIF1 α.
- Exemple 4 : Inhibition des formes sauvages ou mutantes des récepteurs des androgènes dans les cellules de carcinome prostatique.
- Exemple 5 : Inhibition des formes sauvages ou mutantes de la protéine p53.
- Exemple 6 : Inhibition de la protéine virale E6.
- Exemple 7 : Utilisation des hybrides ADN/ARN pour inhiber l'expression de différentes protéines.
- Exemple 8 : Administration in vivo de siRNA par différentes voies.

Il est fait référence dans les exemples aux figures dans lesquelles :

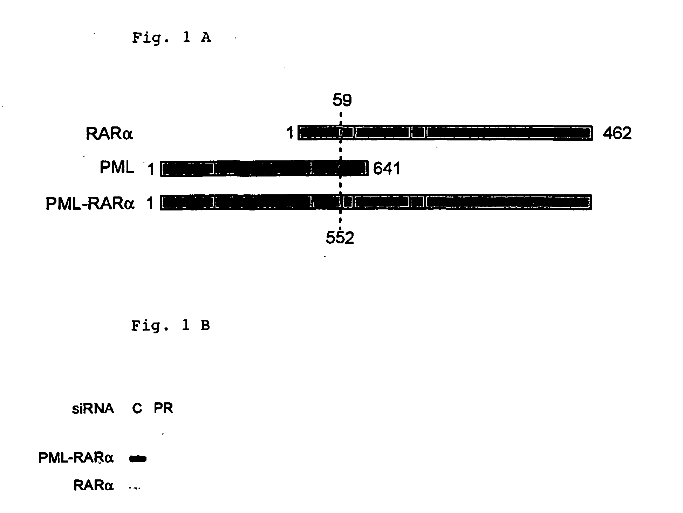

- La figure 1A est une représentation schématique des protéines RARα, PML et de la protéine de fusion associée, PML-RARα. La figure 1B représente les résultats de transfections avec un siRNA dirigé contre PML-RARα.

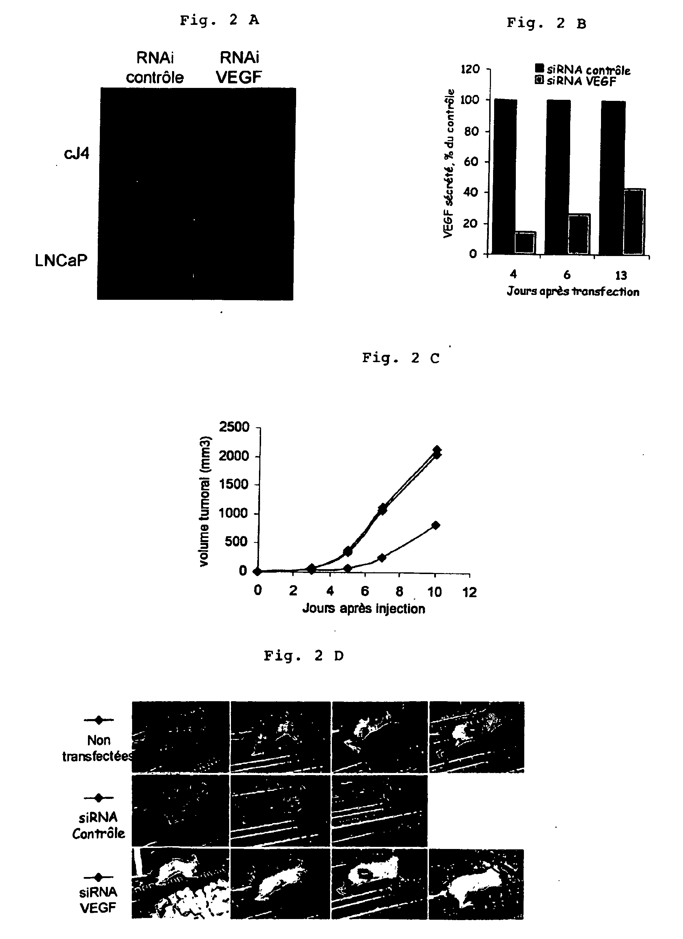

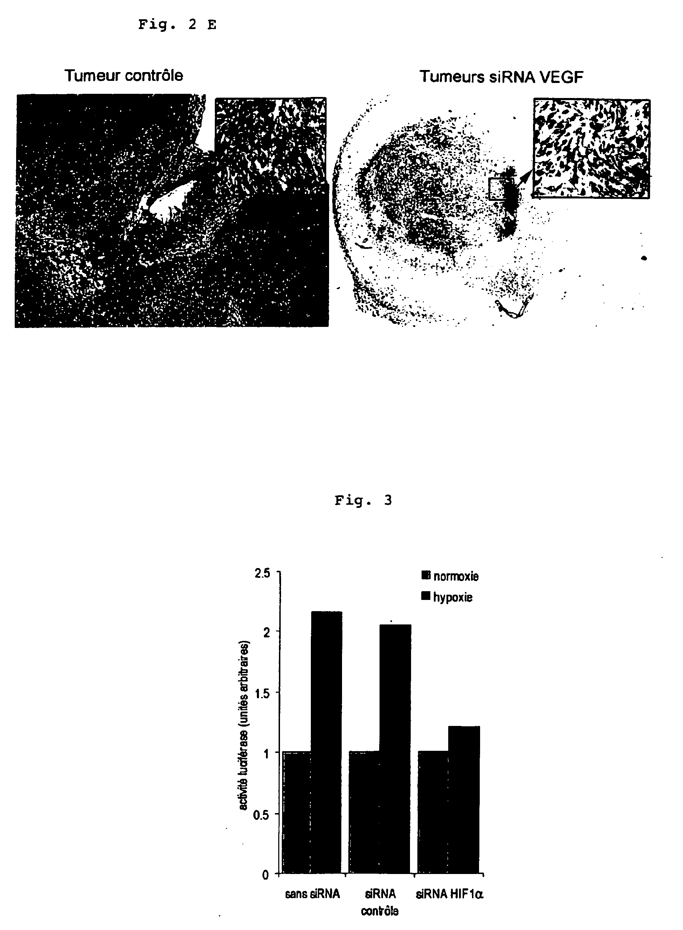

- La figure 2 concerne l'inhibition de l'expression du VEGF par des siRNA dirigés contre cette protéine et les conséquences de cette inhibition. La figure 2A représente l'immunodétection du VEGF dans des cellules cJ4 ou LNCaP transfectées par le siRNA contrôle ou un siRNA dirigé contre le VEGF. La figure 2B représente la quantification par ELISA du VEGF dans le milieu conditionné des cellules cj4 transfectées par le siRNA contrôle ou le siRNA VEGF en fonction du temps après la transfection. La figure 2C représente la courbe de croissance chez des souris nudes de tumeurs provenant de l'injection sous cutanée de 10⁶ cellules cJ4 non transfectées, transfectées par le siRNA contrôle ou le siRNA VEGF. La figure 2D représente l'aspect des tumeurs au jour 7 après injection des cellules. La figure 2E représente l'immunodétection du VEGF dans des tumeurs provenant de l'injection de cellules cj4 transfectées avec le siRNA contrôle ou le siRNA VEGF après 12 jours de développement in vivo.
- La figure 3 concerne l'effet de l'inhibition par un siRNA spécifique de l'expression d'un facteur de transcription, HIFlalpha, sur la réponse transcriptionnelle à l'hypoxie. La figure représente la mesure de l'activité d'un reporter VEGF luciférase en réponse à l'hypoxie dans des cellules cJ4 non transfectées, transfectées par le siRNA contrôle ou par le siRNA dirigé contre HIFlalpha.

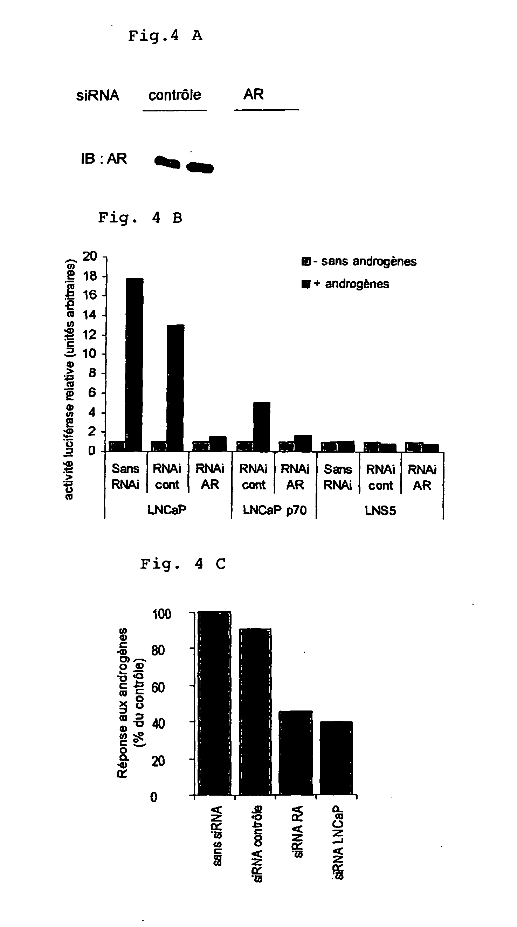

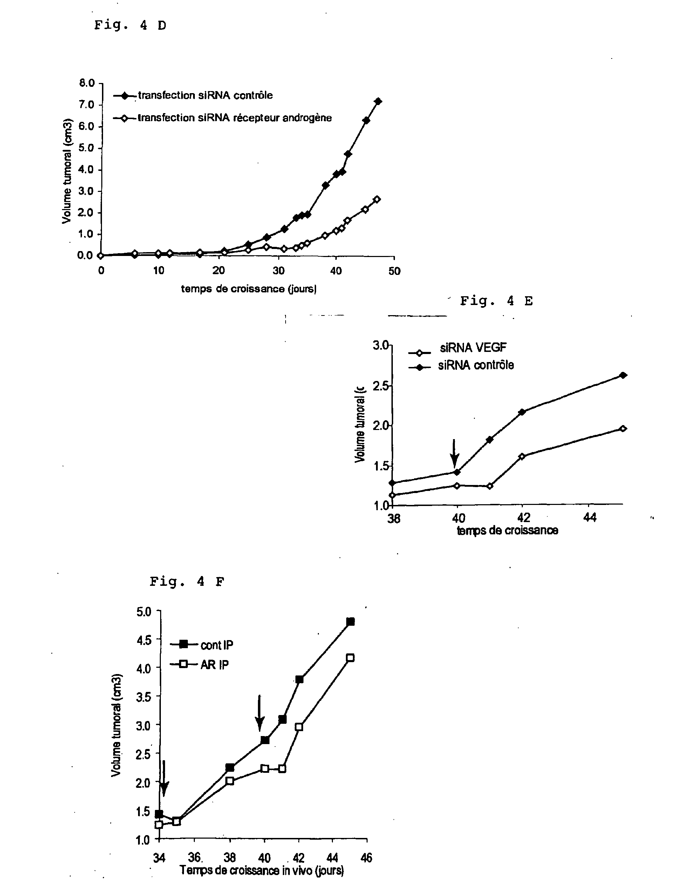

- La figure 4 concerne l'inhibition par des siRNA spécifiques de l'expression du récepteur des androgènes dans des cellules et les conséquences fonctionnelles de ces inhibitions. La figure 4A représente la détection par immunoblot de l'expression du récepteur des androgènes 48h après transfection des cellules LNCaP par un siRNA contrôle ou un siRNA dirigé contre le récepteur des androgènes (AR). La figure 4B représente la mesure de l'activité d'un reporter 4xARE luciferase au R1881 dans divers clones de la lignée LNCaP non transfectée, ou transfectée par le siRNA contrôle ou le siRNA AR. La figure 4C représente la comparaison de la réponse au R1881 de cellules LNCaP non transfectées (100%), et des cellules LNCaP transfectées par un siRNA contrôle, un siRNA dirigé contre le récepteur des androgènes (AR) ou un siRNA reconnaissant spécifiquement une mutation ponctuelle présente dans le récepteur des androgènes de la lignée LNCaP. La figure 4D représente la croissance chez des souris nudes de tumeurs résultant de l'injection sous cutanée de cellules LNCaP transfectées par un siRNA contrôle ou par un siRNA dirigé contre le récepteur des androgènes. La figure 4E représente la croissance de tumeurs LNCaP chez des souris ayant reçu au 40e jour après implantation des cellules une injection intraveineuse dans la veine de la queue de 2µg de siRNA dirigés contre le VEGF ou de siRNA contrôle. La figure 4F représente la croissance de tumeurs LNCaP chez des souris ayant reçu au 34e et au 40e jour après implantation des cellules tumorales une injection intrapéritonéale de 2µg de siRNA dirigé contre le récepteur des androgènes ou de siRNA contrôle.

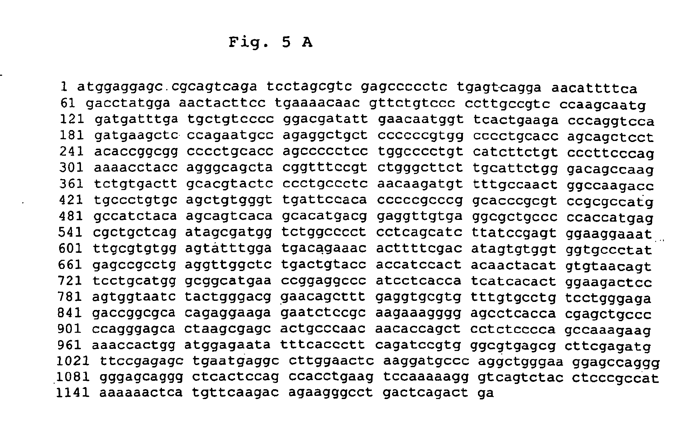

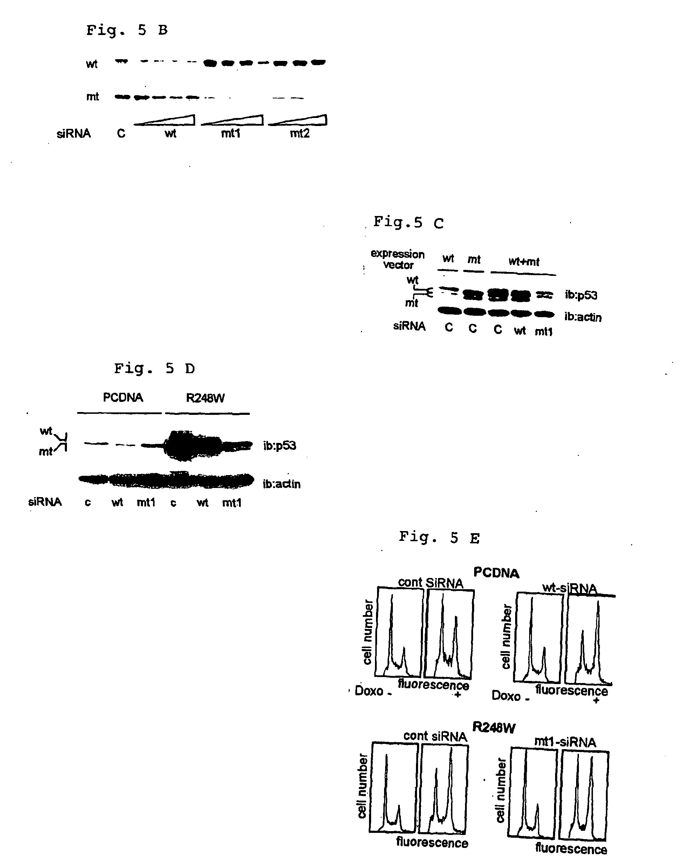

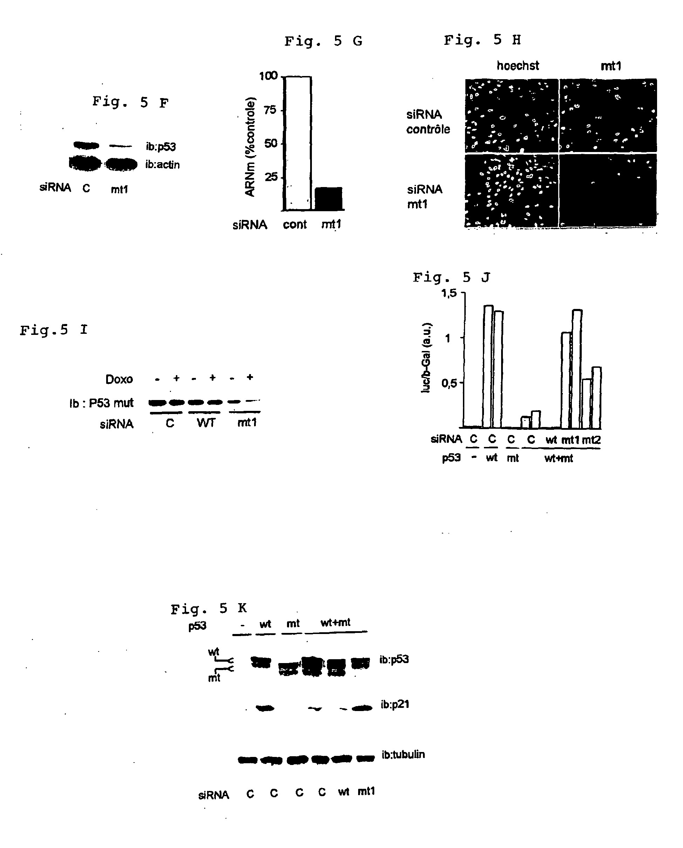

- La figure 5 concerne l'inhibition de l'expression de formes sauvages ou mutantes de la p53 par des siRNA et des conséquences fonctionnelles de ces inhibitions. La figure 5A représente la séquence de protéine p53 humaine. La figure 5B représente l'inhibition spécifique et dépendante de la dose par des siRNA de l'expression de formes sauvages ou mutantes de la p53 transfectées dans des cellules ne l'exprimant pas initialement. La figure 5C représente l'inhibition spécifique par des siRNA de l'expression simultanée ou non de formes sauvages ou mutantes de la p53 transfectées dans des cellules ne l'exprimant pas initialement. La figure 5D représente l'inhibition de l'expression de la p53 endogène sauvage ou d'une forme mutante transfectée de p53 par des siRNA. La figure 5E représente l'effet de l'inhibition de la p53 par des siRNA sur la résistance à un stress génotoxique. Les figures 5 F, G, H et I montrent l'inhibition de l'expression d'un forme mutante de la p53 dans les cellules d'un patient atteint du syndrome de Li Fraumeni sur le niveau de l'ARNm (5G), et l'expression de la protéine par immunoblot (GF) ou en immunofluorescence indirecte (5H) et les conséquences sur la résistance de ces cellules à un stress génotoxique. La figure 5J montre l'inhibition par les siRNA spécifiques de la transcription dépendante de la p53 dans des cellules exprimant par transfection des formes sauvages ou mutantes de la p53. La figure 5K montre l'inhibition de l'expression d'un des gènes cibles de la p53, la p21, protéine inhibitrice de la prolifération cellulaire, par la coexpression de formes mutantes de la p53 et la restauration de cette expression par traitement des cellules avec un siRNA inhibant la synthèse de la forme mutante de la p53.

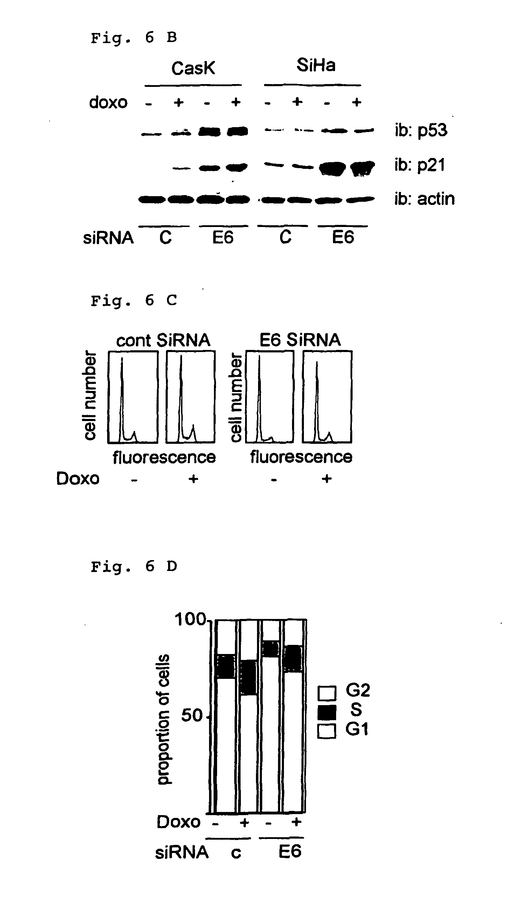

- La figure 6 concerne l'inhibition de l'expression de la protéine E6 du virus du papillome humain HPV par des siRNA spécifiques et les conséquences de cette inhibition. La figure 6A représente la séquence de la protéine HPV. La figure 6B représente l'effet de l'inhibition par des siRNA spécifiques de l'expression de la protéine E6 de HPV dans des cellules qui expriment ce virus, sur l'expression de la p53 et de la p21. Les figures 6C et 6D représentent l'effet de l'inhibition de l'expression de la protéine E6 de HPV sur le cycle cellulaire.

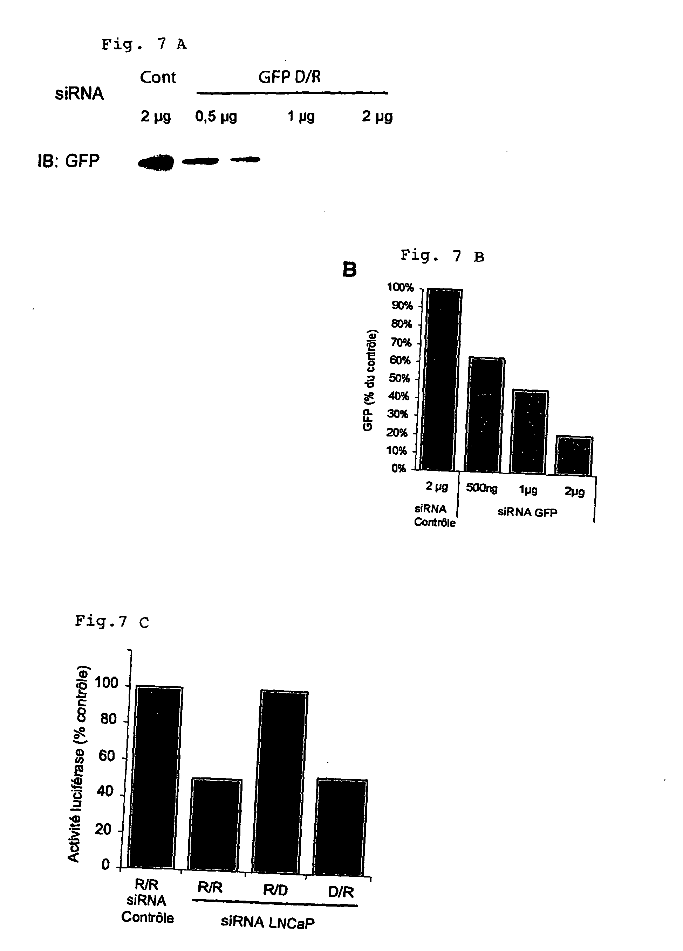

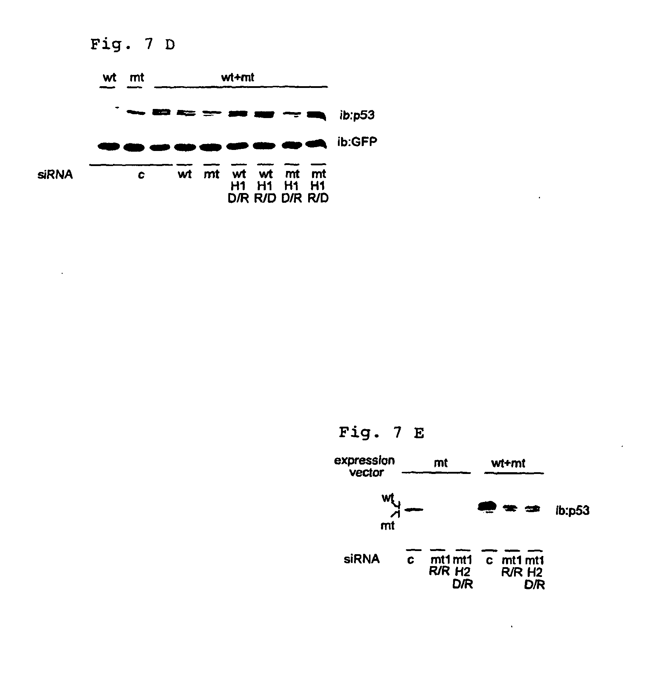

- La figure 7 concerne l'utilisation de siRNA hybrides, comportant des bases ADN et des bases ARN. Les figures 7A et 7B représentent l'effet de siRNA hybrides ADN/ARN sur l'expression de la GFP exprimée par transfection dans des cellules. La figure 7C compare l'effet de siRNA ARN/ARN, ADN/ARN ou ARN/ADN à dose constante sur l'inhibition de la transcription induite par le récepteur des androgènes. Les figures 7D et 7E représentent les effets d'une substitution de bases ARN par des bases ADN dans la séquence de siRNA inhibant la synthèse de la p53.

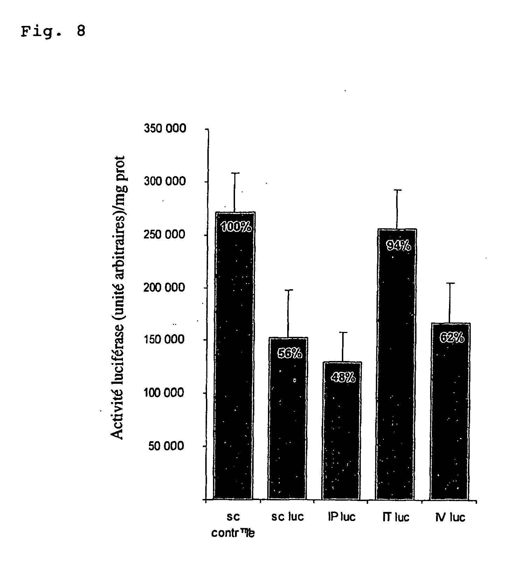

- La figure 8 concerne l'inhibition de la luciférase dans des tumeurs exprimant cette enzyme par injection de siRNA par voie sous cutanée, ou intra-tumorale ou intra-péritonéale ou intra-veineuse

### EXEMPLE 1: INHIBITION DE LA PROTEINE PML-RARα ASSOCIEE A LA LEUCEMIE AIGUË PROMYELOCYTAIRE (APL).

### I - Introduction.

La leucémie aiguë promyélocytaire (APL) est due à la translocation t(15 ;17) sur le chromosome 15. Chez les patients atteints, le récepteur de l'acide rétinoïque RARα (RARα) est fusionné à la protéine PML (*promyelocytic leukemia protein*) générant ainsi la protéine de fusion PML-RARα. Jusqu'à ce jour, cinq protéines de fusion mettant en jeu le RARα ont été identifiées. Tous ces types de leucémies impliquent le récepteur RARα et sont cliniquement similaires, ce qui suggère que la rupture de la voie de transduction de l'acide rétinoïque est cruciale dans la pathogenèse des leucémies APL.

La protéine de fusion PML-RARα a gardé les domaines de liaison à l'ADN et à l'acide rétinoïque du RARα. Il a été montré que la protéine de fusion PML-RARα réprime l'expression des gènes cibles de l'acide rétinoïque et provoque ainsi le blocage de la différenciation des cellules promyélocytaires. Seule l'administration de doses pharmacologiques d'acide rétinoïque permet la levée de la répression transcriptionnelle exercée par PML-RARα et la reprise de la différenciation cellulaire. En outre, la portion protéique PML de la protéine de fusion pourrait également intervenir dans le mécanisme du blocage de la voie de transduction par l'acide rétinoïque. Dans la mesure où PML fonctionne comme un inhibiteur de croissance et un agent apoptotique et qu'elle est nécessaire pour l'expression de certains gènes induit par l'acide rétinoïque, l'effet dominant négatif de PML-RARα sur PML pourrait permettre aux cellules d'acquérir une capacité de croissance, une résistance à l'apoptose et un arrêt de la différenciation.

Des études de biologie cellulaire sur PML ont montré que cette protéine possède une localisation particulière dans le noyau, dans des structures particulières appelées corps nucléaires. Il semble que le rôle de ces structures soit en relation directe avec le rôle antioncogène de PML. Dans les cellules malignes APL, la protéine PML-RARα provoque, par hétérodimérisation avec PML, la délocalisation de PML des corps nucléaires vers des structures microponctuées pouvant correspondre à des sites d'ancrage de PML-RARα sur la chromatine. Cette délocalisation pourrait bloquer la fonction pro-apoptotique de PML et son rôle dans la différenciation myéloïde. Plusieurs équipes ont montré que le traitement combiné à l'acide rétinoïque et à l'AS₂0₃ sur des lignées cellulaires qui expriment la protéine de fusion PML-RARα permet la dégradation des protéines de fusion en même temps qu'une relocalisation de PML sur les corps nucléaires. Cette réorganisation des corps nucléaires restaure les fonctions de PML et contribue à la reprise de la différenciation.

Finalement, la protéine chimère PML-RARα aurait donc un double effet dominant négatif, sur RARα et sur PML, en permettant à la fois aux cellules d'échapper à l'apoptose et de bloquer la différenciation des promyélocytes ainsi transformés.

Plus de 98% des patients atteints par la leucémie APL présentent la translocation t(15 ;17)(q22 ;q21) qui conduit à la formation de gènes fusionnés PML-RARα et RARα-PML. Il existe deux sous-types de protéines de fusion PML-RARα, les fusions S (short) et L (Long). La forme longue de la protéine de fusion PML-RARα correspondant à une protéine de 955 acides aminés représente la forme majoritairement exprimée et a donc été prise comme modèle dans cette étude (Annexes A, B et C). Cette protéine comporte les acides aminés 1 à 552 de la protéine PML fusionnés avec les acides aminés 59 à 462 du récepteur α de l'acide rétinoïque (RARα).

### II - Préparation et administration des oligonucléotides.

Des oligonucléotides RNA complémentaires correspondant à la séquence de la jonction du gène de la protéine de fusion, soit 10 nucléotides du gène PML et 10 nucléotides du gène RARα ont été synthétisés avec adjonction de deux déoxythymidines en 3' (Figure 1). Ces oligonucléotides ont été hybridés et l'obtention de l'oligonucléotide double-brin a été vérifiée sur gel d'acrylamide.

Les séquences des siRNA PML-RAR et contrôle utilisés (5'-3') sont données ci-dessous.

### Contrôle :

FW :
   [CAUGUCAUGUGUCACAUCUC]ARN[TT]ADN (SEQ ID NO.3)
REV :
   [GAGAUGUGACACAUGACAUG] ARN [TT] ADN (SEQ ID NO.4)

### PR:

Sens :
   [GGGGAGGCAGCCAUUGAGAC] ARN [TT] ADN (SEQ ID NO.5)
Antisens :
   [GUCUCAAUGGCUGCCUCCCC] ARN [TT] ADN (SEQ ID NO.6)

### III - Résultats.

Des fibrobastes NIH3T3 ont été cotransfectés avec de la lipofectamine par un vecteur d'expression de la protéine PML-RARα (100ng) et par 500ng de siRNA contrôle (C) ou dirigés contre PML-RARα (PR). 48 h après transfection, un western blot (Figure 1B) a été réalisé sur des extraits cellulaires totaux en utilisant un anticorps qui reconnaît la protéine RARα, entière ou sous forme de protéine de fusion.

La figure 1B montre que la transfection du siRNA PR inhibe très fortement l'expression de la protéine de fusion PML-RARα par rapport aux cellules transfectées avec le siRNA contrôle (C), sans modifier l'expression de la protéine RARα.

### EXEMPLE 2 INHIBITION DE L'ANGIOGENESE TUMORALE PAR LE VEGF.

### I - Introduction.

Le VEGF (vascular endothelial growth factor) est l'un des plus puissants facteurs angiogéniques identifiés. Ces facteurs sont surexprimés dans de nombreuses situations d'hypervascularisation pathologiques et notamment dans le développement tumoral. L'inhibition de cette angiogénèse permet de bloquer la croissance tumorale. Notre procédé a pour but d'inhiber l'angiogénèse tumorale en bloquant l'expression de l'un de ces facteurs angiogéniques et dans cet exemple celui du VEGF par les cellules tumorales.

### II - Préparation et administration des oligonucléotides.

Deux oligonucléotides RNA, complémentaires d'une région de la séquence codante du VEGF humain, conservée chez le rat et la souris ont été synthétisés. Deux déoxynucléotides (TT) ont été ajoutés en 3'
- Séquence des RNAi VEGF :
   5'[AUGUGAAUGCAGACCAAAGAA]RNA-TT[DNA] (SEQ ID NO. 7) 5'[UUCUUUGGUCUGCAUUCACAU]RNA-TT[DNA] (SEQ ID NO. 8)
- Séquence des RNAi contrôle :
   5'[CAUGUCAUGUGUCACAUCUC]RNA-TT[DNA] (SEQ ID NO. 9) 5'[GAGAUGUGACACAUGACAUg]RNA-TT[DNA] (SEQ ID NO. 10)

Ces oligonucléotides ou des oligonucléotides contrôle, dont la séquence ne présente aucune homologie avec les séquences répertoriées dans les bases de données, ont été hybridés et transfectés en utilisant le kit polyfect (Qiagen) dans des cellules d'un fibrosarcome de rat (cJ4) ou dans les cellules humaines du carcinome prostatique LNCaP.

### III - Résultats.

48h après transfection, une immunofluorescence indirecte a été réalisée pour détecter l'expression de la protéine dans les cellules. La figure 2A montre une inhibition massive de l'expression du VEGF.

Pour quantifier cet effet, un dosage du VEGF dans des cellules cJ4 transfectées en parallèle avec le RNAi contrôle ou avec le RNAi VEGF a été effectué par ELISA (quantikine, R&D). Les cellules ont été incubées 48h avant le dosage dans un milieu contenant 1% de sérum. Le dosage a été réalisé 4 jours et 6 jours après la transfection. Dans ces conditions, la figure 2B montre une inhibition de la sécrétion de VEGF de 85% à 4 jours et de 75% à 6 jours et de 60% à 13 jours dans les cellules transfectées avec le RNAi VEGF comparé à celles transfectées avec le RNAi contrôle (figure 2B).

L'effet de l'inhibition de l'expression de VEGF par les cellules tumorales a été testé *in vivo* : 3 jours après transfection, trois groupes de 4 souris nudes femelles de 4 semaines ont été injectés en sous cutané à raison de un million de cellules par souris : Le premier groupe a été injecté avec des cellules non transfectées, le second avec des cellules transfectées par le RNAi contrôle, le troisième par des cellules transfectées avec le RNAi VEGF. Aucune sélection des cellules transfectées n'a été effectuée avant l'injection.

La croissance tumorale a été suivie en mesurant le volume des tumeurs à intervalles réguliers (figure 2C).

Les figures 2C et 2D ne montrent aucune différence significative entre les tailles des tumeurs des groupes A et B. Une très forte réduction du volume tumoral est observée dans le groupe C. L'aspect des tumeurs, beaucoup plus blanches dans le groupe C (Fig 2D) traduit une diminution marquée de la vascularisation tumorale. Après sacrifice des animaux, au jour 12 après l'injection, les tumeurs ont été disséquées, fixées et une immunodétection du VEGF réalisée sur des coupes de ces tumeurs. On observe une très forte réduction de l'expression du VEGF dans les tumeurs du groupe C en comparaison avec celles du groupe B (Figure 2E).

Dans une autre expérience, des tumeurs ont été induites chez des souris nudes males par injection de cellules du carcinome prostatique LNCaP. 40 jours après injection, le volume des tumeurs ayant atteint 1 à 1,5cm³, les souris ont été divisées en deux groupes. Le premier groupe (4 souris) a reçu une injection intraveineuse dans la veine de la queue de 2 microgrammes de siRNA contrôle dans 100µl de PBS. Le second groupe a reçu une dose équivalente de siRNA VEGF dans les mêmes conditions. On observe que le siRNA VEGF, mais pas le siRNA contrôle, induit un arrêt transitoire de la croissance tumorale. (figure 4D).

### EXEMPLE 3: INHIBITION DE LA REPONSE HYPOXIQUE.

### I - Introduction.

Certaines tumeurs sont capables de se développer dans des conditions de forte anoxie. On observe très fréquemment dans les tumeurs des régions très peu vascularisées. Cette faible sensibilité à l'hypoxie a deux conséquences : d'une part un traitement anti-angiogénique a peu de chances d'être efficace sur ces tumeurs ou ces sous populations tumorales. D'autre part, cette faible vascularisation rend difficile la délivrance de molécules thérapeutiques. Le facteur de transcription Hif1α régule l'activité de plus de 100 gènes permettant la réponse hypoxique. L'inhibition de ce facteur de transcription dans les tumeurs hypoxiques a pour but de bloquer leur croissance.

### II - Préparation des oligonucléotides.

- RNAi Hif1α
   5'[CAUGUGACCAUGAGGAAAUGA]RNA-TT[DNA] (SEQ ID NO. 11)
   5'[UCAUUUCCUCAUGGUCACAUG]RNA-TT[DNA] (SEQ ID NO. 12)
- RNAi contrôle
   5'[GAUAGCAAUGACGAAUGCGUA]RNA-TT[DNA] (SEQ ID NO. 13)
   5'[UACGCAUUCGUCAUUGCUAUC]RNA-TT[DNA] (SEQ ID NO. 14)

### III - Résultats.

Le promoteur du VEGF contient un élément de réponse au facteur de transcription Hif1α. Pour tester in vitro l'effet d'un RNAi dirigé contre Hif1α, nous avons transfecté des cellules cJ4 avec un vecteur reporter VEGF-luciférase, seul ou en association avec un RNAi Hif1α ou contrôle.

24h après transfection, les cellules ont été incubées pendant 18h en milieu sans sérum, additionné ou non de chlorure de Cobalt 100µM afin de produire des conditions hypoxiques puis l'activité luciférase a été mesurée.

La figure 3 montre une inhibition complète de l'induction de la réponse du promoteur VEGF à l'hypoxie a été observée lorsque les cellules sont transfectées avec le RNAi Rif1α mais pas avec le RNAi contrôle.

### EXEMPLE 4 inhibition des formes sauvages ou mutantes des récepteurs des androgènes dans les carcinomes prostatiques.

### I - Introduction.

Les carcinomes prostatiques sont la deuxième cause de mortalité par cancer pour les hommes dans les pays industrialisés. En France, ils sont la cause de plus de 9500 morts par an. Les cellules épithéliales prostatiques sont dépendantes des androgènes pour leur croissance. Les carcinomes prostatiques sont initialement androgéno-dépendants. Une castration chimique permet dans un premier temps de bloquer la croissance du carcinome. Cependant dans tous les cas, ces carcinomes deviennent androgéno-indépendants et leur pronostic est alors très pessimiste. Cette androgéno-indépendance est due suivant les individus le plus souvent à une mutation du récepteur (lui conférant par exemple une réponse aux oestrogènes ou aux glucocorticoïdes) ou à une amplification du récepteur.

### II - Préparation des oligonucléotides.

Deux oligonucléotides ARN, complémentaire d'une région de la séquence codante du récepteur androgène (AR) non muté humain, ont été synthétisés. Deux déoxynucléotides (TT) ont été ajoutés en 3'. Dans d'autres expériences, des siRNA, dénommés LNCaP, reconnaissant spécifiquement la mutation du récepteur des androgènes (T877A) dans les cellules de carcinome prostatique humain LNCaP ont été utilisés.
- AR :
   5'[GACUCAGCUGCCCCAUCCACG]ARN-TT[ADN] (SEQ ID NO.15)
   5'[CGUGGAUGGGGCAGCUGAGUC]ARN-TT[ADN] (SEQ ID NO.16)
- Contrôle :
   5'[GAUAGCAAUGACGAAUGCGUA]ARN-TT[ADN] (SEQ ID NO.17)
   5'[UACGCAUUCGUCAUUGCUAUC]ARN-TT[ADN] (SEQ ID NO.18)
- LNCap :
   5'[GCAUCAGUUCGCUUUUGAC]ARN -TT[ADN] (SEQ ID NO.19)
   5'[GUCAAAAGCGAACUGAUGC]ARN -TT[ADN] (SEQ ID NO.20)

Plusieurs sous-clones de la lignée de carcinome prostatique humaine LNCaP ont été utilisés dans cette étude. La lignée originale, LNCaP, est androgéno-dépendante. Les cellules LN70, obtenues par passages répétés de la lignée LNCaP in vitro, ont une diminution de leur réponse aux androgènes. Le clone LNS5, obtenu après passage des cellules chez l'animal, est androgéno-résistant.

### III - Résultats.

Des cellules LNCaP ont été transfectées in vitro avec des siRNA AR ou des siRNA contrôle en utilisant l'agent de transfection polyfect (qiagen). 48h après transfection, les cellules ont été détachées de leur support. La moitié des cellules a été utilisée pour réaliser une détection par western blot du récepteur des androgènes, l'autre moitié a été remise en culture. Le récepteur des androgènes (bande à 110 kDa) n'est plus détectable par western dans les cellules transfectées par le siRNA AR (Figure 4A). Les cellules transfectées par le siRNA remises en culture se sont avérées incapables de poursuivre leur croissance, contrairement aux cellules transfectées par le siRNA contrôle.

Le niveau de réponse aux androgènes a été mesuré en transfectant différents clones cellulaires de la lignée LNCaP avec un vecteur rapporteur plaçant la séquence codante de la luciférase en aval d'un promoteur minimal flanqué de 4 répétitions de l'élément de réponse aux androgènes (4xARE). Après transfection, les cellules ont été incubées pendant 18h en absence de sérum et en présence ou en absence d'un analogue métaboliquement stable de la dihydro testostérone, le R1881 (NEN). Le rapport des activités luciférases dans ces deux conditions permet de mesurer le niveau de réponse aux androgènes du vecteur reporter.

Nous avons mesuré l'effet de la cotransfection dans ces cellules du RNAi contrôle ou du RNAi AR sur la réponse aux androgènes des différents clones de la lignée LNCaP.

La figure 4B montre une inhibition complète de la réponse aux androgènes dans les deux clones sensibles aux androgènes : LNCaP et LNCaP p70. Cette méthode ne permet pas de mesurer la réponse du clone LNS5, androgéno-résistant, au traitement par le RNAi AR.

Le récepteur des androgènes présent dans la lignée LNCaP est porteur d'une mutation. Nous avons utilisé deux siRNA différents pour inhiber sa synthèse, le siRNA AR précédemment utilisé et le siRNA LNCaP reconnaissant spécifiquement la mutation LNCaP. La réponse aux androgènes a été mesurée comme dans l'expérience 4B (Figure 4C).

Pour étudier l'effet de l'inhibition de l'expression du récepteur des androgènes sur la croissance tumorale in vivo des cellules de carcinome prostatique, des cellules du carcinome LNCaP, transfectées par un siRNA contrôle (groupe A) ou AR (groupe B) ont été injectées en sous cutané à des souris nudes males. La croissance tumorale a été suivie à intervalles réguliers. On observe que les tumeurs des animaux du groupe B ont démarré plus tardivement que celles du groupe A et que le volume des tumeurs du groupe B au 48e jour est nettement plus petit que celui des tumeurs du groupe A (Figure 4D).

Dans une autre expérience, des cellules LNCaP ont été injectées chez des souris nudes males. Lorsque, au 34e jour, les tumeurs ont atteint un volume compris entre 1,2 et 1,5 cm³, les souris ont reçu par voie intrapéritonéale une injection de 2µg de siRNA contrôle ou AR dans 100µl de PBS. Cette injection a été répétée au 40e jour. On observe que l'administration de siRNA AR entraîne un ralentissement de la croissance tumorale (Figure 4E).

### EXEMPLE 5 : Inhibition des formes sauvages ou mutantes de la protéine p53.

### I - Préparation des oligonucléotides.

Les trois siRNAs dont la séquence est indiquée ci-dessous ont été préparés, l'un dirigé contre la forme sauvage de la p53, et l'autre dirigé contre la forme mutée exprimée chez un patient et ayant donné lieu à l'établissement d'une lignée.

Cette mutation correspond à l'une des trois observées le plus fréquemment dans les tumeurs humaines.
- p53 sauvage :
   Sense : [GCAUGAACCGGAGGCCCAU]ARN[TT]ADN (SEQ ID NO.21) Anti: [AUGGGCCUCCGGWCAUGC]ARN[TT]ADN (SEQ ID NO.22)
- p53 MT1 (r248w) :
   Sense : [GCAUGAACUGGAGGCCCAU] ARN [TT] ADN (SEQ ID NO.23) Anti: [AUGGGCCUCCAGWCAUGC] ARN [TT] ADN (SEQ ID NO.24)
- p53 MT2 (r248w) :
   Sense: [***U***CAUGAACUGGAGGCCCAU] ARN [TT] ADN (SEQ ID NO.25)
   Anti: [AUGGGCCUCCAGWCAUG***A***] ARN [TT] ADN (SEQ ID NO.26)

Les nucléotides soulignés dans la p53 sauvage sont ceux qui sont mutés dans la forme mutante et qui sont en italique dans les séquences des formes mutées de la p53 mutée (p53 MT1 et MT2). Les bases en gras ci-dessus sont des mutations qui ont été introduites pour augmenter la spécificité.

### II - Résultats.

Comme montré sur la figure 5B, les cellules H1299-NCI, qui n'expriment pas la p53, ont été transfectées (en utilisant la lipofectamine) par des vecteurs d'expression (400 ng) de la p53 sauvage (WT) ou mutée (mt). Les siRNAs (en dose croissante : 0, 125 ng, 250 ng, 500 ng et 1000 ng ) dirigés contre la forme sauvage (WT), la forme mutée (MT1 et MT2), ou un siRNA irrelevant (C) ont été transfectés en même temps. Les cellules ont été collectées 24 heures après, et analysées par Western blot avec un anticorps dirigé contre la p53.

Comme montré sur la figure 5C, les cellules H1299-NCI, qui n'expriment pas la p53, ont été transfectées (en utilisant la lipofectamine) par des vecteurs d'expression (400 ng) de la p53 sauvage (WT), mutée (mt) ou un mélange des deux (WT+MT), comme indiqué. Les siRNAs (400 ng) dirigés contre la forme sauvage (WT), la forme mutée (MT1), ou un siRNA irrelevant (C) ont été transfectés en même temps. Les cellules ont été collectées 24 heures après, et analysées par Western blot (ib _{:} immunoblot) avec un anticorps dirigé contre la p53 (DO1, Santa Cruz), ou l'actine cellulaire (Sigma) pour contrôler la quantité de protéines utilisée dans le test.

Comme montré sur la figure 5D, des cellules U2OS (ostéosarcome humain exprimant une forme sauvage de la p53) ont été transfectées de façon stable soit par un vecteur exprimant une forme mutante de la P53 (R248W) soit par le vecteur vide correspondant (pCDNA3). Ces lignées ont été transfectées par les siRNA indiqués et l'expression des protéines indiquées a été détectée par western blot.

Dans tous les cas, le siRNA dirigé contre la forme mutée de la protéine inhibe la forme mutée et le siRNA dirigé contre la forme sauvage inhibe la forme sauvage. De plus, il n'y a pas de « réaction croisée », puisque le siRNA dirigé contre la forme sauvage n'a pas d'effet sur la forme mutée et réciproquement. Il faut noter que l'expression du mutant stabilise la protéine sauvage lorsqu'elle est co-exprimée. En conséquence, l'inhibition du mutant ramène, par cet effet indirect, la forme sauvage à son niveau de base, sans qu'il n'y ait inhibition de l'expression de la protéine.

Comme montré sur la figure 5E, les cellules utilisées dans la figure 5D ont été transfectées par les siRNA indiqués. Les cellules ont ensuite été soumises à un stress génotoxique par traitement à la doxorubicine (200 ng/ml) pendant 24h.La Figure 5E montre l'analyse du cycle cellulaire de ces cellules par incorporation de Iodure de propidium et analyse au FACS. Les cellules non transfectées avec la forme mutante, et donc n'exprimant que la forme sauvage (cellules PCDNA) montrent un fort pourcentage d'arrêt en G1 en présence de dauxorubicine. Le traitement de ces cellules par le siRNA sauvage, en diminuant la p53 sauvage, réduit cet arrêt en G1. Les cellules qui expriment la forme mutée et sauvage (R248W) s'arrêtent peu en G1 en présence de dauxorubicine, montrant que la forme mutée inhibe l'activité de la forme sauvage. Quand ces cellules sont traitées avec le siRNA mt1, elles récupèrent une capacité normale (à comparer avec les contrôles PCDNA non traitées) de s'arrêter en G1, montrant la restauration de l'activité p53 sauvage dans ces cellules.

Comme montré sur les figures 5F, G, H, les cellules MDA 087 (provenant d'un patient atteint d'un syndrome de Li Fraumeni et exprimant le mutant R248W) ont été transfectées avec un siRNA dirigé contre la forme mutante (MT1) de la p53, ou encore avec un siRNA irrelevant (C) (1,6 µg). L'expression de la P53 a été détectée dans ces cellules par western blot (Figure 5F), les ARN messagers ont été mesurés par PCR quantitative (Light cycler, Roche) (Figure 5G) ou immunofluorescence (Figure 5H).

Les cellules MDA 087 ont été transfectées avec un siRNA reconnaissant la forme sauvage (WT) ou la forme mutée de la p53 (mtl) ou encore par un siRNA contrôle puis soumises à un stress génotoxique par traitement à la doxorubicine (200 ng/ml) pendant 24h. L'expression de la forme mutante de la p53 a été détectée par western blot dans les cellules. On observe que les cellules ayant reçu le siRNA mt1 ne sont pas capables de stabiliser la p53 en réponse à la dauxorubicine (Figure 5I).

La figure 5J montre l'effet des siRNA mt1 et mt2 dans des cellules qui expriment les formes sauvages et mutées de la p53. Les cellules H1299-NCI, qui n'expriment pas la p53, ont été transfectées (en utilisant la lipofectamine) par un vecteur reporter comportant le gène de la luciférase sous contrôle d'un élément de réponse à la p53 et des vecteurs d'expression (400 ng) de la p53 sauvage (WT), mutée (MT) ou un mélange des deux (WT+MT), comme indiqué. Les siRNAs (400 ng) dirigés contre la forme sauvage (WT), la forme mutée (mtl, mt2), ou un siRNA irrelevant (C) ont été transfectés en même temps. Les cellules ont été collectées 24 heures après et analysées pour l'expression de la luciférase. La p53 sauvage seule active le vecteur reporter, et la co-expression de la forme mutante inhibe cette activité. La co-transfection du siRNA sauvage inhibe l'expression de la protéine sauvage et donc l'activation résiduelle du gène reporter. La co-transfection des siRNAs mt1 ou mt2, au contraire, restaure cette activation en bloquant sélectivement l'expression de la forme mutée et en empêchant l'effet de transdominant négatif qu'elle exerce sur la forme p53 sauvage.

La figure 5K montre un résultat similaire sur l'expression d'une des cibles de la p53, la protéine inhibitrice de la prolifération cellulaire p21, dans des cellules traitées comme dans la figure 5F. L'expression de la p21, détectée par western blot est activée par la p53 sauvage et inhibée lorsque le mutant est co-exprimé. Cette inhibition est levée en présence du siRNA mt1.

### EXEMPLE 6 Inhibition de la protéine virale E6.

### I - Préparation des oligonucléotides.

Un siRNA dirigé contre la protéine E6 de HPV a également été préparé. Il répond à la séquence suivante :
- HPV-16-S2
   Sens: 5' [CCACAGUUAUGCACAGAGC] ARN [TT] ADN (SEQ ID NO.27)
   Anti: 5' [GCUCUGUGCAUAACUUGG] ARN [TT] ] ADN (SEQ ID NO.28)

### II - Résultats.

Comme montré sur la figure 6B, des cellules CasKi et SiHA, exprimant toutes deux la protéine E6 de HPV ont été transfectées avec les siRNA indiqués, traitées ou non comme indiqué par la doxorubicine et analysées par western blot en utilisant les anticorps indiqués. Le traitement des cellules par le siRNA E6 induit une augmentation de l'expression de P53. Cette expression de p53 se traduit par une augmentation de l'expression de la protéine p21.

Comme montré sur la figure 6C, le cycle cellulaire de cellules SiHA traitées comme dans la figure 6B a été analysé par FACS. La figure représente une expérience caractéristique. On observe une augmentation de cellules en phase G1 (Figure 6D) dans les cellules traitées par le siRNA E6, augmentation qui est également observée dans ces cellules lorsqu'elles sont traitées par la doxorubicine.

### EXEMPLE 7 Effet des oligonucléotides ARN/ARN et des hybrides ADN/ARN.

### I - Introduction.

L'invention envisage l'utilisation d'oligonucléotides hybrides ADN/ARN comme alternative aux oligonucléotides ARN/ARN pour inhiber spécifiquement l'expression d'un gène. Dans le cas des hybrides ADN/ARN, le brin sens est préférentiellement de nature ADN et le brin anti-sens de nature ARN. Les autres aspects ayant trait notamment à la taille des oligonucléotides, à la nature des extrémités 3' et au mode de synthèse sont les mêmes que pour les oligonucléotides ARN/ARN. Les applications de ces hybrides ADN/ARN sont identiques à celles précédemment décrites pour les siRNA ARN/ARN, notamment en ce qui concerne les applications thérapeutiques, à visées diagnostic ou de validation de gènes. Les doses d'oligonucléotides employées pour obtenir les mêmes effets avec les hybrides ADN/ARN et les ARN/ARN peuvent cependant être différentes

### II - Préparation des oligonucléotides.

Le brin sens est celui dont la séquence est identique à celle de l'ARN messager. Le brin antisens est le brin complémentaire du brin sens. Par convention, dans un duplexe, la nature des brins est indiquée dans l'ordre sens/antisens. Ainsi par exemple, un hybride ADN/ARN, noté D/R est un oligonucléotide dont le brin sens est de nature ADN et le brin antisens, est de nature ARN et de séquence complémentaire de l'ARN messager.

Dans les expériences décrites, les oligonucléotides dont la séquence est indiquée ci-dessous ont été utilisés.
- Pour la GFP :
   GAP
   Sens : [GCAAGCTGACCCTGAAGTTCAT] ADN (SEQ ID NO.29)
   Antisens : [GAACUUCAGGGUCAGCUUGCCG]ARN (SEQ ID NO.30) Contrôle GFP :
   Sens : [CAUGUCAUGUGUCACAUCUC] ARN [TT] ADN (SEQ ID NO.31)
   Antisens : [GAGAUGUGACACAUGACAUG]ARN[TT]ADN (SEQ ID NO.32)
   - Pour le LNCaP : Les bases soulignées ci-dessous correspondant à la mutation du récepteur des androgènes exprimée dans les cellules du carcinome prostatique humain (LNCap).

### LNCaP :

Sens :
   [GCATCAGTTCGCTTTTGACTT] ADN (SEQ ID NO.33)
   [GCAUCAGUUCGCUUUUGAC] ARN-TT [ADN] (SEQ ID NO.34)
Antisens :
   [GTCAAAAGCGAACTGATGCTT] ADN (SEQ ID NO.35)
   [GUCAAAAGCGAACUGAUGC] ARN-TT [ADN] (SEQ ID NO.36)
Contrôle LNCaP :
   Sens :
      [GUUCGGUCUGCUUACACUA] ARN-TT [ADN] (SEQ ID NO.37)
   Antisens :
      [UAGUGUAAGCAGACCGAAC] ARN-TT [ADN] (SEQ ID NO.38)

### - Pour la P53 :

### Les brins ADN des hybrides notés H1 comportent des bases ARN (U, soulignées).

La mutation présente dans les oligonucléotides MT1 est indiquée en italique.
WT :
   Sens: 5' [GCAUGAACCGGAGGCCCAU] ARN [TT] ADN (SEQ ID NO.39)
   Anti: 5' [AUGGGCCUCCGGUUCAUGC] ARN [TT] ADN (SEQ ID NO.40)
wt H1 D/R :
   Sens: 5' [GCA**U**GAACCGGAGGCCCA**U**TT] ADN (SEQ ID NO.41)
   Anti: 5' [AUGGGCCUCCGGWCAUGC] ARN [TT] ADN (SEQ ID NO.42)
wt H1 R/D :
   Sens: 5' [GCAUGAACCGGAGGCCCAU] ARN [TT] ADN (SEQ ID NO.43)
   Anti: 5'[A**U**GGGCC**U**CCGG**UU**CA**U**GCTT]ADN (SEQ ID NO.44)
wt H2 D/R :
   Sens: 5' [GCATGAACCGGAGGCCCATTT] ADN (SEQ ID NO.45)
   Anti: 5' [AUGGGCCUCCGGUUCAUGC] ARN [TT] ADN (SEQ ID NO.46)
wt H2 R/D :
   Sens: 5' [GCAUGAACCGGAGGCCCAU] ARN [TT] ADN (SEQ ID NO.47)
   Anti: 5' ATGGGCCUTCCGGTTCATGCTT] ADN (SEQ ID NO.48)
Mtl (r248w) ** :
   Sens: 5' [GCAUGAAC***U***GGAGGCCCAU]ARN [TT] ADN (SEQ ID NO.49)
   Anti: 5' [AUGGGCCUCC***A***GUUCAUGC] ARN [TT] ADN (SEQ ID NO.50)
Mtl H1 D/R :
   Sens : 5' [GCA**U**GAAC***U***GGAGGCCCA**U**TT] ADN (SEQ ID NO.51)
   Anti : 5' [AUGGGCCUCC***A***GUUCAUGC] ARN [TT] ADN (SEQ ID NO.52)
Mtl H1 R/D :
   Sens: 5' [GCAUGAAC***U***GGAGGCCCAU] ARN [TT] ADN (SEQ ID NO.53)
   Anti: 5' A**U**GGGCC**U**CC***A***G**UU**CA**U**GCTT] ADN (SEQ ID NO.54)
Mtl H2 D/R :
   Sens: 5' [GCATGAAC***T***GGAGGCCCATTT] ADN (SEQ ID NO.55)
   Anti: 5' [AUGGGCCUCC***A***GUUCAUGC] ARN [TT] ADN (SEQ ID NO.56)
Mtl H2 R/D :
   Sens: 5' [GCATGAAC***T***GGAGGCCCAT] ARN [TT] ADN (SEQ ID NO.57)
   Anti: 5' [AUGGGCCUCC***A***GUUCAUGCTT] ADN (SEQ ID NO.58)

### II - Résultats.

### 1) Inhibition de la GFP (Green Fluorescent Protein) par les hybrides ADN/ARN.

Les siRNAs contrôle (R/R) ou GFP (D/R) en doses croissantes ont été introduits par transfection en utilisant le kit Polyfect dans les myoblastes de souris C2C12, en même temps qu'un vecteur d'expression de la GFP. Le niveau de GFP a été suivi par Western Blot (Figure 7A) et par mesure directe de la fluorescence émise par la GFP à l'aide d'un fluorimètre (Figure 7B). On observe une forte inhibition (jusqu'à 80%) de l'expression de la GFP par les siRNA hybrides ADN/ARN.

### 2) Inhibition du récepteur des androgènes par les hybrides ADN/ARN.

Les cellules LNCaP ont été transfectées avec un vecteur reporteur mettant la luciférase sous le contrôle d'un promoteur contenant 4 éléments de réponse au récepteur des androgènes. 24h plus tard, Les siRNA R/R, D/R ou R/D indiqués sur la figure ont été transfectés par l'agent de transfection Transit-tKO (Mirus) à raison de 250ng de chaque double brin pour 80000 cellules. Les cellules ont été incubées en milieu complet contenant des androgènes et l'activité luciférase, normalisée par rapport à la quantité de protéines de chaque échantillon, a été mesurée 24h plus tard (Figure 7C). Les hybrides R/D n'ont pas montré d'activité inhibitrice dans cette expérience. Les hybrides LNCaP D/R inhibent aussi efficacement que les siRNA R/R le récepteur des androgènes.

### 3) Inhibition de la p53 par les hybrides ADN/ARN.

La figure 7D montre que les hybrides H1 D/R sont aussi efficaces que les R/R pour inhiber l'expression des gènes. Les cellules H1299-NCI, qui n'expriment pas la p53, ont été transfectées (en utilisant la lipofectamine) par des vecteurs d'expression (400 ng) de la p53 sauvage (WT), mutée (MT) ou un mélange des deux (WT+MT), comme indiqué. Un vecteur CMV-GFP a été transfecté également comme contrôle interne. Les siRNAs (400 ng) dirigés contre la forme sauvage (WT), la forme mutée (MT), ou un siRNA irrelevant (CTRL) ont été transfectées en même temps. Les cellules ont été collectées 24 heures après, et analysées par Western blot avec un anticorps dirigé contre la p53 (DO1, Santa Cruz), ou la GFP (Santa-Cruz) pour contrôler l'efficacité de transfection. Note : l'expression de la forme mutée de la protéine stabilise la forme sauvage.

La figure 7E montre que les hybrides H2 D/R sont aussi efficaces que les R/R pour inhiber l'expression des gènes. Les cellules H1299-NCI, qui n'expriment pas la p53, ont été transfectées (en utilisant la lipofectamine) par des vecteurs d'expression (400 ng) de la p53 sauvage (WT), mutée (MT) ou un mélange des deux (WT+MT), comme indiqué. Les siRNAs (400 ng) dirigés contre la forme sauvage (WT), la forme mutée (MT), ou un siRNA irrelevant (C) ont été transfectées en même temps. Les cellules ont été collectées 24 heures après, et analysées par Western blot avec un anticorps dirigé contre la p53 (DO1, Santa Cruz) .

### EXEMPLE 8 Administration in vivo de siRNA par différentes voies.

Des cellules tumorales exprimant la luciférase de façon stable ont été injectées en sous cutané à des souris nudes (1 million de cellules dans le flanc droit). Au 8e jour de la croissance tumorale, les tumeurs ayant un volume moyen de 200 mm3 ont été injectées soit avec des siRNA contrôles (séquence mélangée de HIF1 alpha, voir exemple 3) soit avec un siRNA dirigé contre la luciférase. Les siRNA contrôles (3 µg/souris) ont été injectés dans un volume de 50µl de PBS par voie sous cutanée dans le flanc de l'animal.

Les siRNA Luciférase ont été injectés à raison de 3µg/souris (3 animaux dans chaque groupe) dans 50 µl de PBS par voie sous cutanée (sc), ou par voie intrapéritonéale (ip) ou par voie intra-veineuse(iv) (veine de la queue) ou par voie intratumorale (it). Dans ce dernier cas, les siRNA luciferase (3µg/souris) ont été dilués dans 20µl de PBS seulement.

Trois jours après l'injection des siRNA, les animaux ont été sacrifiés, les tumeurs ont été prélevées, homogénéisées à l'aide d'un broyeur polytron. Sur les homogénats, un dosage de protéines et une mesure de l'activité luciférase dans un luminimètre ont été réalisés.

Les résultats représentés à la figure 8 montrent l'activité luciférase rapportée à la quantité de protéine.

**Tableau 2**

| Annexe A(1) | | | |
|---|---|---|---|
| **Maladie** | **Protéine de fusion** | **Translocation chromosomique** | **références** |
| APL (acute promyelocytic leukaemia) | PML-RARalpha | t(15;17)(q22;q21) | De The et al. Cell 1991, 66:675 |
| | PLZF-RARalpha | t(11;17)(q23;q21) | Chen Z et al. EMBO J 1993, 12:1161 |
| | NPM-RARalpha | t(5;17)(q32;q12) | Redner RL et al. Blood 1996, 87:882 |
| | NuMA-RARalpha | t(5;17)(q13;q21) | Wells RA et al. Leukemia 1996, 10:735 |
| | STATSbeta/RARalpha | | Arnould C et al. Hum. Mol Genet. 1999, 8:1741 |
| ALL (acute lymphoblastic | TEL-AML1 | t(12;21) (p13;q22) | |
| leukaemia) | BCR/ABL | t(9;22)(q34;q11) | |
| | MLLIAF4 | t(4;11)(q21;q23) | Domer PH et al. Proc Natl Acad Sci USA 1993, 90:7884-8 |
| | ALL-translocation | t(12:21)(q12;q22) | |
| | CALM/AF10 | t(10;11)(p12-p13;q14-q21) | Dreyling MH et al. Proc Natl Acad Sci USA 1996, |
| | ALL1/AF4 | t(4;11) | **93**:4804 |
| | E2A/HLF | t(17;19)(q22;p13) | Janssen JW et al. Blood 1994, 84:3835 |
| | | | Hunger SP et al. *Genes Dev* 1992, **6**:1608 |
| AML (acute myeloid leukemia) | TLS/FUS-ERG | t(16;21)(p11;q22) AML(M7) | Ichikawa H et al. Cancer Res 1994, 54:2865 |
| | MLL-AF10 | t(10;11)(p12-p13;q23); | Borkhardt A et al. Leukemia 1995, 9:1796 |
| | MLL-ABI1 | t(10;11) | Shibuya et al. Genes Chromosomes Cancer 2001, 32:1 |
| | HLXB9-ETV6 | t(7;12)(q36;p13) | Beverloo et al. Cancer Res 2001, 61:5374 |
| | MLL-ELL | t(11;19)(q23;p13.1) | Rubnitz JE et al. Blood 1996, 87:4804 |
| | CBFbeta/MYH11 | inv[16] | Tobal K et al. Br J Haematol 1995, 91:104 |
| | AML1-MTG8 | t(8;21) | Miyoshi et al. EMBO J 1993, 12:2715 |
| | TEL-TRKC | t(12;15)(p13;q25) | Eguch et al. Blood, 1999, 93:1355 |
| | AML1/ETO | t(8;21) | Kusec R et al. Leukemia, 1994, 8:735 |
| | CALM/AF10 | t(10;11) (p12-p13;q 14-q21 ) | Dreyling MH et al. Proc Natl Acad Sci USA 1996, |
| | ETV6-BTL | t(4;12)(q11-q12;p13). | **93**:4804 |
| | CBFbeta-SMMHC | inv(16)(p13;q22) | Cools et al. Blood 1999, 94:1820 |
| | FUS/ERG | t(16;21)(p11;q22) | Wijmenga C et al. Proc Natl Acad Sci U S A 1996, |
| | DEK/CAN | t(6;9)(p23;q34). | 93:1630 |
| | MLL-AF9 | t(9;11)(p22;q23) | Panagopoulos I et al. Genes Chromosomes Cancer, 1994, |
| | MLL-ENL | (11q23) | 11:256 |
| | MLL-AF4 | t(4;11)(q21;q23) | on Lindem M et al. Mol Cell Biol, 1992, 12:1687 |
| | MLL-AF6 | t(6;11)(q27;23) | Super HJ et al. Blood, 1995, 85:855 |
| | MLL-AF17 | t(11;17)(q23;q21) | Schreiner SA et al. Leukemia 1999, 13:1525 |
| | MLL-AFX | t(X;11)(q13;q23). | Domer PH et al. Proc Natl Acad Sci U S A 1993, 90:7884 |
| | MLL-AF1p | | Tanabe S et al. Genes Chromosomes Cancer 1996, 15:206 |
| | MLL-AF1q | t(1;11)(q21;q23) | Prasad R et al. Proc Natl Acad Sci U S A 1994, 91:8107 |

| Annexe A(2) | | | |
|---|---|---|---|
| | MLL self | | Borkhardt A et al. Oncogene 1997,14:195 |
| | MLL-CBP | t(11;16)(q23;p13) | So CW et al. Leukemia 2000, 14:594 |
| | AML1-ETO | t(8;21) | Busson-Le Coniat M et al. Leukemia 1999, 13:302 |
| | | | So CW et al. Cancer Res 1997, 57:117 |
| | | | Taki T et al. *Blood* 1997, **89**:3945 |
| | | | Erickson P et al. *Blood* 1992, **80**:1825 |
| MDS/AML (myelodysplasia/acute myeloid leukemia) | NPM-MLF1 | t(3;5)(q25.1;q34) | Yoneda-Kato N et al. Oncogene 1996, 12:265 |
| CML (chronic myelogenous leukemia) | Bcr-Ab1/p210 | | Ben-Neriah Y et al. Science 1986, 233:212 |
| | AML1-MDS1-EVI1 | t(3;21)(q26;q22) | Fears S et al. Proc Natl Acad Sci U S A 1996, 93:1642 |
| | (AME) | | |
| BpALL (cell acute lymphoblastic leukemia) | TEL-AML1 | t(12;21) (p13;q22) | Golub TR et al. Proc Natl Acad Sci U S A 1995, 92:4917 |
| MPD (myeloproliferative disease) | TEL-JAK2 | t(9;12)(p24;q13) | Lacronique et al. Science 1997, 278 :1309 |
| | TEL-PDGFbetaR | t(5;12)(q33;p13) | Jousset C et al. EMBO J, 1997, 16 :69 |
| | TEL-TRKC | t(12;15)(p13;q25) | Eguch et al. Blood, 1999, 93:1355 |
| CMML (chronic myelomonocytic leukemia) | involving PDGFbetaR | t(5;17)(q33;p13) | Magnusson et al. Blood 2001 98:2518 |
| | HIP1/PDGFbetaR | t(5;7)(q33;q11.2) | Ross TS et al. Blood 1998, 91:4419 |
| | TEL/PDGFbetaR | t(5;12)(q33;p13) | Tomasson MH et al. Blood 1999, 93:1707 |
| MALT (gastric mucosa-associated lymphoid tissue lymphoma) | API2-MALT1 | t(11;18)(q21;q21) | Motegi M et al. Am J Pathol 2000, 156:807 |
| ALCL (anaplastic large cell lymphoma) | NPM-ALK | t(2;5)(p23;q35) | Waggott W et al. Br J Haematol 1995, 89:905 |
| | SU-DHL-1 | t(2;5) | Siminovitch KA et al. Blood 1986, 67:391 |
| | ATIC-ALK | inv(2)(p23q35) | Colleoni GW et al. Am J Pathol 2000, 156:781 |
| | ALK-related translocation | t(2;17)(p23;q25) | Maes et al. Am J Pathol 2001, 158:2185 |
| MPD (myeloproliferative disease) | NUP98-HOXA9 | t(7;11)(p15;p15) | Nakamura T et al. Nat Genet 1996, 12:154 |

| Annexe A(3) | | | |
|---|---|---|---|
| APP (amyloid precursor protein) in sporadic Alzheimer's disease (AD) or Down's syndrome | APP+1 (38-kDa) | | Hersberger et al. J Neurochem 2001 76(5):1308-14 |
| primary pleural monophasic synovial sarcomas (SS) | SYT-SSX1 | t(X;18)(p11.2;q11.2) | Crew AJ et al. EMBO J 1995,14:2333 |
| | SYT-SSX2 | t(X;18)(p11.2;q11.2) | Crew AJ et al. EMBO J 1995,14:2333 |
| Dermatofibrosarcoma protuberans (DP) | COL1A1/PDGFB rearrangement | t(17;22) | O'Brien KP et al. Genes Chromosomes Cancer 1998, 23:187 |
| ARMS (pediatric alveolar rhabdomyosarcoma) | EWS-FLII | | Athale et al. J Pediatr Hematol Oncol 2001, 23:99 |
| | EWS-ERG | t(11;22)(q24;q12) | Sorensen PH et al. Nat Genet 1994, 6:146 |
| ESFT (Ewing sarcoma family of tumors) | PAX3-FKHR | t(2;13)(q35;q14) | Fredericks WJ et al. Mol Cell Biol 1995,15:1522 |
| | PAX7-FKHR | t(1;13)(p36;q14) | Barr FG et al. Hum Mol Genet 1996, 5:15 |
| DSRCT (desmoplastic small round cell tumors) | EWS-WTI | t(11:22)(p13:q12) | Benjamin et al. Med Pediatr Oncol 1996 27(5):434-9 |
| | EWS/FI-1 | t(11-22) (q24;q12) | Fidelia-Lambert et al. Hum Pathol 1999, 30:78 |
| MM (multiple myeloma) | IGH-MMSET | t(4;14)(p16.3;q32) | Malgeri et al. Cancer Res 2000 60:4058 |
| MPD (stem cell myeloproliferative disorder) | FGFR1-CEP110 | t(8;9)(p12;q33) | Guasch et al. Blood 2000 95:1788 |
| Ewing sarcoma (ES)-peripheral primitive neuroectodermal tumor (pPNET) | EWS-FEV | t(2;22)(q13;q22,t(3;18) (p21;q23) | Llombart-Bosch et al. Diagn Mol Pathol 2000, 9:137 |
| | EWS-FLI1 | t(11;22;14)(q24;q12;q11) | Bonin G et al. Cancer Res 1993, 53:3655 |
| | EWS-ERG | t(21;22)(q22;q12) | Sorensen PH et al. Nat Genet. 1994, 6:146 |
| | ETV6/CBFA2 | t(12;21)(p12;q22) | Fears S et al. Genes Chromosomes Cancer 1996, 17:127 |
| MLS (myxoid liposarcomas) | FUS/CHOP | t(12;16)(q13;p11) | Rabbitts TH et al. Nat Genet 1993, 4:175 |
| | EWS/CHOP | t(12;22;20)(q13;q12;q11) | Zinszner H et al. Genes Dev 1994, 8:2513 |

| Annexe B(1) | | | | | | | | | |
|---|---|---|---|---|---|---|---|---|---|
| Codon | Event | Codon | Event | Codon | Event | Codon | Event | Codon | Event |
| 248 | G->A | 129 | C->A | 189 | C->G | 217 | Stop at 219 | 202 | Ins |
| 248 | C->T | 281 | A->G | 290 | G->T | 239 | Stop at 259 | 247 | Ins |
| 282 | C->T | 293 | Fr. | 136 | Stop at 169 | 187 | G->C | 171 | Ins |
| 175 | G->A | 157 | DEL | 201 | Stop at 208 | 273 | Stop at 343 | 203 | Ins |
| 196 | C->T | 161 | C->A | 275 | Stop at 344 | 182 | C->T | 290 | Stop at 303 |
| 213 | G->A | 195 | A->T | 132 | Stop at 148 | 263 | Stop at 344 | 233 | del |
| 234 | T->C | 197 | G->C | 176 | Stop at 180 | 307 | Stop at 344 | 210 | Stop at 244 |
| 237 | T->G | 342 | Fr. | 191 | del | 261 | Stop at 344 | 201 | G->A |
| 244 | G->T | 135 | G->C | 218 | G->A | 285 | Stop at 344 | 92 | Ins |
| 256 | A->G | 145 | T->A | 234 | T->A | 159 | G->A | 44 | Fr. |
| 259 | A->G | 276 | G->C | 136 | C->A G->C/G- | 168 | C->T | 109 | ins G->A/G- |
| 260 | T->G | 173 | G->T | 245 | >A | 230 | C->T | 279 | >A |
| 245 | G->T | 270 | T->G | 126 | Stop at 148 | 228 | A->C | 168 | Stop at 170 |
| 278 | C->T | 158 | G->C | 259 | G->C | 230 | C->A | 153 | Stop at 178 |
| 134 | T->A | 152 | Fr. | 171 | G->C | 287 | Stop at 300 | 247 | C->A |
| 194 | C->T | 132 | G->T | 197 | T->A | 269 | Stop at 343 | 272 | Stop at 305 |
| 273 | G->A | 288 | A->C | 236 | T->G | 227 | Stop at 227 | 137 | Stop at 169 |
| 309 | C->T | 247 | A->T | 239 | C->A | 231 | Stop at 238 | 148 | Stop at 180 |
| 274 | T->A | 273 | G->C | 288 | A->T | 275 | G->C | 157 | Stop at 180 |
| 156 | G->C | 283 | G->C | 161 | Fr. | 142 | T->C | 191 | Stop at 208 |
| 245 | G->A | 109 | Fr. | 164 | Fr. | 312 | C->G | 243 | Stop at 260 |
| 193 | A->G | 174 | G->C | 142 | Stop at 148 | 282 | C->G/G->C | 251 | C->A |
| 229 | T->A | 300 | C->G | 240 | A->C | 235 | Stop at 244 | 242 | C->A |
| 237 | G->A | 205 | A->G | 137 | T->C | 156 | Stop at 179 | 244 | C->A |
| 277 | G->T | 224 | G->T | 100 | G->A | 207 | A->G | 239 | C->G |
| 194 | T->G | 168 | A->T | 106 | C->G | 179 | Stop at 246 | 142 | T->A |
| 242 | G->C | 167 | Fr. | 215 | A->G | 210 | Stop at 214 | 248 | C->A |
| 246 | G->C | 136 | C->G | 246 | Fr. | 315 | T->C | 177 | Stop at 246 |
| 68 | G->T | 164 | A->C | 117 | G->A | 229 | Stop at 229 | 296 | Fr. |
| 147 | T->A | 179 | C->G | 271 | Stop at 344 | 167 | A->C | 303 | C->T |
| 151 | C->A | 187 | G->T | 324 | T->G | 256 | Stop at 343 | 140 | C->A |
| 209 | A->T | 201 | Stop at 246 | 346 | Fr. | 176 | Stop at 176 | 268 | C->T |
| 213 | C->T | 213 | C->A | 174 | Stop at 246 | 309 | Stop at 336 | 254 | A->C |
| 214 | A->G | 238 | G->T T->G/C- | 170 | Stop at 177 | 270 | Stop at 344 | 291 | G->A |
| 248 | G->T | 113 | >T | 234 | T->G | 129 | G->A | 139 | Stop at 148 |
| 266 | G->T | 143 | G->T | 354 | A->G | 46 | Stop at 50 | 251 | A->G |
| 273 | C->T | 160 | G->T | 259 | Stop at 344 | 160 | T->G | 221 | Stop at 224 |
| 273 | G->T | 198 | G->T | 319 | Stop at 344 | 56 | A->T | 237 | A->T/G->T |
| 282 | C->G | 203 | G->T | 332 | Ins | 74 | Stop at 144 | 234 | Stop at 234 |
| 334 | G->T | 238 | T->A | 340 | Ins | 118 | A->G | 215 | T->A |
| 342 | C->T | 272 | G->C | 177 | del | 257 | del | 191 | T->A |
| 132 | A->C | 276 | Ins | 179 | T->A | 192 | G->T | 290 | C->A |
| 249 | G->C | 277 | T->G | 190 | Stop at 246 | 294 | G->T/G->C | 60 | A->T |
| 280 | G->A | 302 | G->T | 254 | Ins | 240 | del | 93 | C->A |
| 285 | G->A | 131 | C->G | 194 | C->A | 306 | G->T C->G/G- | 143 | T->C/G->C |
| 241 | C->T | 168 | A->G | 172 | T->A G->T/T- | 175 | >A | 319 | G->T |
| 249 | G->T | 258 | G->T | 173 | >G | 246 | Stop at 261 | 110 | Stop at 122 |
| 158 | G->A | 278 | C->A | 261 | T->C | 279 | Stop at 305 | 190 | T->G |
| 163 | T->C | 285 | G->C | 266 | A->G | 146 | T->G/G->T | 192 | C->G |
| 176 | G->A | 287 | G->A | 199 | Fr. | 154 | Stop at 169 | 126 | T->G/A->G |

| Annexe B(2) | | | | | | | | | |
|---|---|---|---|---|---|---|---|---|---|
| 206 | Fr. | 294 | Fr. | 236 | C->G | 132 | del | 273 | Stop at 305 |
| 234 | A->G | 236 | Fr. | 168 | C->G | 175 | Stop at 175 | 266 | Stop at 344 |
| 238 | G->A A->G/T- | 301 | Ins | 201 | G->C | 152 | Stop at 165 | 64 | Stop at 122 |
| 254 | >A | 228 | A->G | 203 | Stop at 208 C->A/C- | 260 | Stop at 262 | 103 | C->A |
| 287 | G->T | 175 | Fr. | 250 | >G | 194 | Stop at 246 | 343 | A->G |
| 143 | Fr. | 282 | G->T | 283 | G->T | 170 | C->A | 317 | Stop at 344 |
| 205 | A->T | 152 | Stop at 180 | 256 | C->A | 213 | C->G | 125 | Stop at 148 |
| 262 | Fr. | 177 | C->G | 245 | C->T | 213 | A->C | 239 | A->G/C->T |
| 171 | G->T | 216 | T->A | 342 | G->C | 232 | A->G | 119 | Fr. |
| 126 | C->G | 232 | T->G | 243 | G->A | 294 | G->C | 162 | T->G/C->G |
| 138 | Fr. | 275 | Stop at 305 | 296 | A->G | 240 | T->A | 12 | C->A/C->G |
| 223 | C->T | 216 | G->T | 68 | G->C | 223 | C->G | 247 | C->G |
| 274 | G->T | 137 | Fr. | 102 | C->T | 171 | A->C | 190 | T->A |
| 218 | Fr. | 251 | T->G | 104 | G->C | 328 | T->G | 240 | T->C |
| 246 | A->G | 252 | Ins | 117 | G->C | 150 | C->T | 315 | C->T |
| 250 | Fr. | 254 | T->A | 175 | Stop at 246 | 252 | C->A | 313 | C->T |
| 143 | T->C | 49 | G->C | 138 | Stop at 169 | 256 | Stop at 342 | 42 | G->T |
| 173 | G->A | 53 | G->T | 215 | T->G | 200 | Stop at 246 | 73 | G->T |
| 242 | G->T | 60 | C->T | 247 | Stop at 262 | 239 | del | 231 | C->A |
| 190 | Fr. | 202 | G->T | 104 | C->T | 215 | A->T | 172 | Stop at 173 |
| 246 | T->C | 204 | A->G | 297 | A->C | 147 | Stop at 169 | 211 | Stop at 214 |
| 157 | G->T | 265 | T->A | 252 | T->C | 276 | G->A | 150 | Stop at 180 |
| 239 | Fr. | 135 | T->C | 276 | C->T | 210 | A->C | 145 | C->A |
| 240 | A->T | 147 | G->A | 349 | A->C G->A/T- | 182 | T->C | 335 | C->G G->A/A- |
| 238 | T->C | 153 | C->T | 173 | >G/G->T | 161 | G->A/C->T | 285 | >G |
| 35 | Stop at 42 | 170 | G->T | 225 | G->C | 83 | C->A | 85 | Stop at 122 |
| 47 | C->T | 260 | C->T | 250 | del | 304 | Stop at 344 | 98 | C->T |
| 89 | Fr. | 255 | Stop at 263 | 224 | A->G | 225 | ins | 113 | C->T |
| 102 | Fr. | 139 | G->C | 166 | T->A | 314 | Stop at 344 | 87 | Stop at 148 |
| 141 | C->G | 234 | A->C | 156 | C->A | 301 | A->G | 97 | C->T |
| 144 | C->T | 152 | C->A | 291 | A->G | 224 | G->A | 217 | Stop at 246 |
| 146 | G->A | 170 | C->T | 305 | A->G | 112 | C->G | 226 | G->T |
| 158 | G->T | 175 | G->C | 306 | A->T | 163 | C->A | 278 | T->A |
| 161 | G->A | 240 | A->G | 296 | C->T | 299 | T->A | 145 | C->T |
| 164 | G->T | 259 | G->T | 267 | del | 251 | del | 133 | Stop at 148 |
| 165 | Ins | 87 | Fr. | 151 | Stop at 169 | 162 | Stop at 180 | 136 | A->C |
| 176 | C->G | 142 | Fr. | 228 | Stop at 238 | 44 | G->T | 239 | A->T/C->A G->A/C- |
| 191 | Fr. | 175 | C->G | 165 | Stop at 180 | 177 | C->A/C->T | 245 | >A |
| 215 | G->T | 126 | A->G | 176 | C->A | 236 | A->C | 252 | T->A G->A/C- |
| 217 | G->T | 128 | T->G | 192 | A->G | 243 | T->C | 244 | >A |
| 220 | A->G | 128 | C->T | 167 | Ins | 137 | G->A | 299 | Stop at 305 |
| 224 | G->C | 134 | T->C | 166 | T->C | 218 | G->C | 305 | A->T/G->A |
| 242 | C->G | 172 | Fr. | 120 | A->G | 277 | G->C | 310 | A->C |
| 259 | A->T | 237 | T->A | 150 | C->A | 54 | Fr. | 322 | C->G |
| 267 | G->C | 193 | A->C | 155 | A->C | 40 | Ins | 323 | Stop at 344 |
| 291 | A->T | 213 | Fr. | 203 | Stop at 246 | 156 | Stop at 166 | 315 | C->G |
| 298 | G->T | 246 | G->A | 221 | A->G | 168 | C->G/A->T | 308 | G->C |
| 182 | C->A Stop at | 235 | Fr. | 50 | Stop at 109 | 249 | G->T/G->T | 323 | T->G |
| 233 | 239 | 329 | Fr. | 191 | Stop at 243 | 158 | C->A | 201 | T->A |

| Annexe B(3) | | | | | | | | | |
|---|---|---|---|---|---|---|---|---|---|
| 173 | T->C | 155 | A->G | 205 | T->C | 209 | G->T | 190 | T->C |
| 251 | A->C | 7 | G->C | 210 | Stop at 246 | 184 | Stop at 207 | 278 | T->C |
| 219 | Fr. | 56 | G->T | 110 | C->G | 146 | G->T | 305 | G->C |
| 280 | A->T | 104 | Fr. G->A/G- | 166 | C->A | 250 | C->A | 176 | del |
| 126 | T->A | 245 | >A | 269 | G->T | 74 | Stop at 122 | 217 | T->G |
| 132 | G->C | 317 | C->T | 155 | Stop at 179 | 225 | del | 174 | A->C |
| 181 | C->T | 125 | G->A | 155 | Stop at 169 | 253 | C->A | 289 | C->G |
| 184 | G->T | 214 | Fr. | 156 | Stop at 169 | 269 | INS | 234 | C->G |
| 220 | T->C | 248 | G->C | 162 | C->T | 184 | T->C | 232 | A->C |
| 266 | G->A | 307 | Ins | 196 | A->G | 304 | T->G | 317 | A->T |
| 279 | G->A | 152 | G->T | 213 | Stop at 246 | 204 | A->C | 132 | Fr. |
| 305 | Ins | 178 | C->G | 214 | C->T | 66 | Stop at 145 | 299 | Fr. |
| 220 | A->C | 253 | C->T | 269 | C->T | 259 | Stop at 263 | 158 | C->G/G->T |
| 284 | A->C | 270 | T->C | 287 | A->G | 263 | Stop at 271 | 142 | Stop at 169 |
| 280 | G->C Stop at | 281 | C->A | 313 | C->G | 280 | Stop at 344 | 203 | Stop at 207 |
| 172 | 231 Stop at | 216 | Fr. | 108 | Stop at 144 | 237 | Stop at 246 | 248 | G->C/G->C |
| 174 | 176 | 131 | Fr. | 321 | A->G | 289 | C->A | 256 | A->C |
| 224 | Ins Stop at | 141 | Ins | 244 | C->T | 315 | Stop at 344 | 262 | Stop at 343 |
| 251 | 344 | 140 | Fr. | 198 | Stop at 246 | 312 | C->T | 301 | Stop at 343 |
| 261 | del | 163 | T->A | 135 | Ins | 145 | C->G | 335 | G->A |
| 181 | G->A | 178 | A->C | 187 | Stop at 246 | 169 | G->T | 179 | Fr. |
| 265 | C->T | 186 | G->T | 264 | del | 184 | G->A | 341 | Stop at 344 |
| 272 | T->C | 208 | A->T | 52 | C->T | 364 | G->A | 103 | C->G |
| 136 | C->T | 255 | Fr. | 141 | G->C | 144 | del | 159 | Stop at 179 |
| 281 | G->T | 307 | G->A | 167 | A->G | 146 | Stop at 169 | 189 | Stop at 246 |
| 316 | C->T | 130 | T->G | 84 | C->T | 190 | C->A | 274 | Stop at 304 |
| 130 | C->G | 356 | G->T | 122 | Stop at 169 | 249 | A->C | 149 | Fr. |
| 234 | C->A | 43 | T->C | 140 | A->T | 214 | T->A | 183 | Stop at 183 |
| 368 | Fr. | 159 | G->C | 153 | Ins | 204 | G->A G->A/C- | 227 | Stop at 245 |
| 301 | Fr. | 280 | Ins | 173 | Fr. | 242 | >G | 292 | Stop at 343 |
| 148 | Fr. | 327 | Fr. | 186 | Fr. | 208 | Stop at 241 | 178 | A->G |
| 176 | G->T | 87 | C->A | 152 | C->G | 158 | Stop at 180 | 251 | Stop at 343 |
| 152 | C->T | 156 | G->T | 171 | A->G | 217 | Stop at 221 | 252 | Stop at 263 |
| 248 | C->G | 158 | C->G | 180 | G->T | 262 | Stop at 344 | 64 | Fr. |
| 255 | T->G | 161 | G->T | 202 | G->A | 239 | Stop at 246 | 89 | Stop at 122 |
| 271 | Fr. | 173 | Stop at 180 | 227 | T->G | 205 | Stop at 246 | 108 | Stop at 122 |
| 274 | Fr. | 199 | G->T | 298 | G->A | 214 | T->C | 110 | Ins |
| 225 | G->A | 144 | Fr. | 303 | G->A | 297 | ins | 124 | Stop at 124 |
| 176 | T->A | 233 | Fr. | 261 | Ins | 268 | Fr. | 285 | del |
| 135 | Fr. | 275 | T->G | 276 | C->G | 256 | A->T | 342 | del |
| 135 | C->G | 162 | T->G | 305 | Fr. | 223 | C->A | 313 | A->T |
| 151 | C->T | 178 | Fr. | 117 | Stop at 122 | 26 | Stop at 43 | 217 | T->A |
| 159 | C->T | 256 | Fr. | 155 | Stop at 177 | 186 | A->T | 167 | Stop at 169 |
| 179 | A->G | 225 | Fr. | 277 | T->A | 214 4 | Stop at 246 | 278 | C->T/T->C |
| 306 | C->T | 148 | T->A | 298 | A->C | 245 | C->A | 290 | Stop at 304 |
| 174 | G->A | 187 | G->A C->A/C- | 141 | C->T | 287 | G->C | 173 | Stop at 173 |
| 208 | Fr. | 250 | >A | 115 | T->C | 96 | C->T | 259 | C->T |
| 126 | Fr. | 254 | T->G | 119 | G->A | 164 | Stop at 166 | 288 | T->A |
| 173 | del | 257 | T->C | 120 | Fr. | 255 | Ins | 207 | T->A |

### Annexe B(4)

| | | | | | | | | | |
|---|---|---|---|---|---|---|---|---|---|
| Codon | Event | Codon | Event | Codon | Event | Codon | Event | Codon | Event |
| 192 | C->T | 275 | T->C G->T/G- | 127 | T->A | 275 | del | 197 | Stop at 208 |
| 209 | Fr. | 216 | >T | 133 | Fr. | 284 | ins | 214 | A->T |
| 216 | T->G | 149 | T->C | 144 | A->T | 161 | G->C | 127 | C->G |
| 258 | G->A | 240 | G->T | 187 | T->C | 246 | A->T/G->T | 337 | G->C |
| 282 | G->C | 65 | A->T | 205 | T->A | 199 | G->C | 102 | Stop at 122 |
| 308 | Fr. | 125 | C->T | 209 | A->G | 195 | Stop at 246 | 187 | Stop at 202 |
| 332 | Fr. | 166 | C->T | 237 | A->T | 275 | Fr. | 100 | A->G |
| 173 | T->G | 242 | C->T | 337 | G->T | 283 | Stop at 305 | 140 | Stop at 143 |
| 249 | Fr. | 263 | A->C | 342 | G->A | 233 | ins | 176 | Stop at 179 |
| 275 | G->A | 139 | G->T | 377 | C->A | 127 | Stop at 169 | 235 | Ins |
| 294 | G->T | 165 | A->T | 93 | C->T | 138 | G->A | 250 | Stop at 262 |
| 316 | Fr. | 241 | T->G | 202 | C->T | 208 | A->T/C->T | 284 | Stop at 305 |
| 159 | C->A | 255 | T->A | 199 | Stop at 246 | 106 | del | 132 | G->A |
| 118 | Ins | 265 | Fr. | 252 | C->T | 245 | G->C/G->T | 129 | C->T |
| 277 | G->A | 279 | Fr. | 254 | C->T | 212 | Stop at 246 | 210 | C->T |
| 244 | G->C | 241 | Fr. | 262 | G->A | 133 | G->A | 232 | C->T |
| 264 | Fr. C->T/C- | 151 | C->G | 263 | A->G | 124 | T->C | 257 | C->T |
| 278 | >T | 156 | Fr. | 274 | Stop at 344 | 51 | Stop at 122 | 164 | Stop at 169 |
| 177 | C->T | 170 | A->T | 293 | Stop at 344 | 170 | G->A | 249 | del |
| 179 | C->T | 204 | G->T | 156 | C->G | 150 | del | 187 | Fr. |
| 281 | C->T | 249 | A->G | 157 | Stop at 169 | 85 | Stop at 143 | 210 | Fr. |
| 141 | G->A Stop at | 280 | G->T | 92 | C->T | 195 | T->A | 207 | T->C |
| 283 | 344 Stop at | 281 | A->C | 201 | G->T | 314 | Stop at 338 | 226 | G->C |
| 136 | 148 | 94 | T->A | 202 | Stop at 246 | 307 | Stop at 340 | 168 | C->G/C->G |
| 286 | G->A | 153 | C->A | 222 | C->T | 67 | Stop at 122 | 185 | A->G |
| 109 | C->A | 172 | T->C | 223 | Stop at 246 | 255 | Stop at 344 | 198 | Stop at 208 |
| 164 | A->G | 173 | T->A | 264 | Stop at 344 C->A/G- | 163 | del | 208 | G->C |
| 238 | G->C | 296 | C->G | 273 | >A | 191 | Stop at 246 | 331 | A->C |
| 110 | G->T | 284 | A->G | 316 | C->A | 255 | Stop at 257 | 320 | Stop at 336 A->C/G- |
| 113 | T->G | 135 | G->T | 271 | Ins | 262 | Stop at 263 | 331 | >A |
| 162 | C->G | 31 | G->A | 129 | Fr. | 264 | C->A | 338 | T->A |
| 183 | C->G Stop at | 72 | Fr. | 192 | Ins | 348 | G->T | 280 | Fr. |
| 287 | 344 | 91 | G->A | 307 | G->T | 232 | Stop at 246 | 290 | Fr. |
| 152 | G->A | 110 | Fr. | 220 | T->A | 170 | ins | 297 | Fr. |
| 138 | C->T | 154 | Fr. | 285 | A->G | 114 | T->A | 297 | C->G |
| 278 | C->G | 158 | Fr. | 226 | G->A | 343 | G->T | 136 | Stop at 164 |
| 236 | T->C | 167 | C->T | 137 | Ins | 26 | Stop at 36 | 149 | Stop at 180 |
| 237 | A->G | 178 | Stop at 180 | 259 | Ins | 137 | G->T | 221 | Stop at 246 |
| 289 | T->A | 195 | Stop at 208 | 234 | Fr. | 145 | G->C | 228 | ins |
| 237 | G->T | 197 | G->A | 135 | del | 146 | T->C | 243 | Stop at 340 |
| 136 | Ins Stop at | 199 | G->A | 102 | Stop at 116 G->A/A- | 286 | A->T | 292 | Stop at 304 |
| 99 | 147 Stop at | 227 | Ins G->T/G- | 324 | >G | 296 | A->T | 328 | del |
| 134 | 169 | 248 | >T T->C/G- | 27 | Fr. | 164 | G->A | 338 | Stop at 346 |
| 242 | T->C | 265 | >T | 162 | del | 148 | T->G | 243 | A->C |
| 193 | C->T | 272 | T->A | 277 | Ins | 274 | del | 348 | T->A |

**Tableau 3**

| Annexe B(5) | | | | | | | | | |
|---|---|---|---|---|---|---|---|---|---|
| Codon | Event | Codon | Event | Codon | Event | Codon | Event | Codon | Event |
| 188 | Fr. Stop at | 274 | T->G | 135 | T->G | 21 | Stop at 215 | 304 | C->T |
| 152 | 169 | 349 | Fr. | 69 | C->G | 239 | A->T | 228 | G->T |
| 57 | Fr. | 203 | Fr. | 242 | T->G | 313 | ins | 370 | A->C |
| 281 | C->G Stop at | 205 | T->G | 157 | G->A | 327 | T->G | 149 | C->T/C->T |
| 260 | 263 | 205 | A->C | 198 | G->C | 211 | C->A | 158 | C->T/G->A |
| 132 | A->T Stop at | 246 | A->T | 157 | T->G | 246 | Stop at 246 | 240 | G->A |
| 249 | 263 | 282 | Stop at 305 | 279 | G->C | 163 | C->T | 258 | Stop at 263 |
| 167 | G->T | 133 | A->C | 134 | Ins | 252 | del | 317 | C->A |
| 17 | A->T | 162 | A->T | 239 | Stop at 263 | 129 | del | 262 | T->A |
| 24 | A->T | 174 | A->T | 168 | Stop at 169 | 215 | G->C | 263 | A->T |
| 175 | C->T | 253 | C->G | 134 | Fr. | 253 | A->C | 163 | T->G |
| 358 | G->A | 131 | A->G | 253 | A->T | 274 | Ins | 312 | Fr. |
| 175 | Ins | 137 | T->A | 254 | A->T | 154 | C->A | 301 | C->A G->A/G- |
| 115 | C->T | 141 | Fr. | 247 | A->G | 183 | C->T | 226 | >A |
| 103 | Fr. | 157 | T->A | 235 | A->T | 225 | T->A | 200 | A->C/A->C |
| 237 | Fr. | 157 | Fr. | 176 | Stop at 243 | 149 | ins | 207 | G->C |
| 250 | C->T | 176 | Ins | 163 | Stop at 169 | 171 | Fr. | 226 | Stop at 227 |
| 365 | A->G | 240 | Fr. | 248 | Stop at 344 | 287 | A->T | 266 | |
| 271 | G->A | 274 | T->C | 289 | Stop at 304 | 133 | G->T | 113 | del |
| 320 | G->C | 46 | Fr. | 163 | Fr. | 137 | C->A | 226 | Fr. |
| 349 | G->T | 112 | Fr. | 207 | Fr. | 148 | G->T | 94 | C->A |
| 126 | del | 295 | C->T | 251 | A->T | 246 | A->C | 127 | Fr. |
| 36 | G->A | 193 | T->A | 112 | Stop at 120 | 251 | T->C | 133 | Stop at 145 |
| 76 | Fr. | 221 | G->T | 120 | Stop at 122 | 273 | T->C | 153 | Stop at 180 |
| 241 | C->G | 227 | Fr. | 231 | C->T | 297 | C->A | 75 | C->T |
| 281 | G->C | 241 | C->A | 212 | Stop at 214 | 192 | G->A | 116 | Stop at 122 |
| 244 | G->A | 281 | G->A | 179 | A->C | 244 | C->G | 184 | del |
| 218 | T->G Stop at | 316 | Ins | 174 | G->T | 221 | Stop at 222 | 106 | Stop at 122 |
| 256 | 344 A->C/G- | 344 | Fr. | 232 | del | 243 | T->A | 69 | Stop at 147 |
| 280 | >C | 145 | T->G | 173 | Stop at 195 | 308 | C->G | 298 | Stop at 344 |
| 258 | A->G | 145 | T->C | 273 | Stop at 344 | 189 | C->A | 182 | ins |
| 270 | T->A | 194 | T->C | 143 | T->A | 239 | | 133 | del |
| 176 | T->G Stop at | 162 | A->G | 161 | C->T | 142 | C->G | 163 | Stop at 168 |
| 171 | 231 Stop at | 315 | Ins | 72 | Stop at 120 | 295 | C->A | 174 | del |
| 251 | 263 | 203 | T->A | 265 | del | 156 | Stop at 168 | 330 | T->G |
| 337 | C->T | 273 | Ins | 214 | Stop at 214 | 213 | Stop at 245 | 125 | C->G |
| 266 | G->C | 62 | G->T | 107 | Stop at 147 | 243 | Stop at 244 | 258 | Stop at 344 |
| 203 | G->C Stop at | 71 | Fr. | 317 | Ins | 289 | C->T | 330 | Stop at 335 |
| 241 | 252 | 128 | Fr. | 165 | C->A | 211 | del | 113 | T->C |
| 193 | A->T | 203 | T->C | 99 | Stop at 122 | 220 | Stop at 244 | 265 | T->G |
| 255 | A->G | 254 | C->G | 36 | C->T | 229 | T->G | 126 | T->C |
| 194 | C->G Stop at | 282 | Fr. | 245 | Ins | 253 | del | 214 | Stop at 218 |
| 342 | 342 | 258 | G->C | 76 | C->T | 302 | Stop at 303 | 284 | C->T |
| 55 | Ins | 217 | Fr. | 160 | T->A | 208 | A->G | 96 | ins |
| 257 | C->G | 139 | A->C | 165 | A->C | 212 | Stop at 244 | 62 | Stop at 121 |

| Annexe B(6) | | | | | | | | | |
|---|---|---|---|---|---|---|---|---|---|
| 282 | ins | 215 | A->C | 269 | Fr. G->T/C- | 129 | Stop at 145 | 285 | A->C |
| 245 | G->C | 243 | Ins | 245 | >A | 190 | del | 358 | G->T |
| 209 | ins | 295 | Fr. | 208 | G->A | 216 | Stop at 221 | 122 | G->A |
| 239 | ins | 285 | A->T | 236 | C->T | 275 | Stop at 304 | 69 | Stop at 122 |
| 179 | T->G | 170 | Stop at 179 | 294 | G->A | 150 | ins | 155 | C->T/C->G G->A/C- |
| 314 | C->T | 208 | Stop at 246 | 251 | Fr. | 188 | C->G | 245 | >G |
| 155 | C->T Stop at | 209 | Stop at 214 | 215 | Ins | 220 | Ins | 181 | C->G |
| 249 | 344 | 240 | Stop at 263 | 154 | C->T | 292 | A->C | 185 | Ins |
| 116 | C->G | 141 | G->T | 293 | G->C | 305 | G->T | 52 | Stop at 56 |
| 163 | A->G | 151 | Fr. | 161 | C->G | 48 | A->T/C->T | 112 | Stop at 122 |
| 173 | G->C | 182 | Stop at 246 | 56 | G->A | 154 | C->G | 165 | Stop at 169 |
| 255 | C->T | 140 | A->G | 139 | G->A | 150 | Fr. | 323 | del |
| 255 | C->G | 142 | C->T | 222 | G->C | 329 | C->A | 67 | C->G |
| 218 | T->C Stop at | 169 | T->A | 302 | Stop at 344 | 80 | C->T | 148 | A->T |
| 301 | 344 | 170 | A->G | 144 | Stop at 169 | 243 | T->G | 230 | Stop at 246 |
| 271 | A->T | 271 | A->G | 166 | T->G | 104 | Stop at 148 | 95 | Stop at 148 |
| 286 | A->G | 331 | C->T | 149 | T->A | 117 | Stop at 148 | 276 | Stop at 286 |
| 294 | A->G | 194 | Stop at 245 T->G/T- | 204 | del | 138 | C->A | 249 | Stop at 342 |
| 264 | C->T | 113 | >G | 127 | C->A | 248 | C->T/G->C | 208 | del |
| 235 | A->G | 165 | C->T | 289 | T->C | 167 | G->A | 163 | Stop at 163 |
| 249 | A->T | 176 | Stop at 246 | 261 | A->G | 214 | C->G | 213 | A->G |
| 216 | G->A | 207 | T->G | 269 | A->G | 272 | Fr. | 282 | Stop at 304 |
| 215 | G->A | 297 | C->T | 128 | Stop at 169 | 186 | A->G | 295 | Stop at 344 |
| 272 | G->A Stop at | 141 | T->G | 159 | Stop at 169 | 147 | Ins | 160 | del |
| 267 | 344 | 181 | G->C | 204 | Ins | 261 | T->G | 233 | A->T |
| 242 | G->A | 229 | Fr. | 242 | Stop at 242 | 240 | T->G | 186 | T->C |
| 195 | T->C | 276 | Fr. | 237 | del | 288 | Fr. | 243 | T->C/G->A |
| 172 | G->T | 149 | Stop at 169 | 284 | Stop at 304 | 286 | A->G/A->T | 142 | C->A |
| 239 | A->G | 193 | C->G | 331 | G->T | 126 | Stop at 169 | 144 | G->T |
| 262 | G->T | 293 | Stop at 304 | 130 | T->A | 182 | Fr. | 231 | Fr. |
| 255 | T->C | 147 | Fr. | 39 | C->T | 298 | Fr. | 254 | Fr. |
| 286 | A->C | 286 | G->T | 352 | G->C | 220 | T->G | 266 | ins |
| 283 | G->A | 287 | Stop at 303 | 209 | Stop at 246 | 269 | G->A | 258 | A->C |
| 190 | C->T | 293 | G->A | 90 | C->T | 232 | Fr. | 239 | Stop at 261 |
| 154 | G->A Stop at | 295 | T->C | 111 | G->A | 131 | del | 262 | G->C/G->C |
| 272 | 344 | 215 | Fr. | 119 | C->T | 261 | Fr. | 296 | Stop at 334 |
| 143 | G->A | 333 | Fr. | 141 | T->C | 111 | T->A | 284 | del |
| 271 | A->C | 28 | A->C | 202 | T->C | 285 | Fr. | 150 | A->C |
| 133 | T->A | 67 | C->T | 326 | A->G | 266 | G->A/A->T | 225 | G->T |
| 174 | Fr. | 288 | A->G | 36 | G->T | 162 | Stop at 169 | 247 | Fr. |
| 132 | A->G | 276 | del | 68 | A->G | 208 | Ins | 322 | C->T |
| 252 | Fr. | 292 | Fr. | 117 | G->T | 250 | Ins | 85 | Stop at 117 |
| 330 | T->A | 189 | C->T | 145 | G->T G->A/T- | 130 | Stop at 169 | 86 | ins |
| 179 | C->A | 210 | A->G | 215 | >A | 289 | Fr. | 189 | Fr. |
| 309 | C->G | 217 | T->C | 325 | G->A | 198 | Fr. | 315 | Fr. |
| 212 | ins | 135 | Stop at 169 | 112 | G->A | 302 | Fr. | 169 | Stop at 180 |
| 175 | G->T | 165 | A->G | 308 | G->A | 137 | C->T | 245 | G->T/G->T |

| Annexe B(7) | | | | | | | | | |
|---|---|---|---|---|---|---|---|---|---|
| 153 | C->G C->G/G- | 234 | del | 63 | C->T | 191 | Ins | 175 | >C/C->G |
| 145 | >T | 218 | del | 104 | A->T | 186 | G->A | 71 | C->T |
| 277 | Fr. | 100 | C->T | 212 | T->C | 237 | ins | 72 | Stop at 148 |
| 275 | G->T | 169 | G->A | 217 | G->A | 230 | A->G | 98 | T->A |
| 110 | C->T | 158 | Stop at 179 | 328 | T->C | 184 | A->G | 287 | Stop at 304 |
| 232 | T->A C->A/C- | 143 | Stop at 169 | 45 | C->T | 157 | ins | 162 | A->G/C->T |
| 151 | >T | 200 | Ins | 299 | G->C | 95 | T->A/T->G | 130 | T->C |
| 218 | G->T | 185 | Stop at 246 | 111 | T->C | 314 | Fr. | 215 | Stop at 243 |
| 139 | A->G | 11 | G->A | 127 | T->C | 306 | A->G | 204 | Stop at 207 |
| 250 | C->G | 217 | G->C | 162 | Ins | 45 | C->A | 315 | Stop at 336 |
| 280 | A->C | 72 | Stop at 122 | 360 | G->T | 100 | Fr. | 33 | Stop at 43 |
| 127 | C->T | 105 | G->T | 257 | Ins | 162 | Fr. | 41 | Stop at 43 |
| 176 | G->C | 221 | G->A | 341 | T->G | 319 | Fr. | 80 | Stop at 120 |
| 274 | G->C | 253 | A->G | 242 | Stop at 246 | 113 | Fr. | 96 | Stop at 147 |
| 246 | T->G Stop at | 300 | Stop at 344 | 262 | del | 126 | C->A | 207 | Stop at 212 |
| 229 | 238 | 250 | Stop at 342 | 257 | T->G | 196 | Stop at 246 G->A/C- | 215 | Stop at 245 |
| 247 | A->C | 135 | T->A C->T/C- | 229 | T->C | 175 | >G | 224 | Stop at 246 |
| 290 | G->A Stop at | 159 | >T | 196 | G->A | 182 | T->G | 260 | del |
| 219 | 246 Stop at | 249 | G->A | 200 | A->G | 190 | C->G | 276 | Stop at 339 |
| 88 | 122 | 198 | A->G | 278 | Stop at 344 | 141 | Stop at 148 | 290 | Stop at 339 |
| 254 | T->C | 238 | T->G | 144 | G->C | 166 | Stop at 180 | 300 | Stop at 343 |
| 283 | C->G | 243 | A->T | 158 | Stop at 169 | 345 | Stop at 369 | 51 | Stop at 121 |
| 299 | G->A | 259 | G->A | 252 | Stop at 344 | 192 | C->A | 301 | Stop at 303 |
| 346 | G->A | 268 | A->G | 241 | Stop at 261 C->T/G- | 65 | Fr. | 236 | A->C/C->G |
| 116 | T->C | 287 | Fr. | 282 | >A | 185 | G->T | 83 | C->T |
| 150 | A->G | 302 | G->A | 276 | G->T | 181 | C->A | 237 | T->C |
| 95 | T->C | 189 | G->C | 196 | C->A | 190 | Stop at 208 | 156 | ins |
| 54 | T->A | 212 | Fr. | 193 | T->C | 155 | C->G | 128 | C->G |
| 256 | C->T | 51 | G->T | 160 | A->C | 242 | G->T/C->T | 243 | T->G/G->C |
| 309 | C->A | 160 | G->C | 243 | A->G | 269 | A->T | 133 | A->G |
| 109 | T->C | 207 | Ins | 206 | Stop at 246 | 283 | Fr. | 125 | C->A |
| 265 | T->C Stop at | 147 | T->G | 194 | T->A | 189 | G->A G->A/G- | 62 | A->G |
| 139 | 169 | 177 | Fr. | 212 | T->A | 244 | >C | 54 | C->T |
| 154 | G->T | 121 | Fr. | 169 | A->G | 138 | Stop at 148 | 84 | C->G |
| 179 | A->T | 147 | T->C | 183 | T->C | 188 | Stop at 208 | 202 | G->C/T->G |
| 255 | del Stop at | 160 | A->G | 77 | C->G | 246 | del | 319 | A->C |
| 342 | 344 | 230 | Fr. | 188 | Ins G->T/C- | 180 | G->C | 138 | C->G |
| 11 | G->C Stop at | 237 | A->C | 158 | >T | 175 | del | 229 | T->A/G->A |
| 121 | 122 | 47 | Stop at 121 | 194 | Stop at 207 | 290 | Stop at 301 1 | 101 | Stop at 122 |
| 34 | ins | 78 | Fr. | 253 | Ins | 271 | del | 278 | Stop at 304 |
| 53 | G->A | 81 | Stop at 122 | 360 | Stop at 369 | 156 | Stop at 180 | 339 | Fr. |
| 144 | A->C | 108 | Stop at 146 | 191 | C->G | 69 | C->A | 303 | G->T |
| 280 | A->G | 110 | G->C | 141 | T->A | 112 | C->A | 247 | Stop at 344 |

| Annexe B(8) | | | | | | | | | |
|---|---|---|---|---|---|---|---|---|---|
| 326 | G->T Stop at | 156 | C->T | 303 | A->T | 193 | C->A | 299 | Ins |
| 332 | 344 | 217 | del | 49 | Ins | 222 | Fr. | 293 | del |
| 256 | Ins | 242 | T->A | 62 | Stop at 141 | 228 | Stop at 245 | 247 | Stop at 343 |
| 283 | C->T | 245 | Stop at 340 | 103 | del | 145 | del | 5 | C->T |
| 232 | T->C | 251 | Ins | 105 | del | 148 | Stop at 167 | 123 | C->T |
| 184 | Fr. | 91 | G->T | 121 | del | 140 | Stop at 168 | 126 | C->T |
| 273 | C->G | 136 | A->G | 124 | Ins | 171 | Stop at 180 | 320 | G->A |
| 133 | T->C | 146 | G->C | 124 | Stop at 167 | 304 | Fr. | 356 | G->A |
| 272 | G->T | 164 | A->T | 338 | Stop at 343 | 159 | ins | 379 | G->A |
| 293 | G->T | 194 | Fr. | 336 | G->T | 261 | G->A | 154 | Stop at 180 |
| 267 | G->A | 255 | A->T | 124 | C->G | 304 | A->G | 164 | G->C |
| 325 | G->T | 339 | Ins | 284 | A->T | 222 | G->T | 75 | C->G |
| 71 | Ins | 35 | G->T | 144 | G->A | 291 | G->C | 163 | Stop at 165 |
| 120 | A->T | 213 | G->T | 227 | C->T | 147 | T->A/T->A | 238 | Stop at 244 |
| 151 | Ins | 261 | Stop at 263 | 208 | G->T | 216 | G->C | 8 | C->T |
| 307 | Fr. | 299 | T->C | 228 | G->A | 91 | Ins | 15 | A->C |
| 108 | Fr. | 204 | A->T | 196 | G->T | 311 | A->C | 61 | A->G |
| 257 | T->A Stop at | 47 | Fr. | 195 | C->G | 334 | Stop at 344 | 72 | C->T |
| 257 | 344 | 178 | C->A | 272 | T->G | 211 | T->C | 102 | ins |
| 138 | G->T | 257 | G->A | 53 | G->C | 197 | G->T | 104 | G->T |
| 155 | C->A | 341 | C->T | 290 | C->G A->T/A- | 202 | C->A | 106 | A->G |
| 167 | C->A | 290 | C->T | 292 | >T | 219 | C->A | 365 | C->T |
| 174 | A->G | 169 | T->C | 245 | Stop at 246 | 228 | G->C | 10 | C->T |
| 181 | G->T | 233 | C->T | 188 | Stop at 246 | 163 | A->C G->C/A- | 21 | C->T |
| 241 | T->A | 198 | G->A | 288 | Stop at 344 | 271 | >G | 361 | G->A |
| 305 | A->T | 200 | Fr. | 176 | Fr. | 238 | Fr. | 364 | C->T |
| 273 | C->A | 228 | C->G | 148 | Stop at 179 | 206 | T->A | 385 | T->C |
| 219 | C->T | 236 | C->A G->T/C- | 161 | Stop at 169 | 52 | del | 307 | A->G |
| 251 | C->G | 245 | >T | 211 | Stop at 246 | 94 | Stop at 122 | 161 | G->T/C->T |
| 233 | C->G Stop at | 249 | Ins | 244 | Stop at 246 | 236 | Stop at 236 | 241 | Stop at 263 |
| 215 | 246 | 251 | T->A | 247 | del | 107 | C->A | 327 | Stop at 335 |
| 216 | Ins | 258 | Fr. | 260 | Stop at 344 | 106 | Fr. | 157 | T->C |
| 344 | T->C | 278 | Ins | 216 | T->C | 69 | Fr. | 132 | Stop at 169 |
| 213 | G->C | 279 | Ins | 231 | A->T | 204 | Fr. | 221 | A->C |
| 82 | C->T | 296 | A->C | 208 | C->A | 305 | A->C/G->T | 184 | G->C |
| 151 | del | 255 | Stop at 343 | 301 | C->G | 269 | Stop at 344 | 157 | Stop at 179 |
| 180 | G->A | 290 | Stop at 344 | 208 | C->G | 230 | Stop at 238 | 289 | Stop at 305 |
| 337 | G->A | 137 | Stop at 145 | 237 | G->C | 227 | Stop at 228 | 105 | G->C |
| 281 | A->T | 155 | Fr. | 243 | G->C | 363 | G->A | 215 | Stop at 221 |
| 133 | T->G | 206 | Ins | 159 | G->T | 253 | Fr. | 179 | Stop at 180 |
| 236 | del | 242 | Ins | 33 | Ins | 250 | Stop at 344 | 128 | Stop at 148 |
| 306 | G->C | 300 | Fr. | 192 | del | 259 | C->A | 256 | Stop at 263 |
| 227 | T->A | 191 | T->C | 312 | C->A | 167 | del | 131 | Stop at 169 |
| 138 | G->C Stop at | 191 | C->A | 321 | Stop at 344 C->G/C- | 173 | Stop at 246 | 143 | Stop at 167 |
| 178 | 246 C->T/A- | 246 | T->A | 283 | >G | 313 | Fr. | 158 | C->T/G->T |
| 213 | >G | 258 | A->T | 285 | G->T | 346 | Ins | 207 | A->T |
| 191 | Stop at | 143 | Ins | 283 | C->A | 293 | Ins | 245 | Stop at 262 |

| Annexe B(9) | | | | | | | | | |
|---|---|---|---|---|---|---|---|---|---|
| 236 | A->G | 159 | Fr. | 216 | del | 224 | A->T | 258 | Stop at 291 |
| 196 | G->C | 165 | Stop at 178 | 318 | C->T G->C/C- | 158 | Stop at 167 | 266 | Stop at 271 |
| 156 | G->A | 168 | Ins | 119 | >G/C->G | 154 | Stop at 167 | 284 | Stop at 344 |
| 339 | G->T | 169 | del | 344 | T->G | 283 | Stop at 304 | 285 | ins |
| 166 | C->G Stop at | 195 | T->G | 216 | Stop at 246 | 284 | C->A | 290 | ins |
| 184 | 246 Stop at | 191 | C->T | 240 | G->C | 176 | G->T/C->T | 294 | Stop at 344 |
| 279 | 344 | 152 | Ins | 306 | Stop at 344 | 47 | C->T/C->T | 308 | Stop at 344 |
| 140 | C->T | 168 | A->C | 210 | A->T | 151 | C->T/C->T | 49 | Stop at 50 |
| 282 | C->A | 209 | G->C | 198 | A->T | 88 | C->T/C->T | 254 | Stop at 260 |
| 162 | T->A | 209 | G->A | 273 | Fr. | 71 | C->A | 301 | Stop at 305 |
| 251 | C->T Stop at | 214 | T->G | 138 | del | 162 | T->C | 311 | Stop at 344 |
| 241 | 246 G->A/G- | 166 | Fr. | 171 | Stop at 173 | 180 | A->G | 320 | Stop at 344 |
| 248 | >A Stop at | 265 | Stop at 344 | 174 | Ins | 150 | Stop at 163 | 324 | Stop at 344 |
| 362 | 369 | 163 | C->G | 209 | A->C | 218 | Stop at 219 | 244 | Stop at 263 |
| 81 | C->T G->A/G- | 182 | T->A | 208 | Stop at 21 | 218 | ins | 265 | C->A |
| 224 | >A | 211 | Ins | 285 | Stop at 304 | 228 | C->A | 222 | Stop at 246 |
| 197 | T->G | 236 | T->A | 286 | G->C | 248 | Fr. | 296 | C->A |
| 301 | C->T | 267 | C->T | 130 | C->A | 282 | G->A/G->T | 205 | ins |
| 157 | G->C | 148 | G->A | 201 | Fr. | 61 | A->T | 77 | Fr. |
| 282 | G->A | 133 | Stop at 169 | 330 | Stop at 344 | 75 | T->C | 86 | C->T |
| 276 | C->A C->T/C- | 174 | Stop at 179 | 131 | Stop at 148 | 76 | G->A | 112 | C->T |
| 250 | >T | 239 | A->C | 236 | Stop at 239 | 295 | C->G | 320 | ins |
| 279 | G->T C->T/C- | 244 | Fr. | 236 | Ins | 340 | G->A | 125 | G->T |
| 219 | >T C->T/C- | 271 | G->T | 238 | del | 144 | A->G | 266 | Fr. |
| 152 | >T | 278 | Fr. | 313 | Stop at 334 | 126 | T->G | 135 | Stop at 148 |
| 157 | C->T | 171 | G->A | 240 | Stop at 262 | 234 | T->C/C->G | 316 | Stop at 336 |
| 158 | C->T C->T/C- | 229 | G->A C->G/C- | 266 | del | 165 | Fr. | 234 | T->A/C->A |
| 222 | >T | 161 | >A | 276 | Stop at 344 | 101 | A->T | 233 | C->T/A->C |
| 195 | C->T | 168 | C->A | 166 | Ins | 254 | Stop at 344 G->A/G- | 206 | T->A/G->T |
| 291 | G->T | 241 | T->C | 150 | Stop at 169 | 217 | >A | 226 | C->A |
| 192 | A->T | 154 | Ins C->T/C- | 222 | C->A | 169 | Fr. | 302 | G->C |
| 333 | G->A | 177 | >T | 218 | T->A | 167 | A->T | 260 | C->G |
| 369 | del | 202 | G->C C->T/C- | 228 | C->T | 148 | A->C | 220 | A->G/T->A |
| 203 | G->A | 250 | >G | 141 | C->A | 155 | Stop at 180 | 233 | Stop at 246 |
| 232 | C->G | 261 | A->T | 221 | G->C | 195 | del | 325 | G->C |
| 188 | G->A | 303 | G->C | 226 | C->T | 248 | del | 185 | A->T |
| 224 | Fr. | 182 | G->A | 215 | T->C | 275 | T->A | 186 | Stop at 208 |
| 199 | A->G | 177 | C->A | 85 | C->T | 230 | Stop at 239 | 184 | Stop at 185 |
| 277 | T->C | 271 | G->C | 89 | C->T | 303 | Stop at 344 | 187 | Stop at 208 |
| 176 | T->C | 292 | A->T | 101 | A->G | 279 | del | 234 | C->T |
| 227 | T->C | 300 | C->T | 132 | A->T/A- | 153 | Fr. | 235 | Stop at 239 |

| Annexe B(10) | | | | | | | | | |
|---|---|---|---|---|---|---|---|---|---|
| 327 | T->C | 319 | A->T | 160 | G->A | 235 | A->C | 141 | Stop at 169 |
| 247 | C->T | 195 | Fr. | 46 | C->T | 82 | Stop at 145 | 264 | T->C |
| 135 | C->T | 269 | G->C | 122 | G->T | 196 | Fr. | 76 | A->T |
| 211 | C->T | 172 | T->G | 108 | G->A | 275 | ins | 96 | T->C |
| 149 | C->T C->T/C- | 35 | G->C | 103 | C->T G->C/A- | 82 | ins | 308 | C->A |
| 142 | >T C->T/C- | 90 | Fr. | 281 | >G/C->G | 273 | T->G | 96 | C->G |
| 138 | >T | 135 | C->A | 105 | C->T C->T/G- | 276 | Stop at 305 | 206 | G->A |
| 178 | C->T C->T/C- | 131 | A->T | 273 | >A | 254 | A->G | 241 | Ins |
| 241 | >T | 155 | del | 140 | Stop at 148 | 225 | T->G | 111 | T->G |
| 130 | C->T C->T/C- | 228 | Stop at 239 | 270 | Stop at 337 | 246 | G->T | 238 | Ins |
| 127 | >T | 292 | Stop at 305 | 190 | Stop at 195 | 93 | G->A | 347 | C->G |
| 135 | G->A | 387 | del | 275 | Stop at 341 | 254 | C->A | 154 | G->T/C->T |
| 211 | A->G | 231 | A->G | 143 | Stop at 148 | 294 | Ins | 212 | T->G |
| 286 | Fr. | 268 | A->C | 107 | C->G | 139 | Fr. | 260 | C->A |
| 168 | Fr. | 179 | T->C | 194 | Stop at 206 | 356 | G->C | 123 | Stop at 148 |
| 175 | C->A | 232 | A->T | 271 | Stop at 343 | 182 | G->C | 146 | T->G |
| 381 | Fr. | 134 | T->G | 254 | del | 73 | Stop at 122 | 73 | T->A |
| 263 | Fr. | 274 | G->A | 255 | Stop at 262 | 76 | C->G | 165 | G->T |
| 292 | A->G | 243 | Stop at 246 | 322 | A->C | 150 | Stop at 165 | 313 | G->A |
| | | 131 | A->C | 323 | Stop at 340 | 214 | Stop at 220 | 130 | ins |
| | | | | | | | | 133 | A->T |
| | | | | | | | | 289 | Ins |

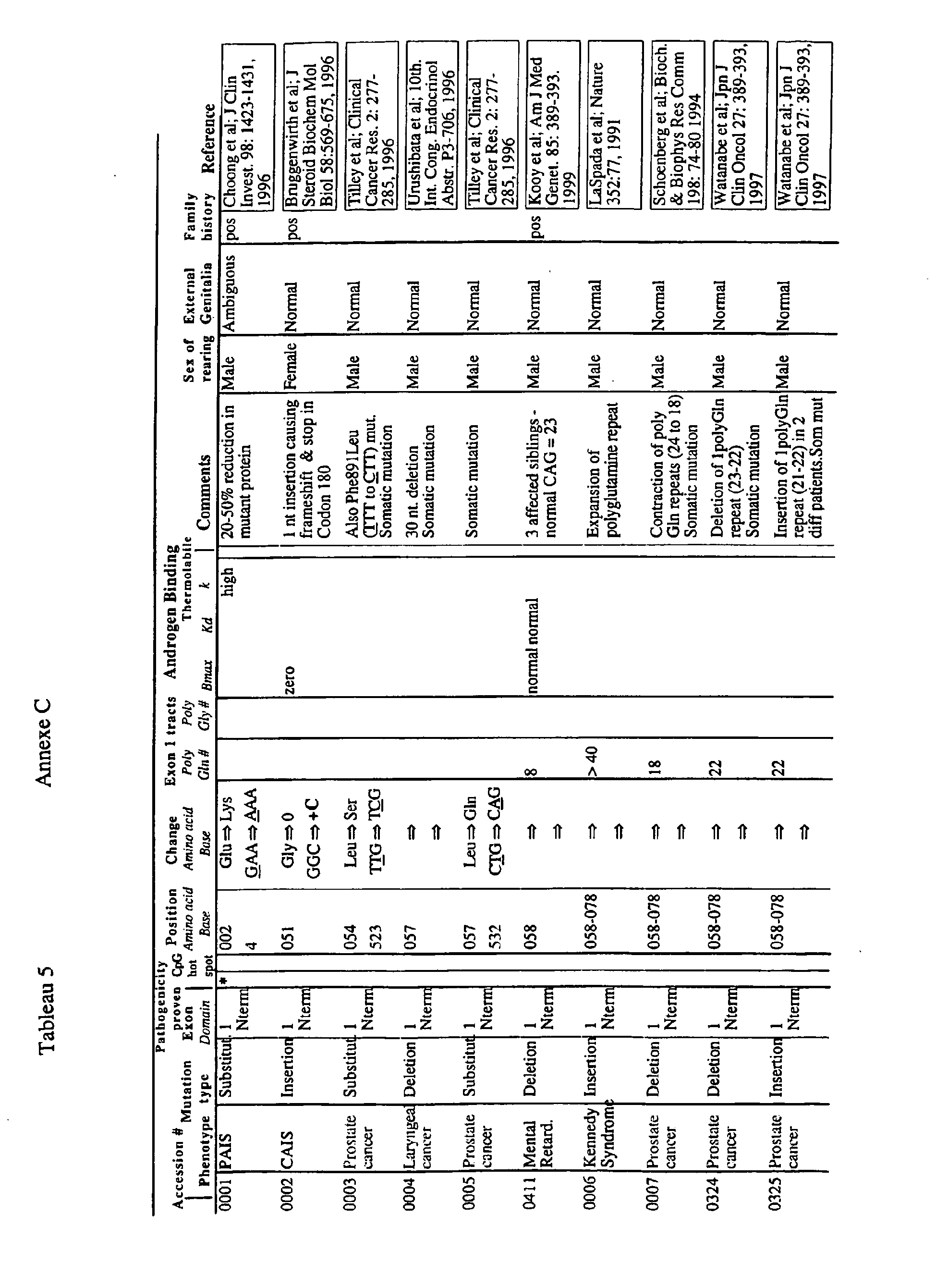

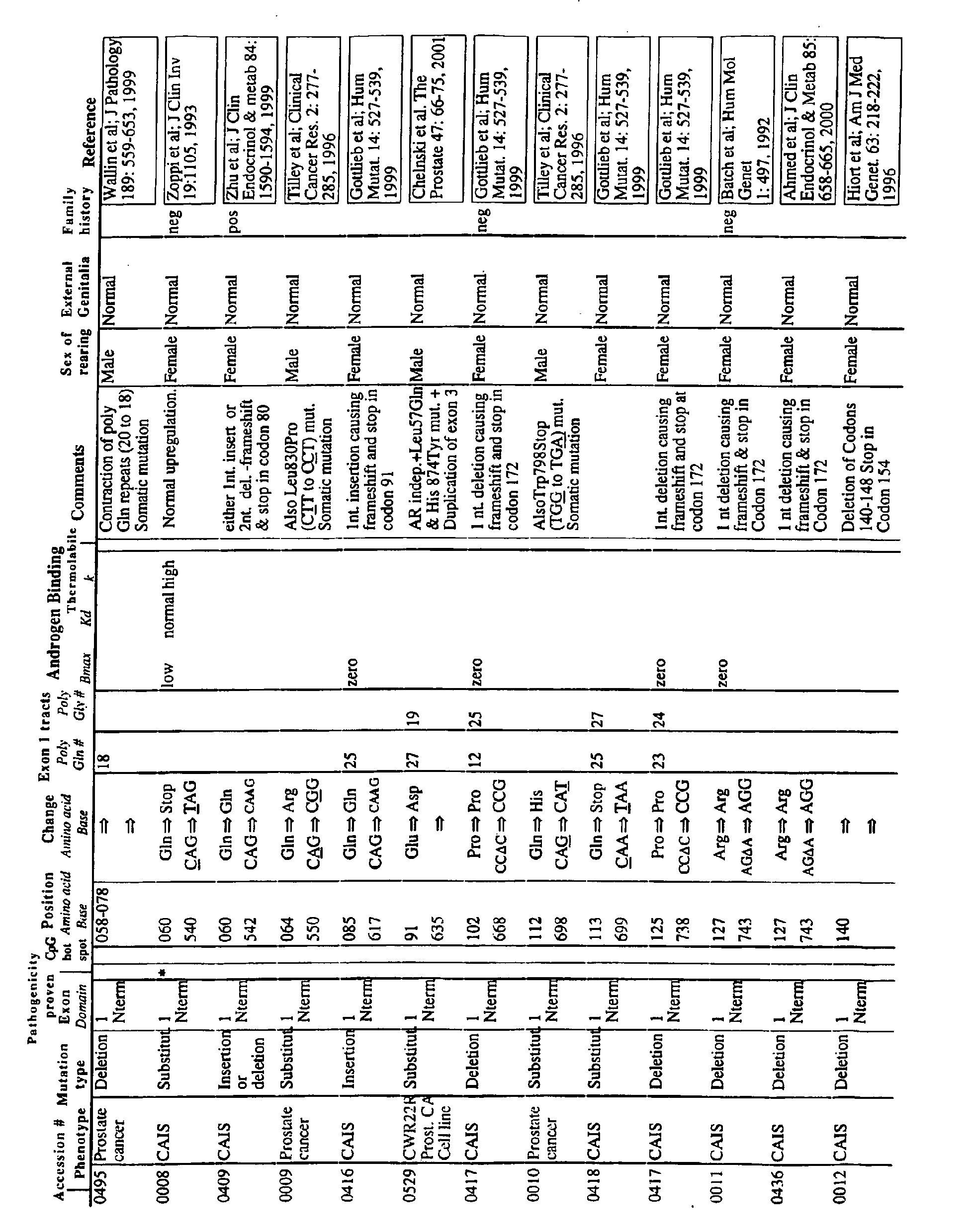

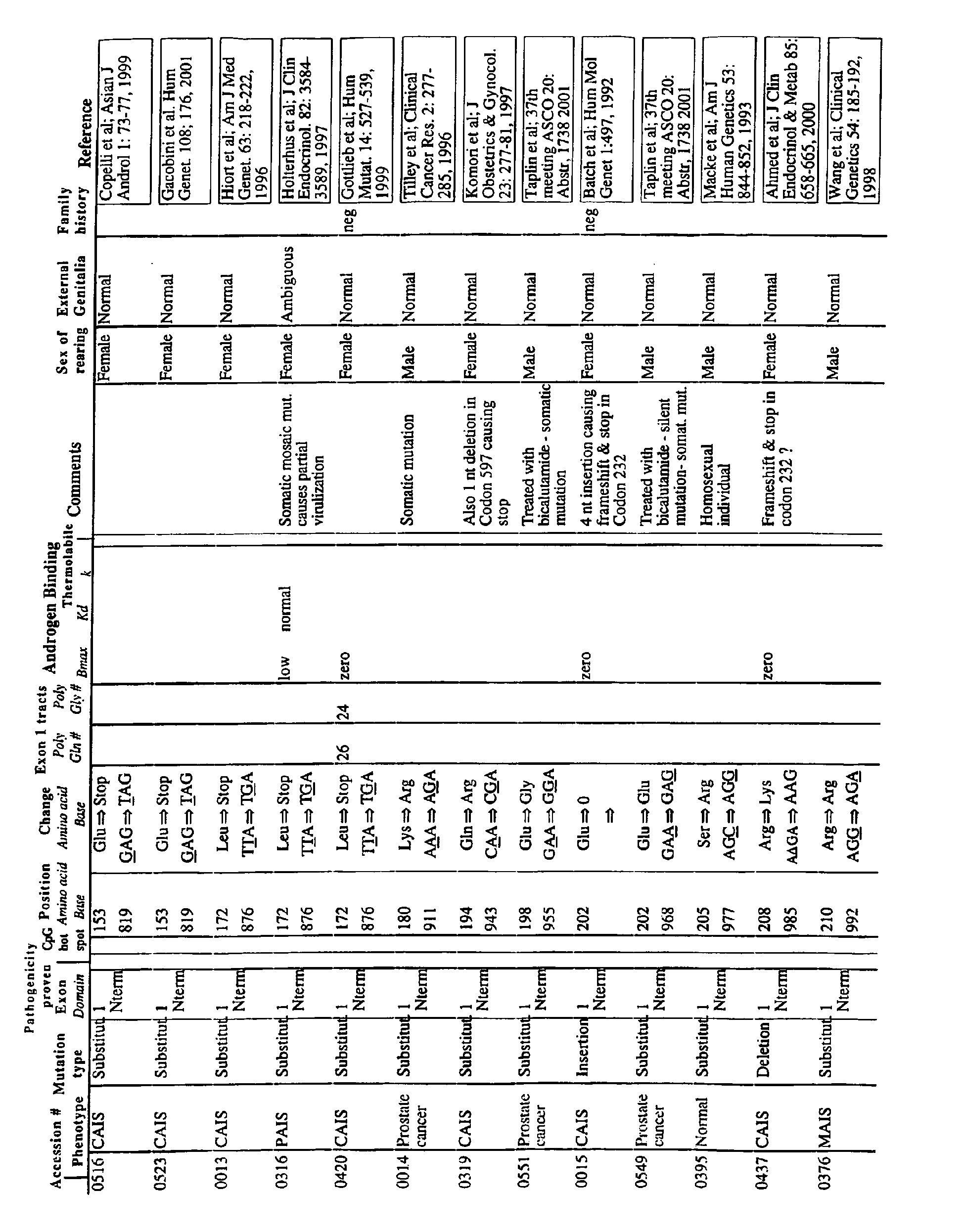

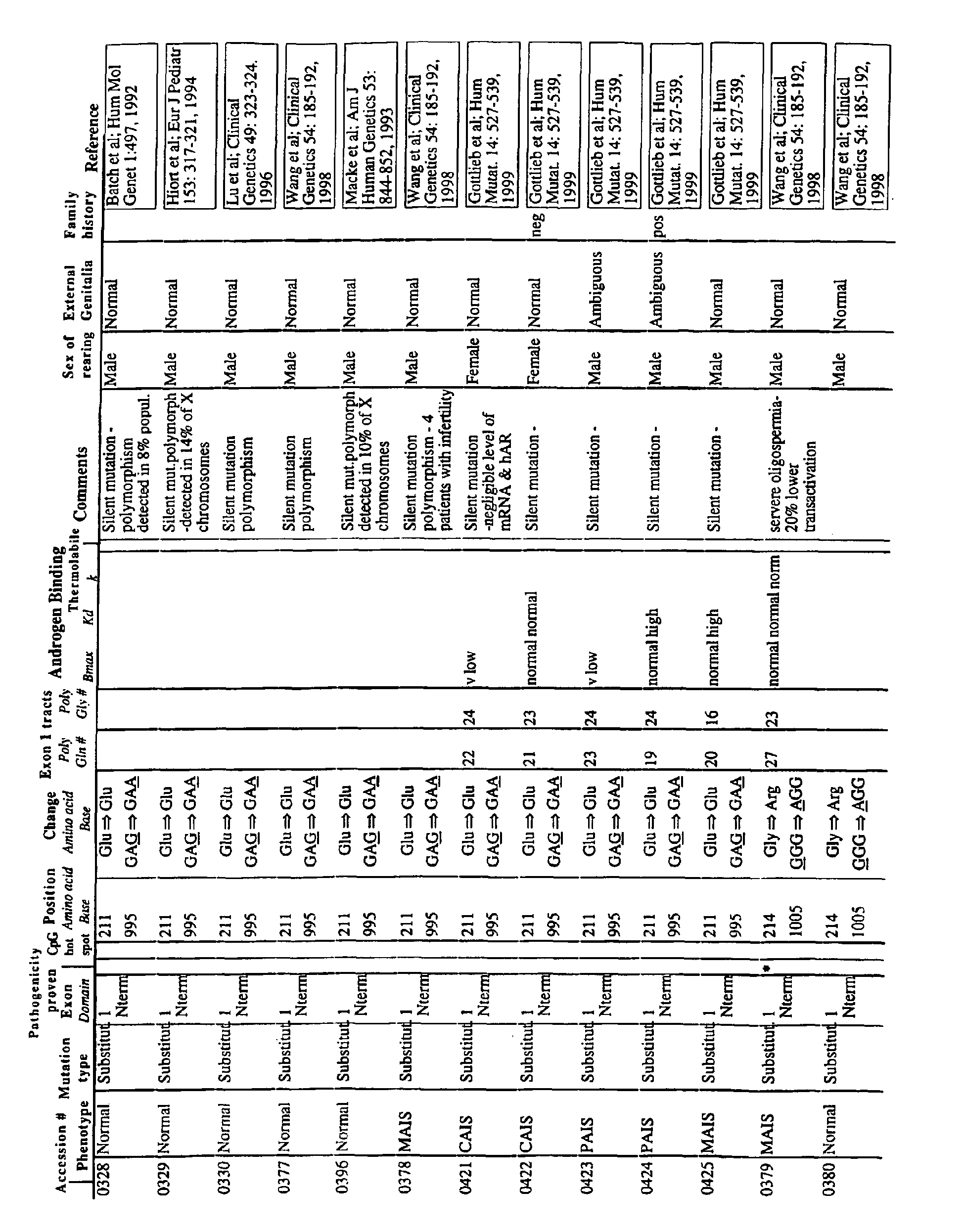

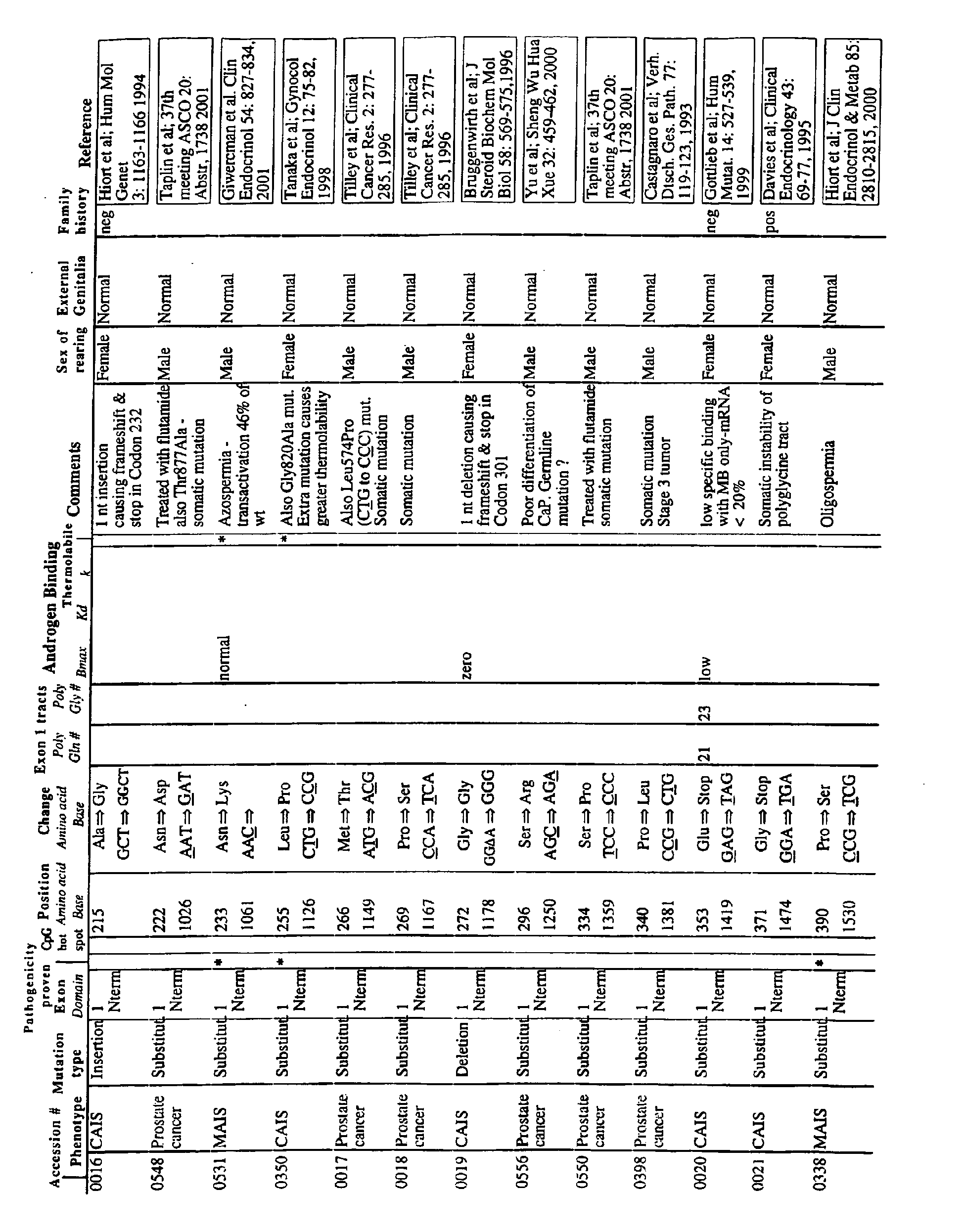

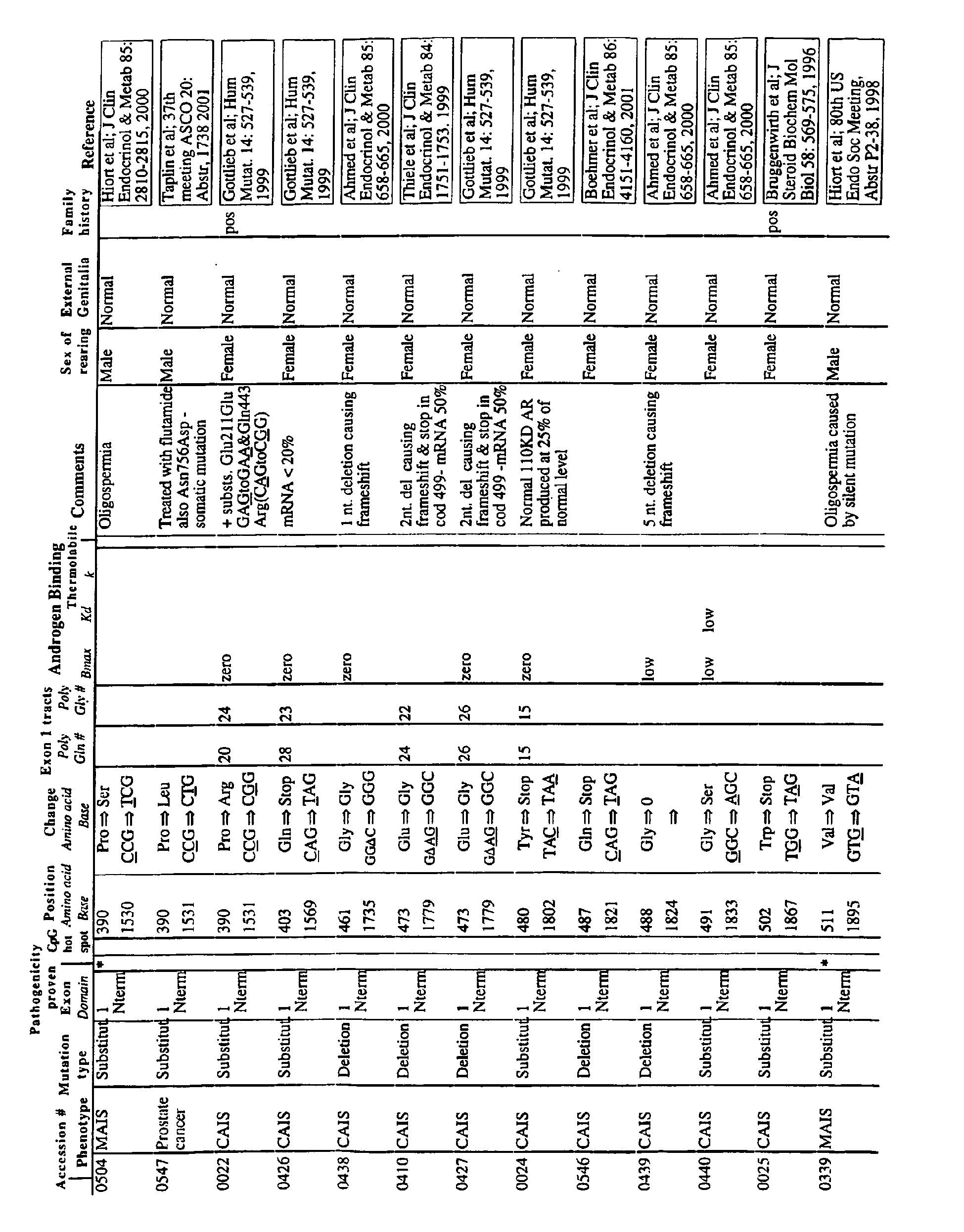

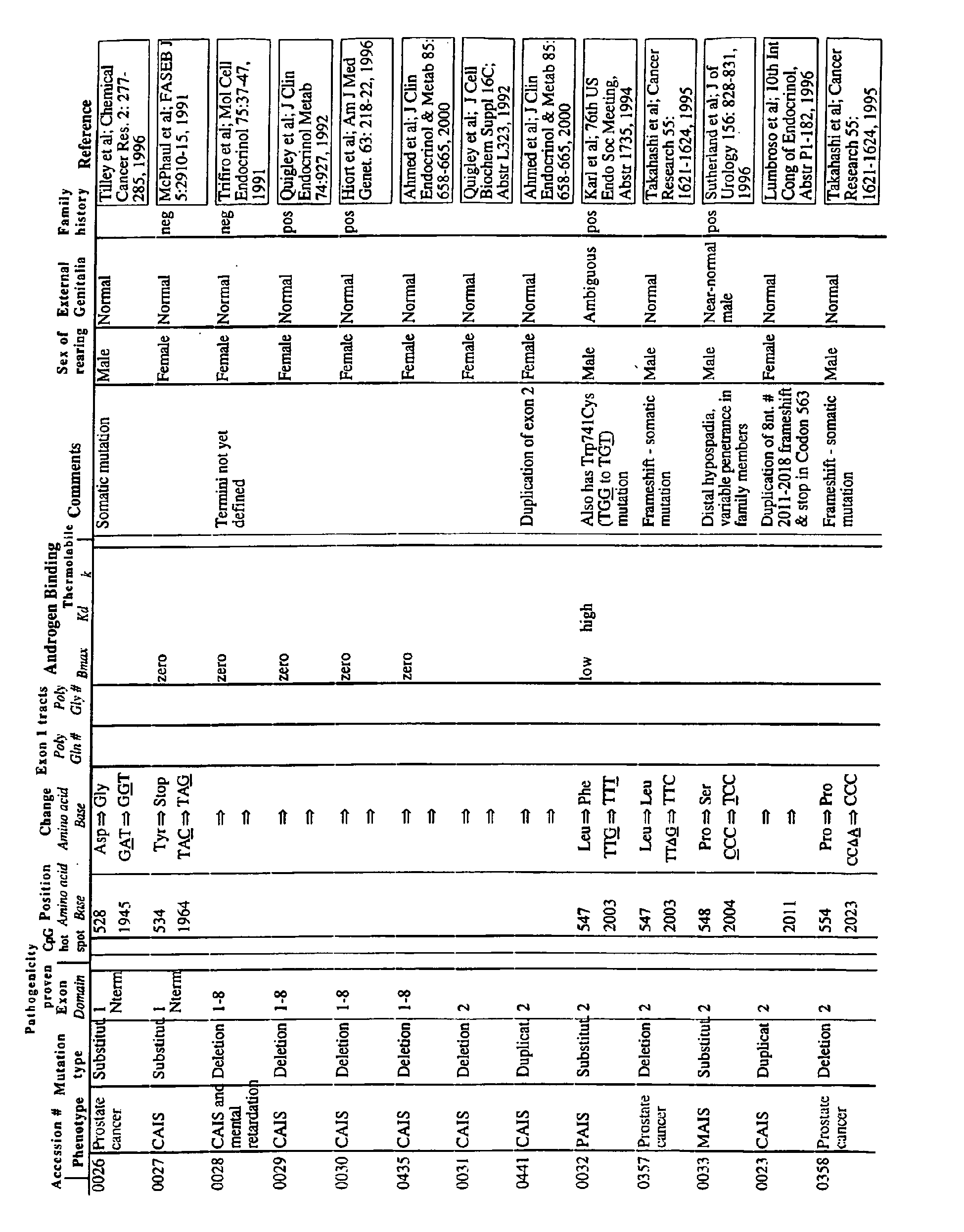

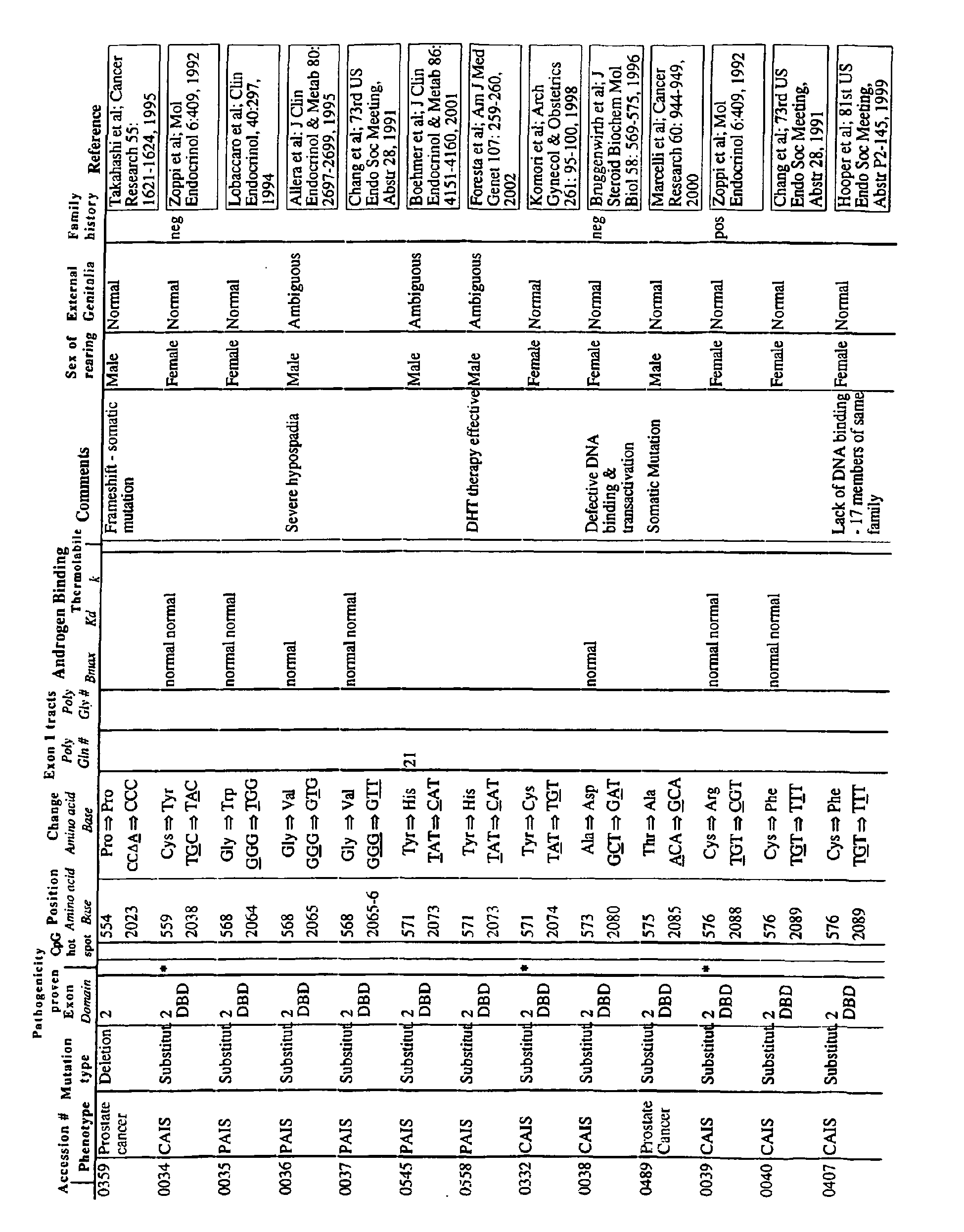

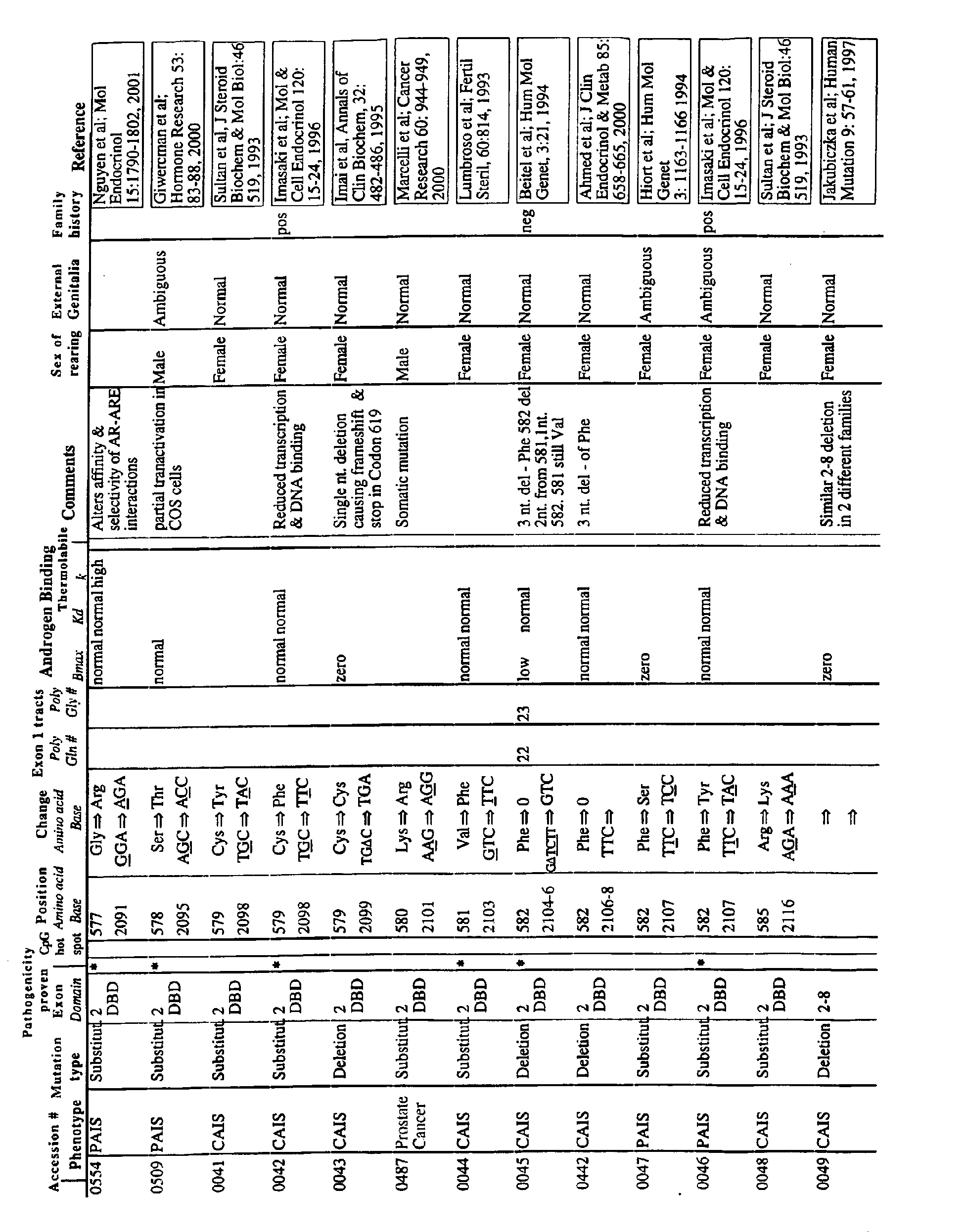

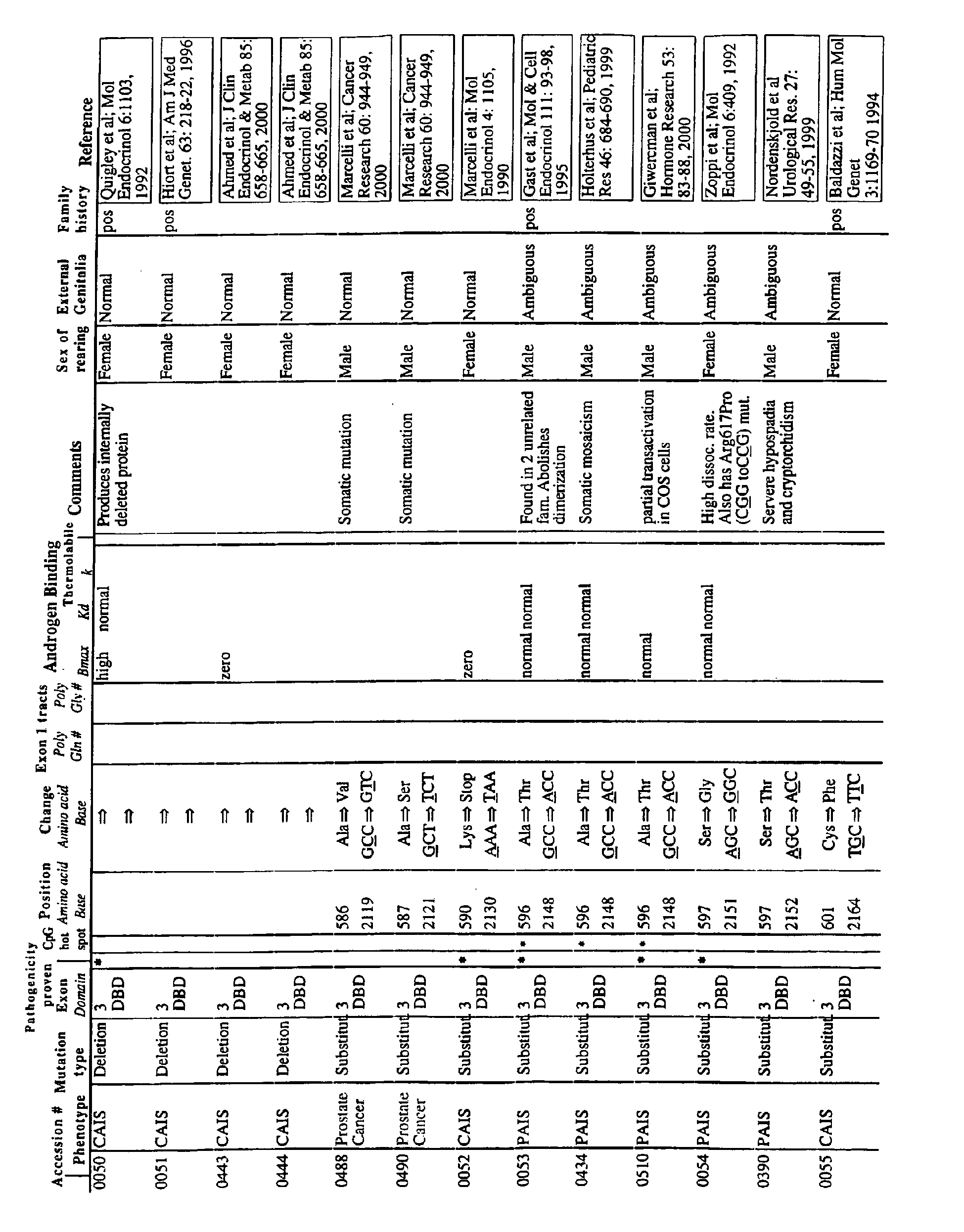

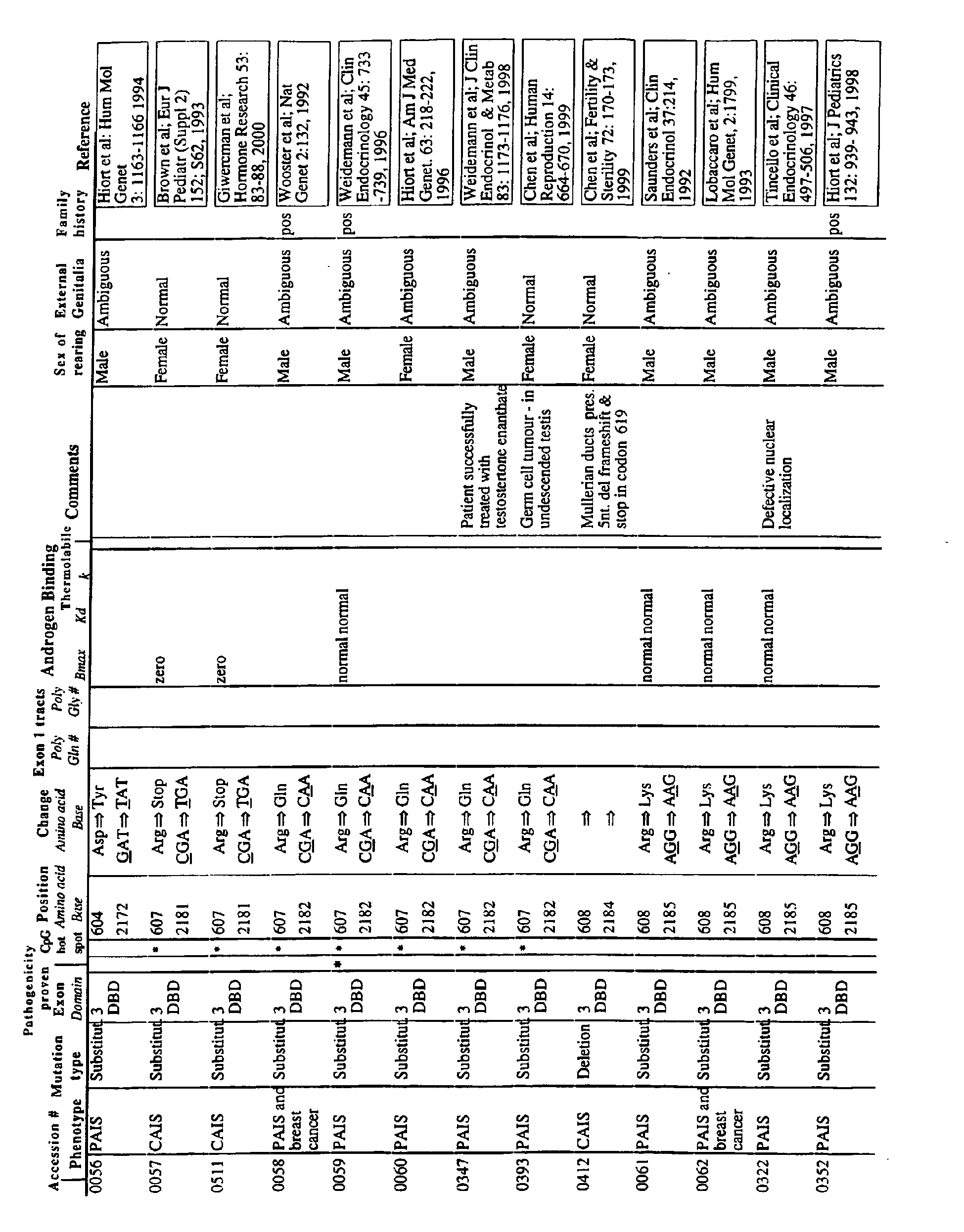

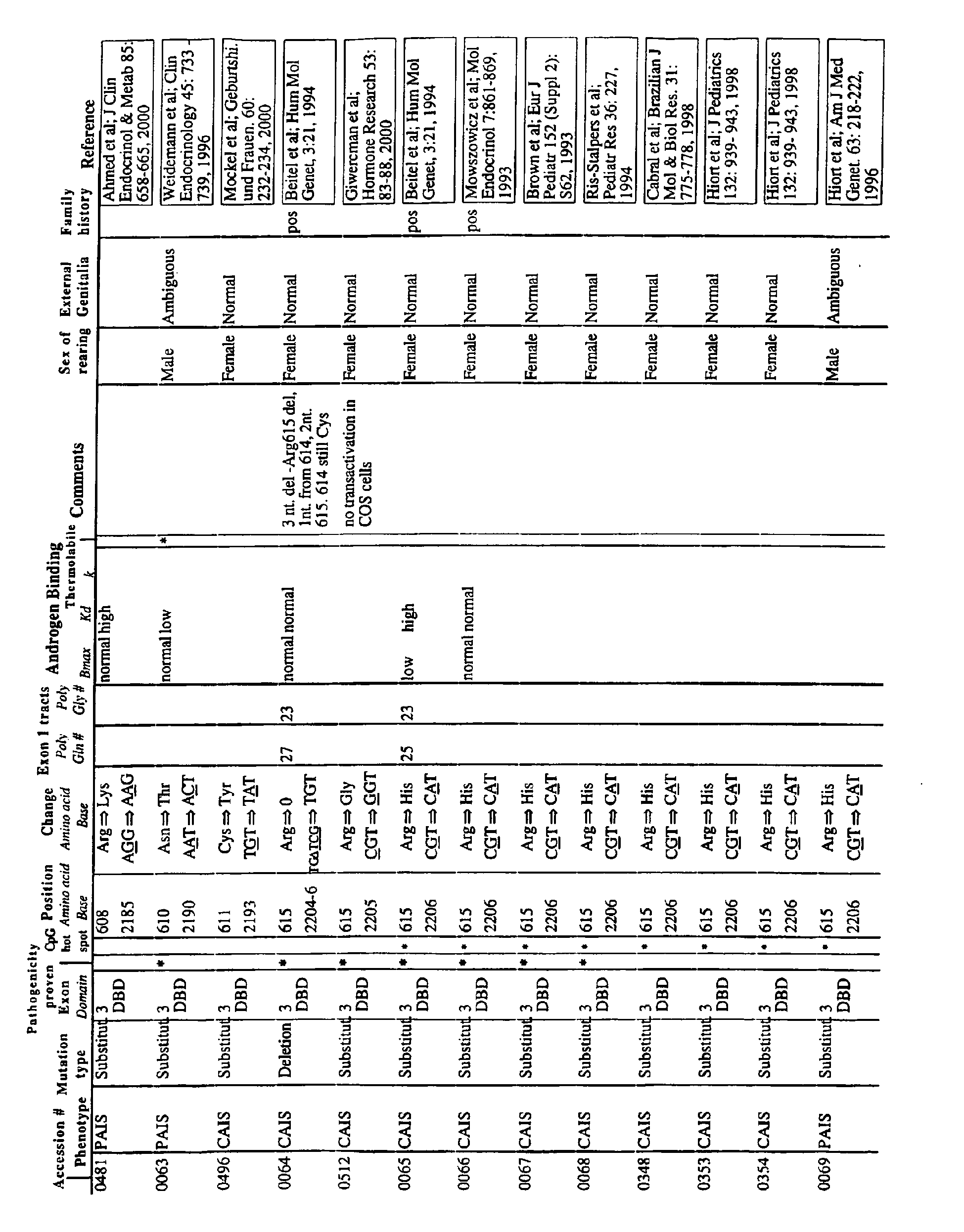

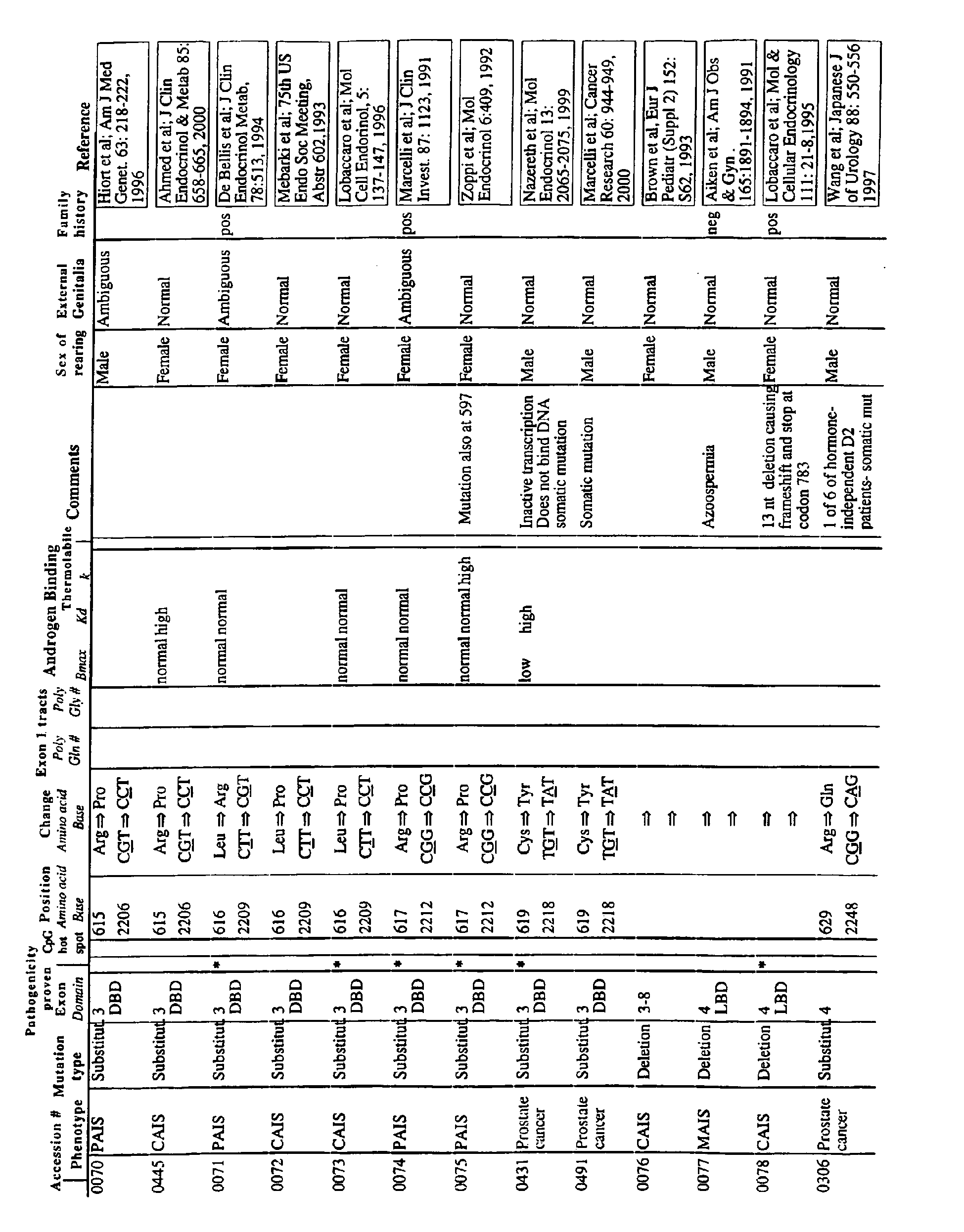

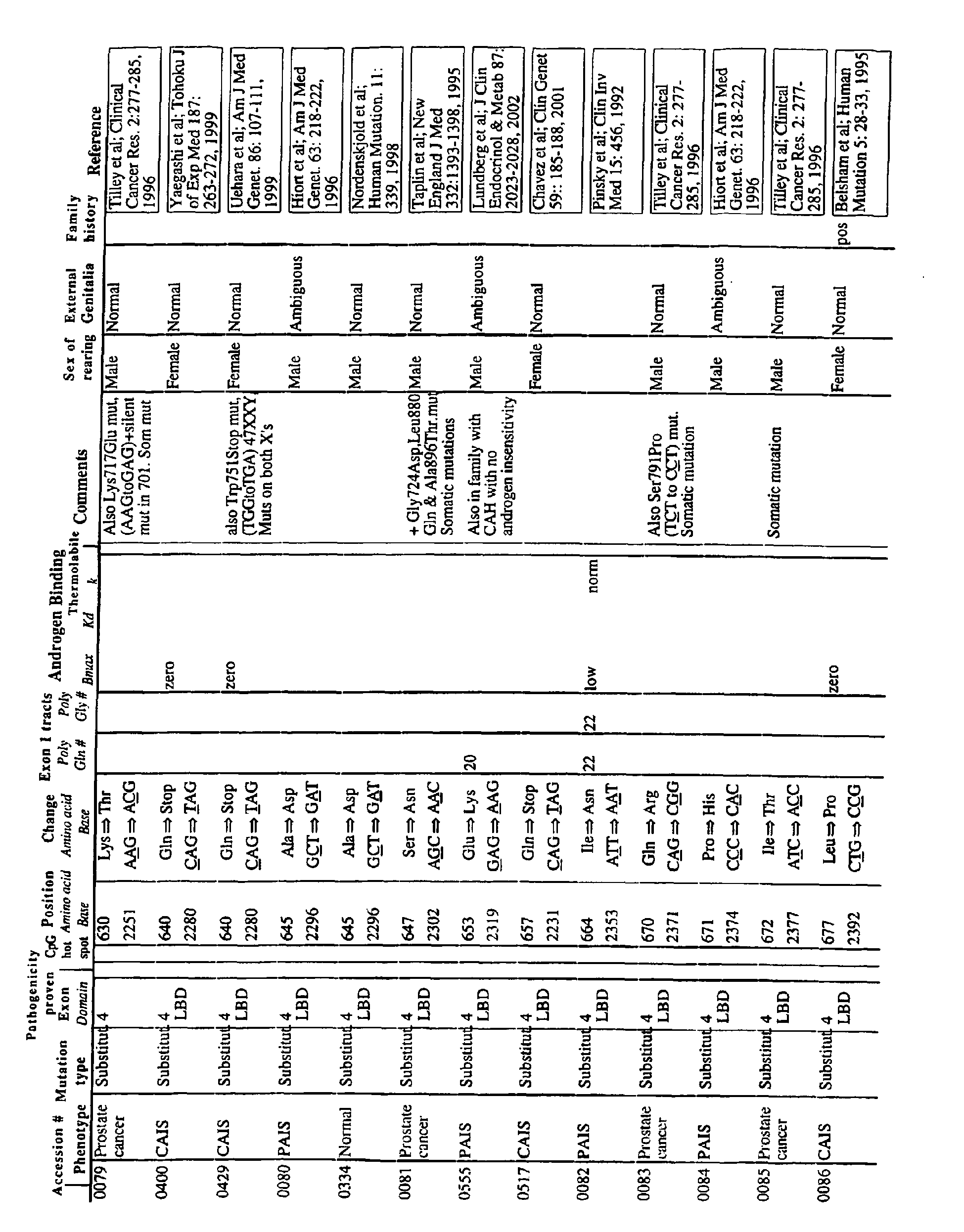

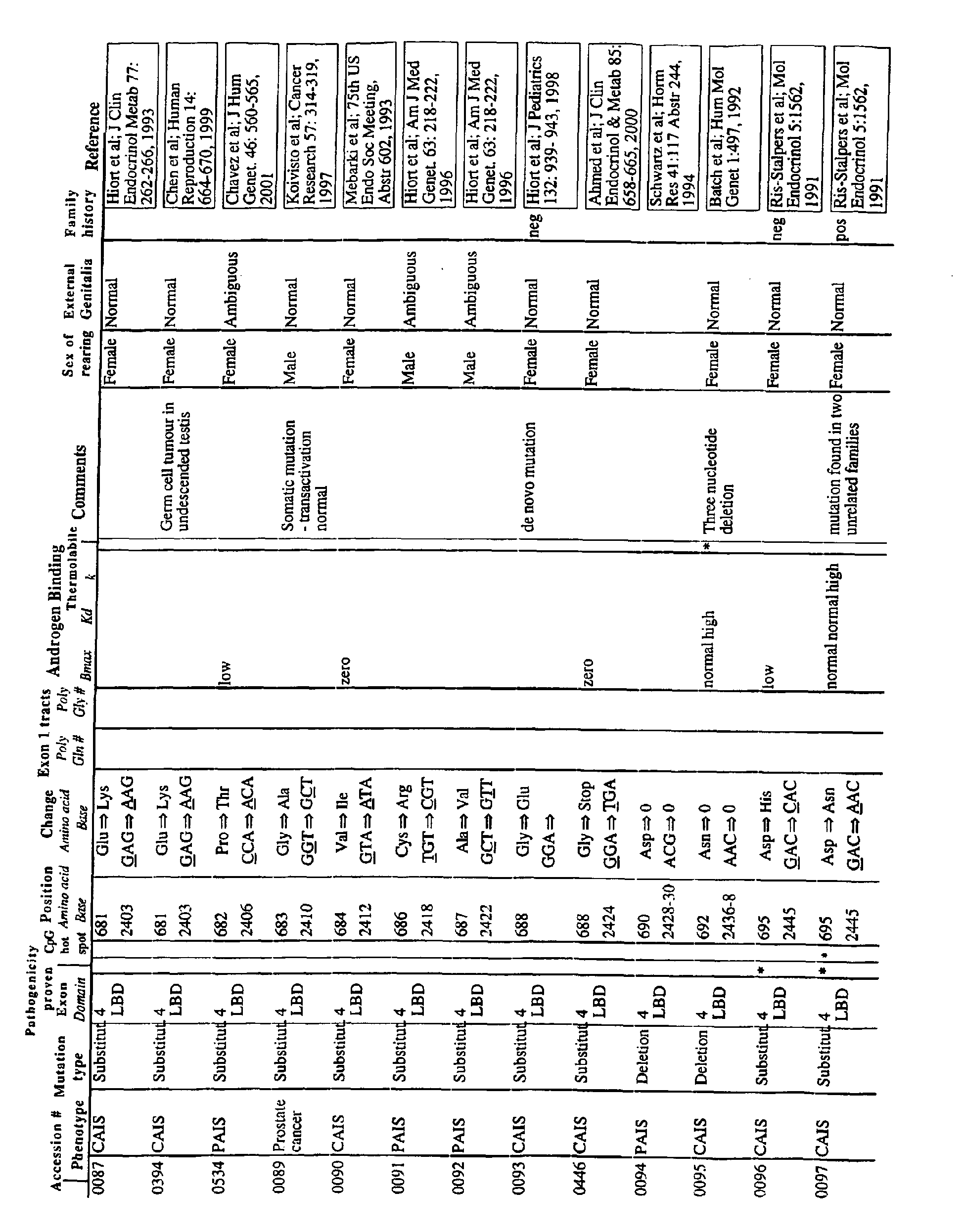

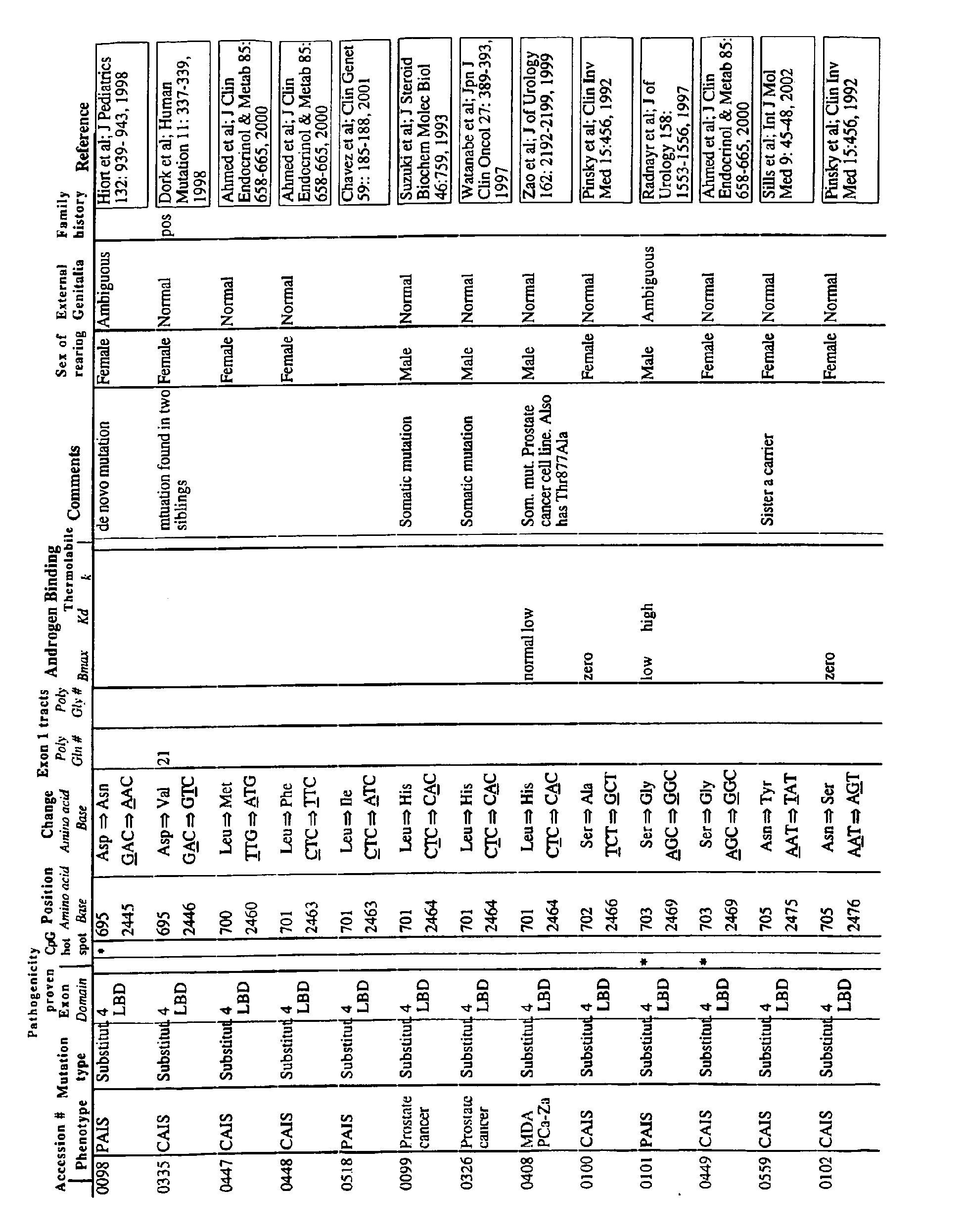

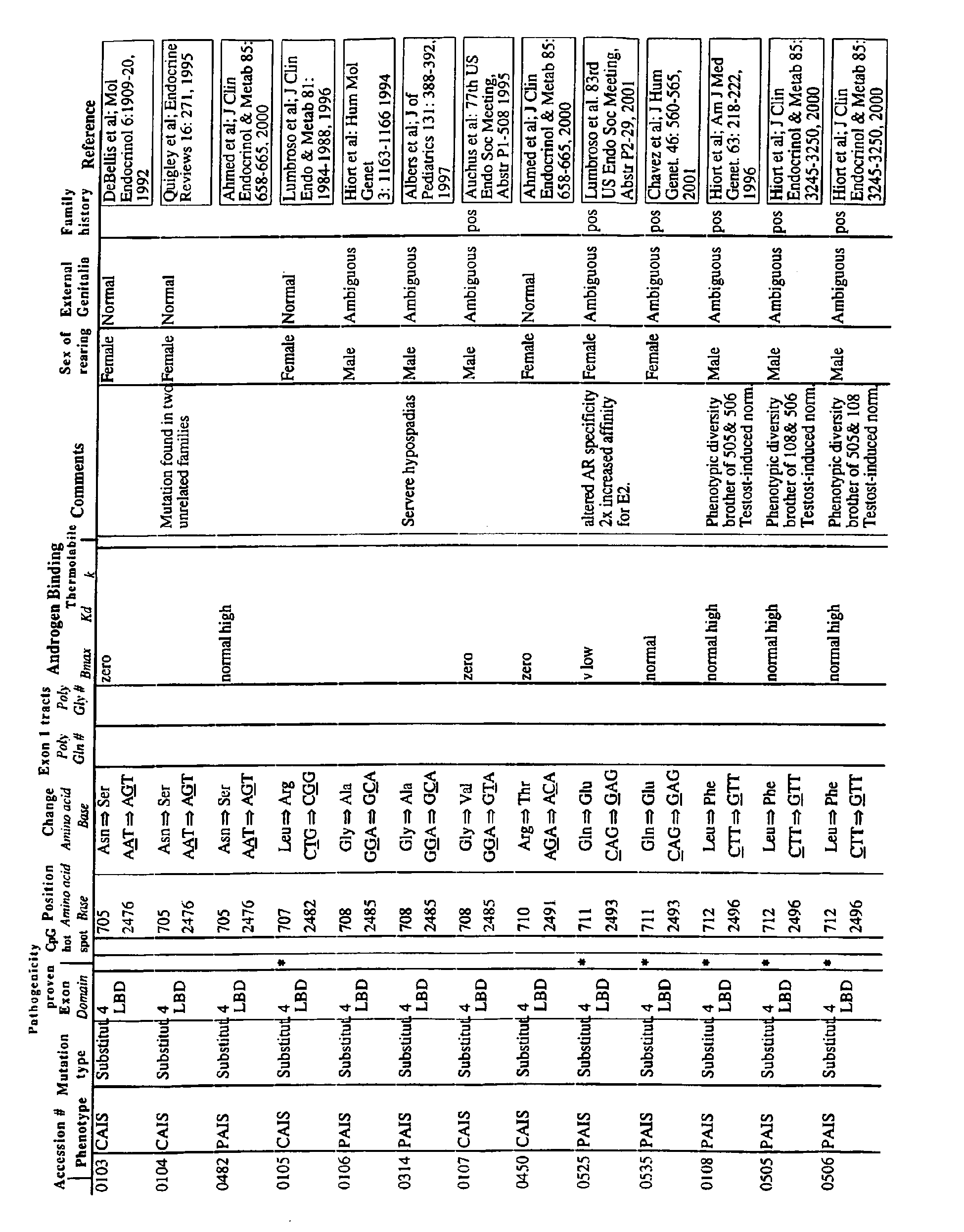

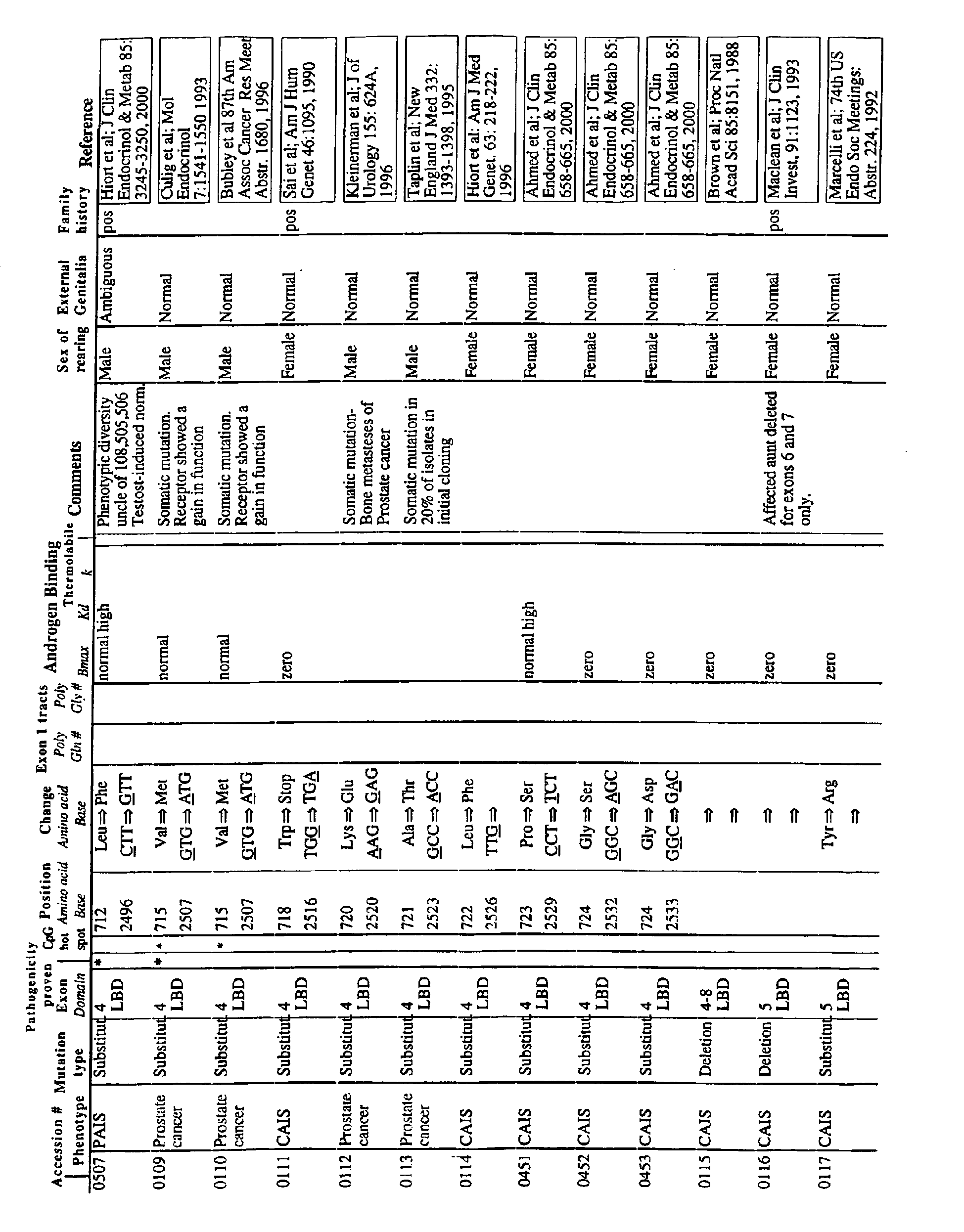

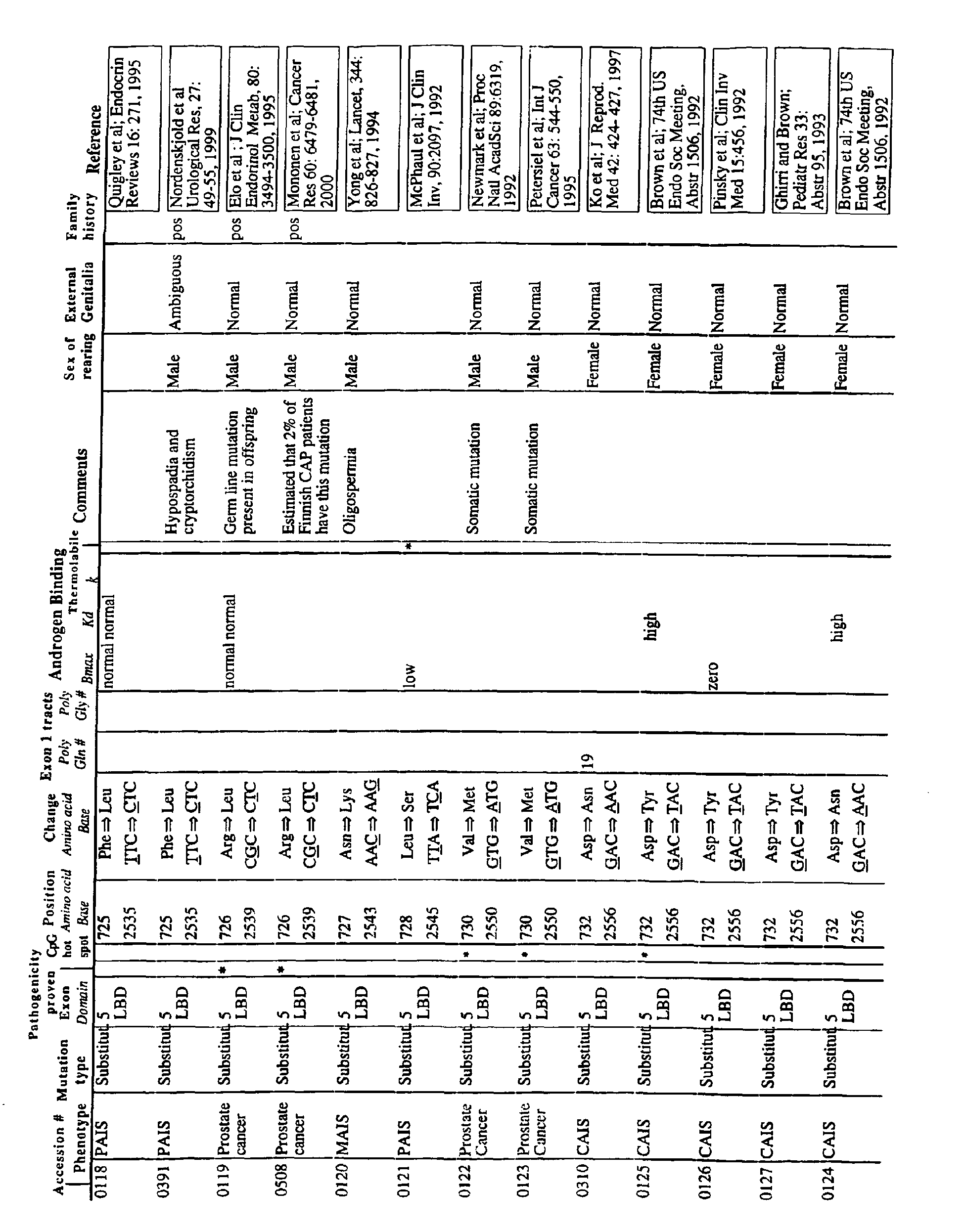

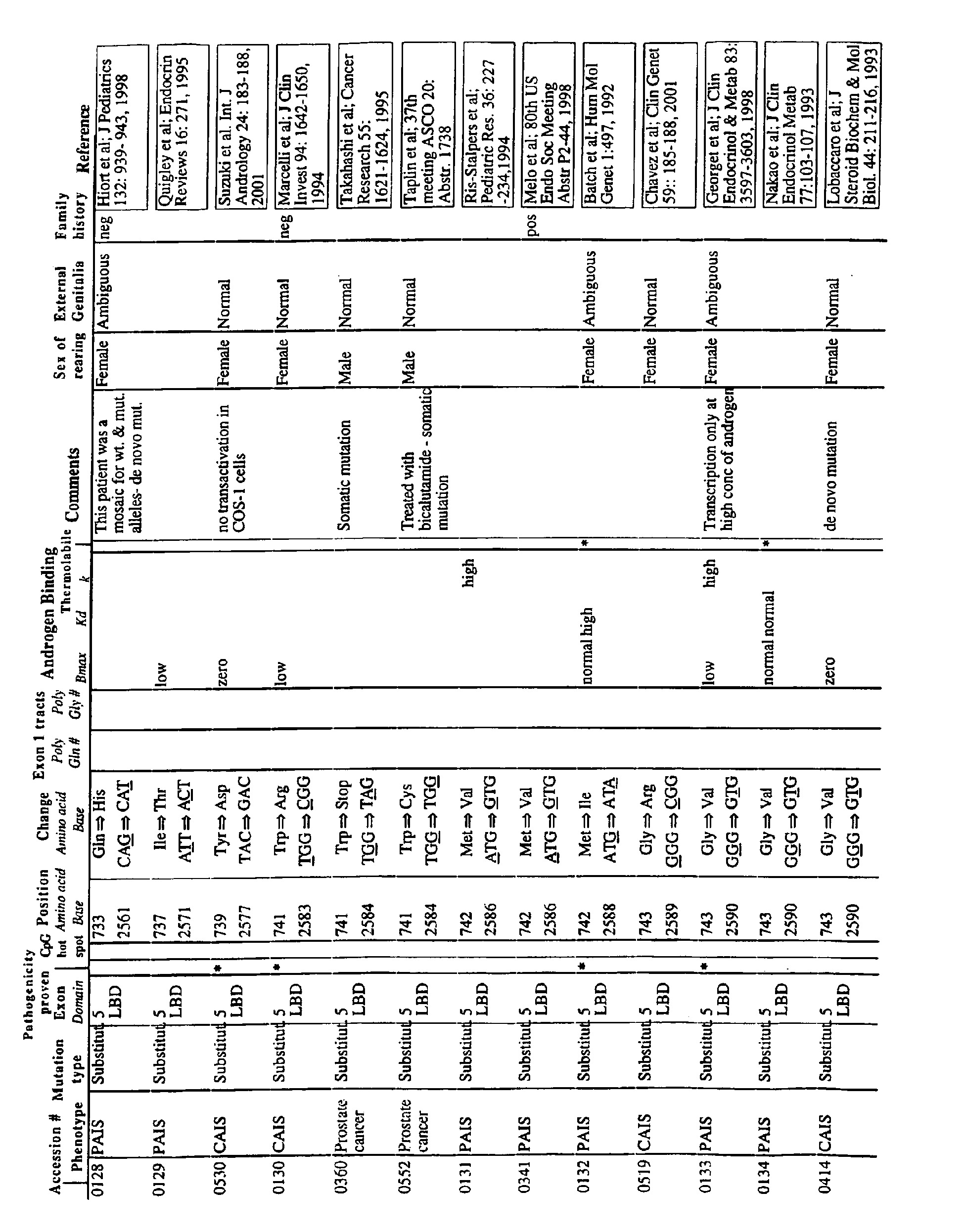

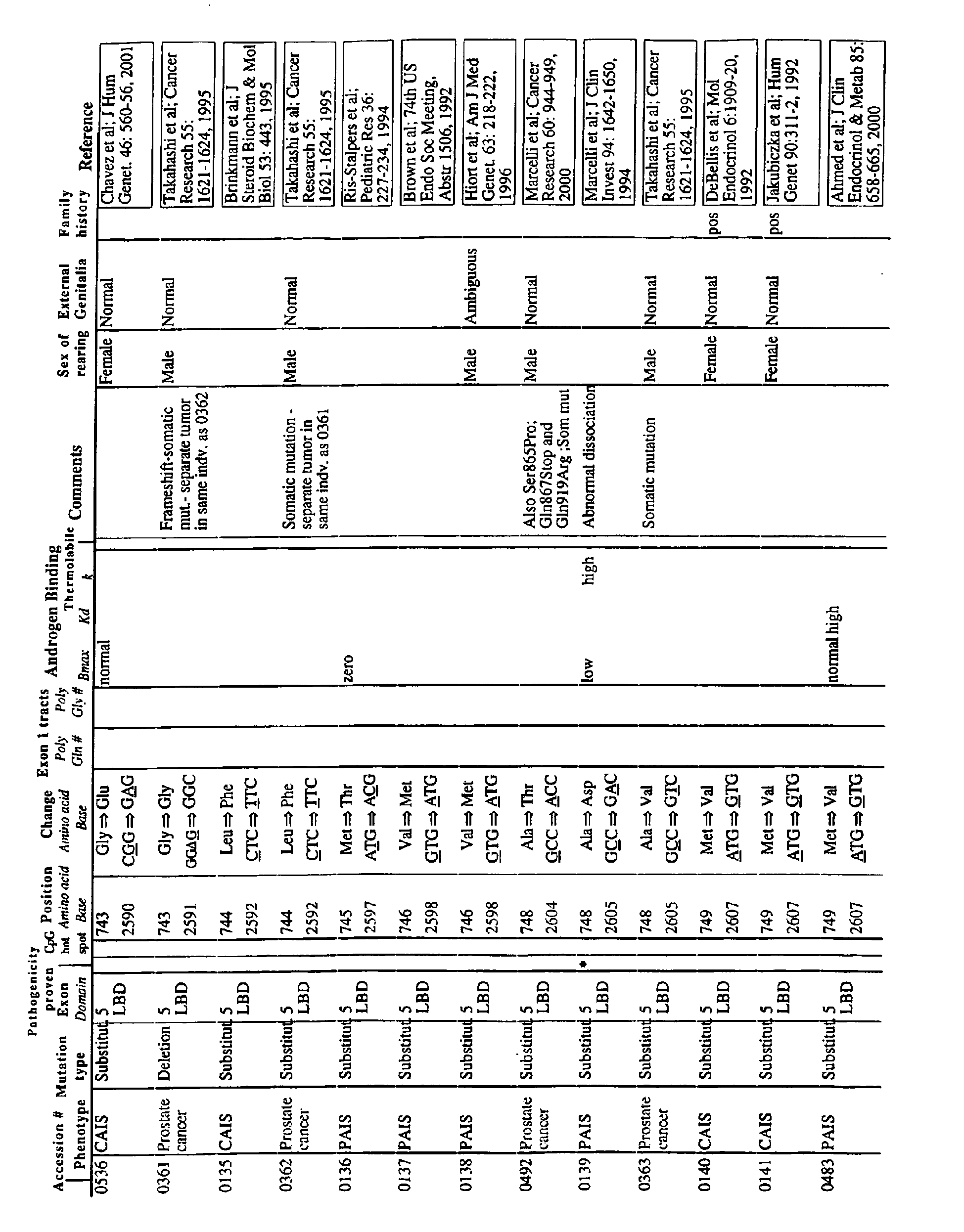

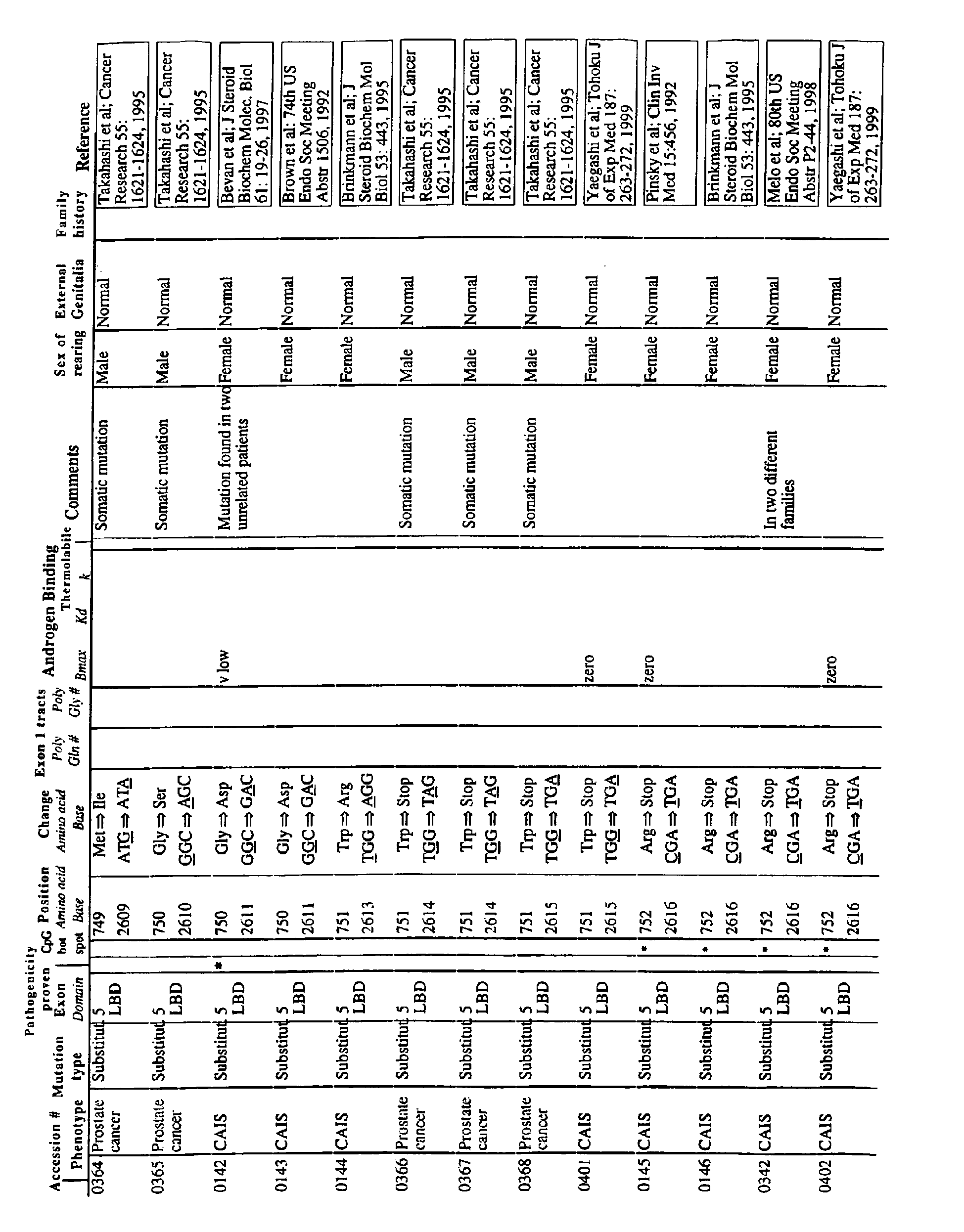

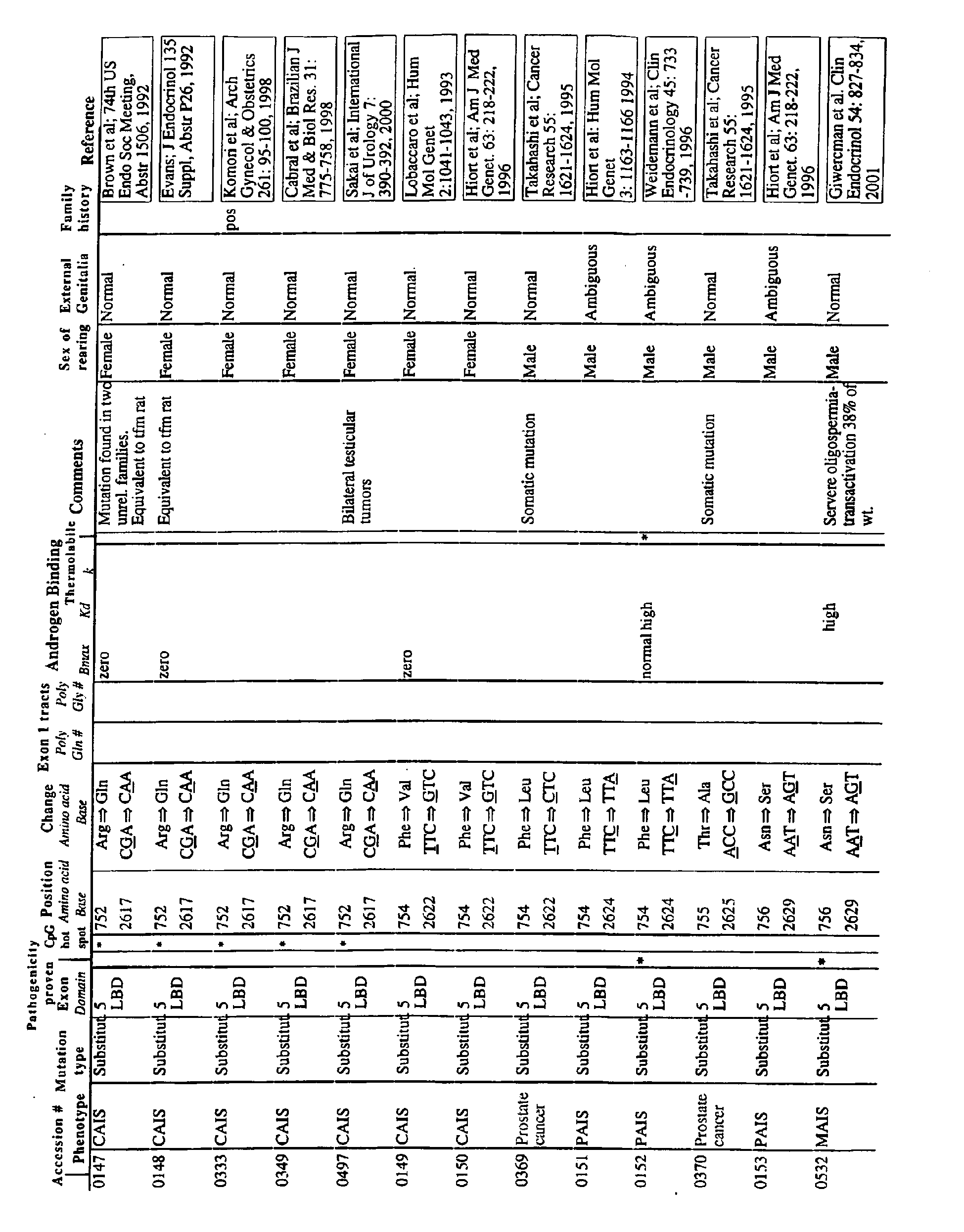

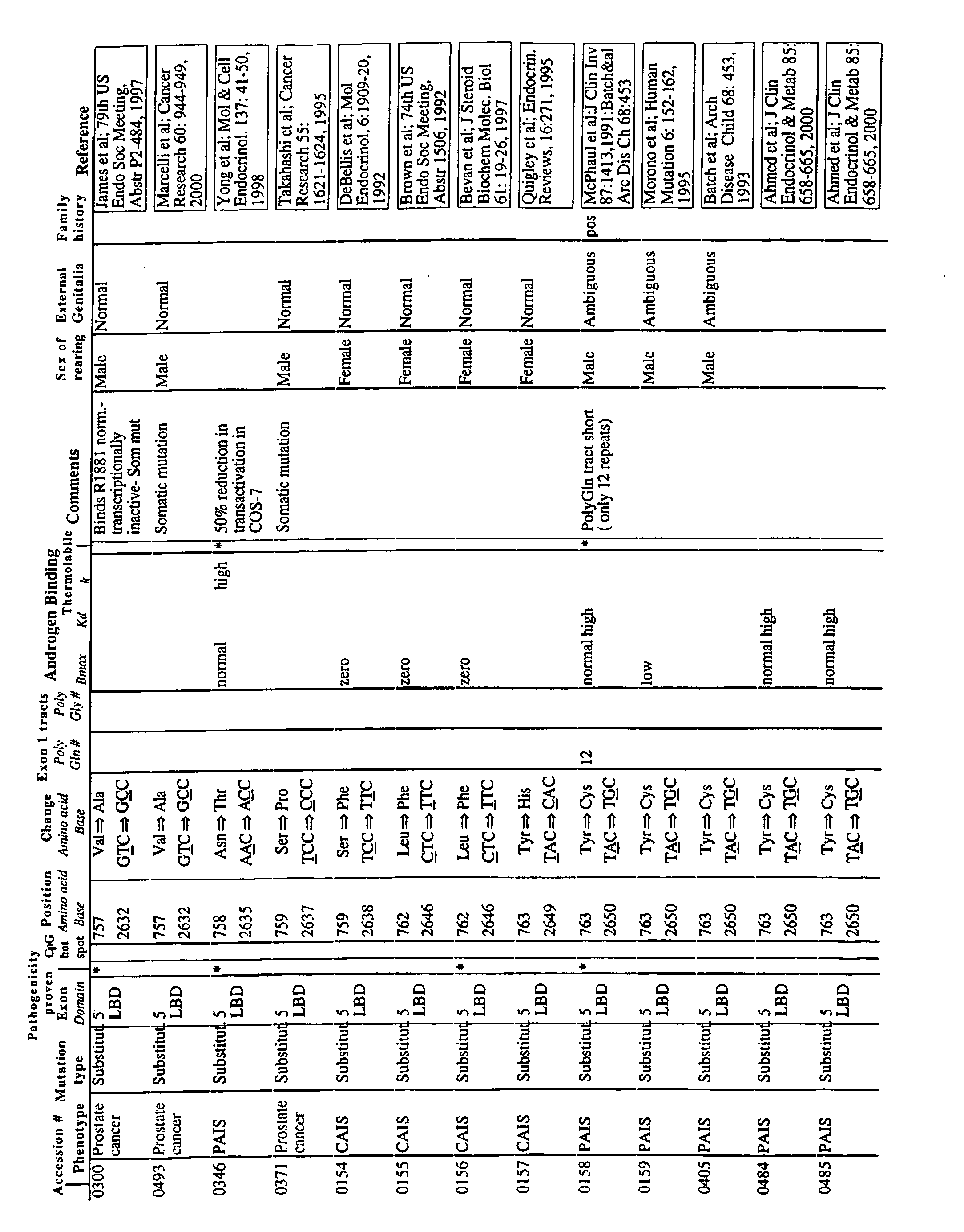

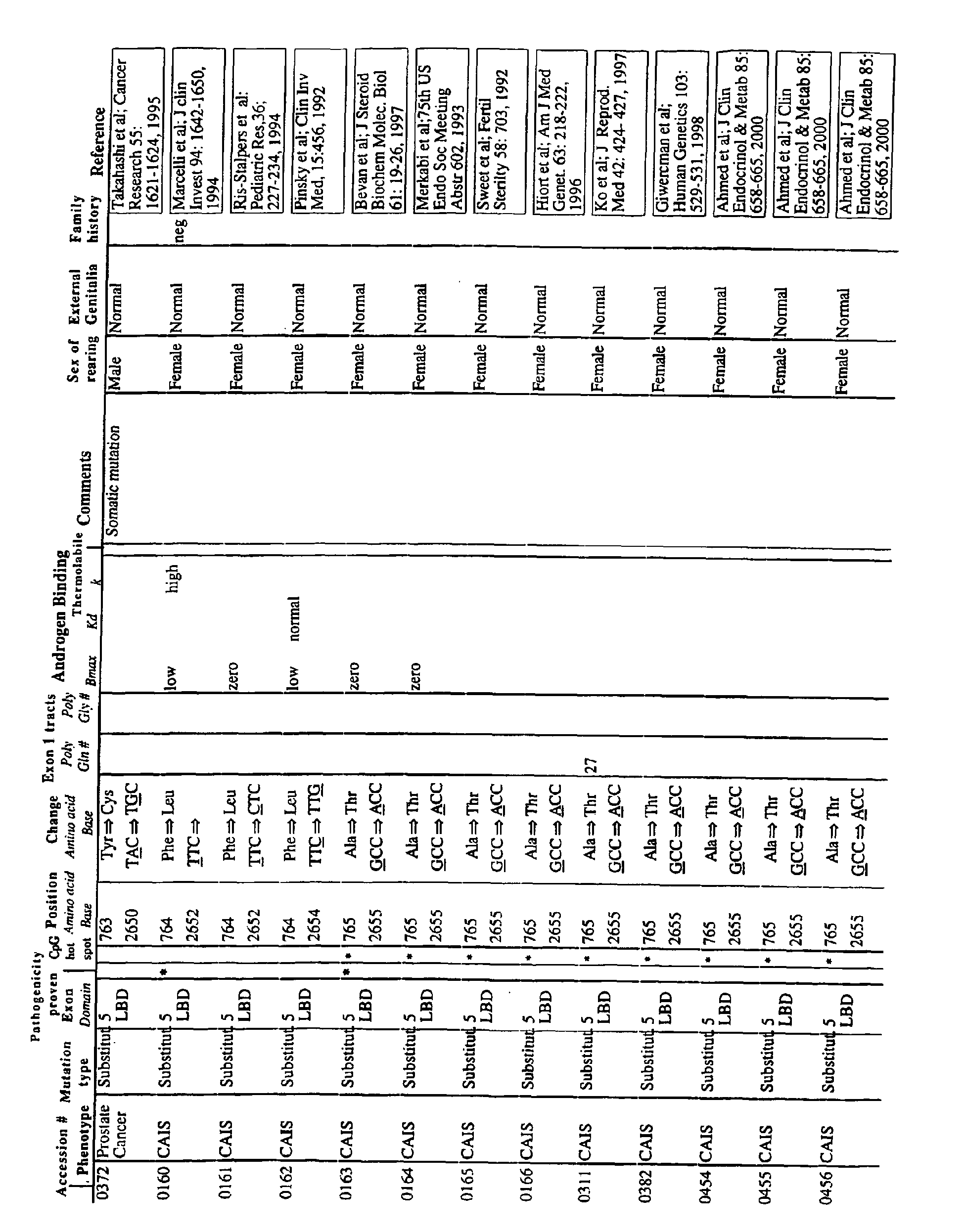

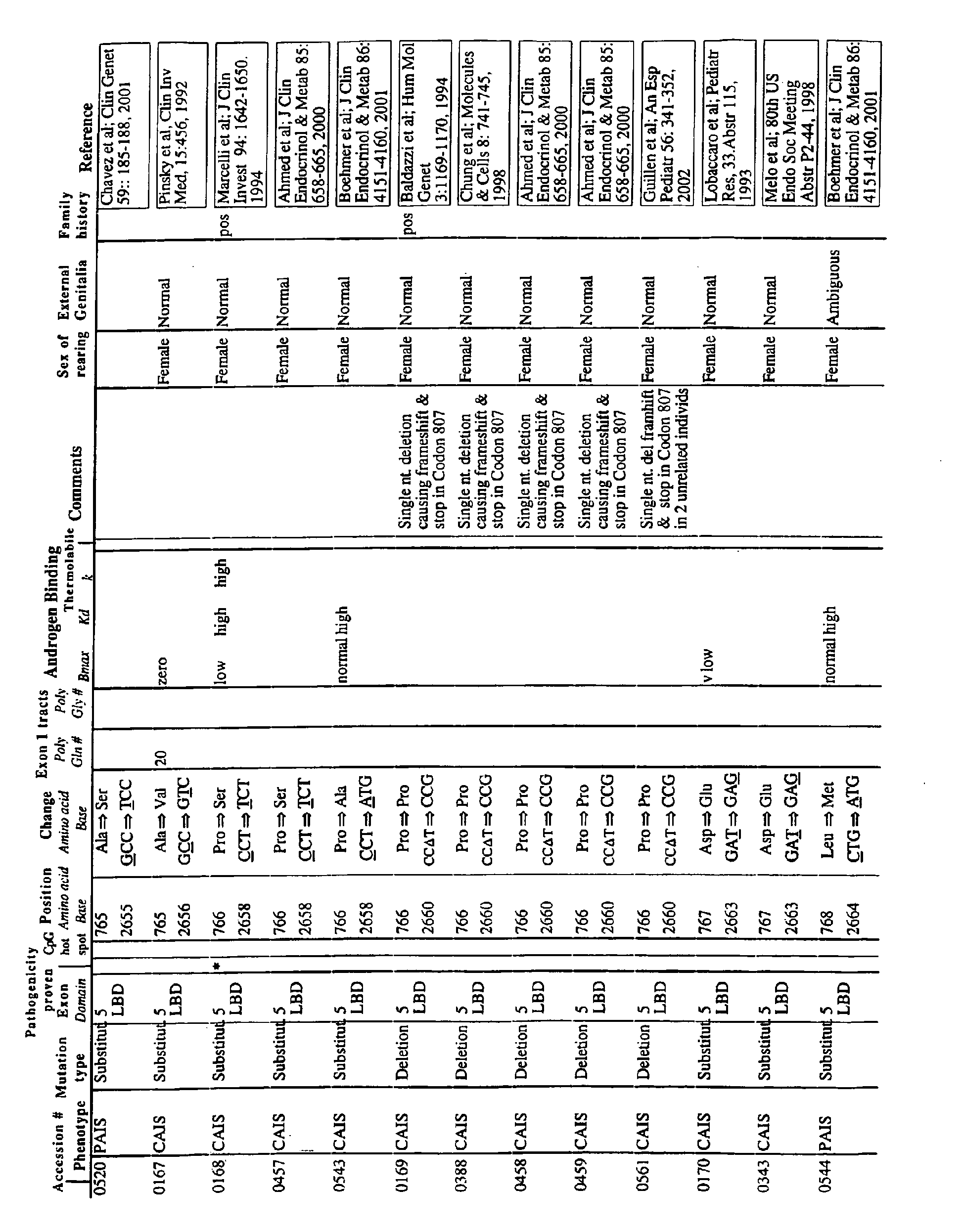

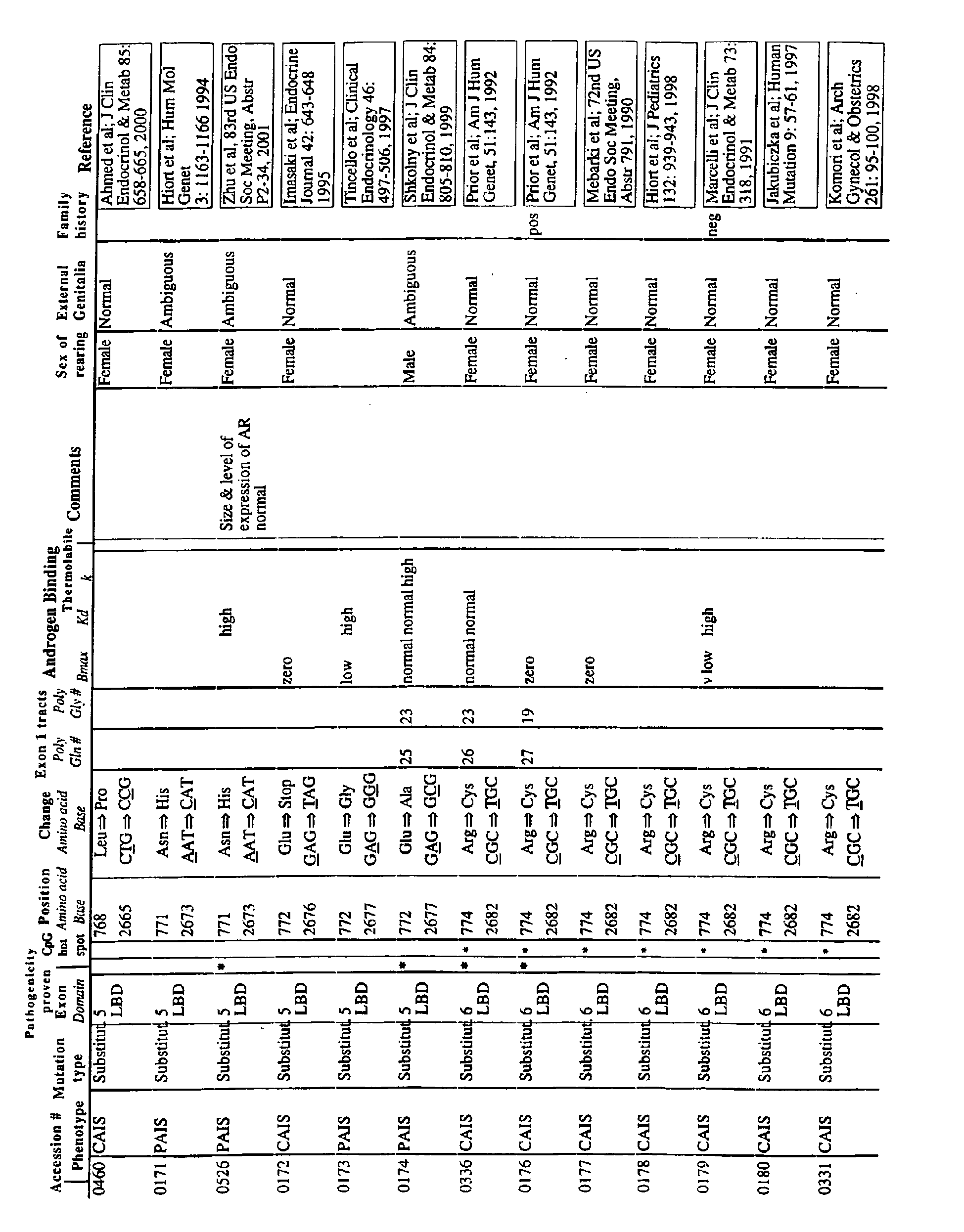

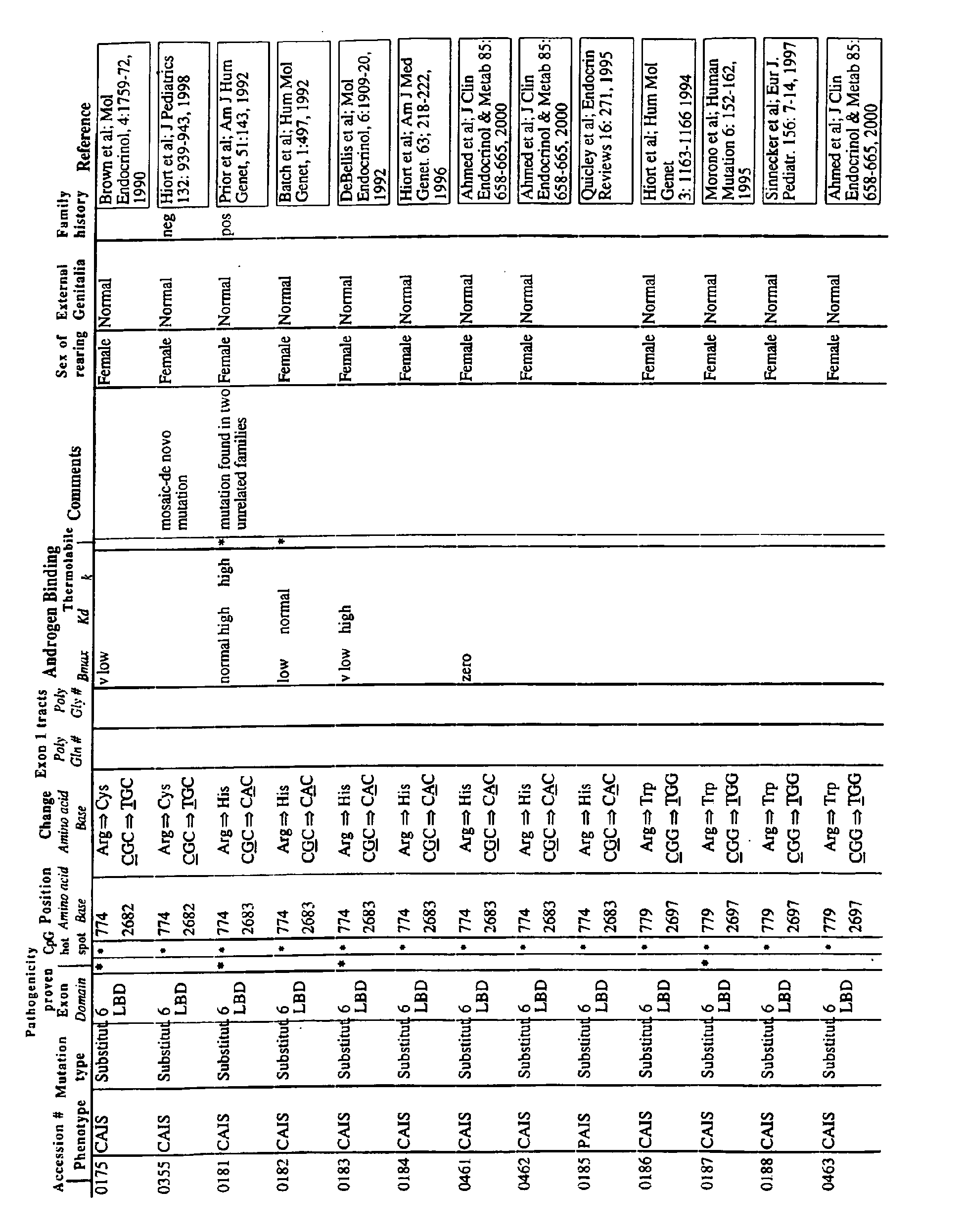

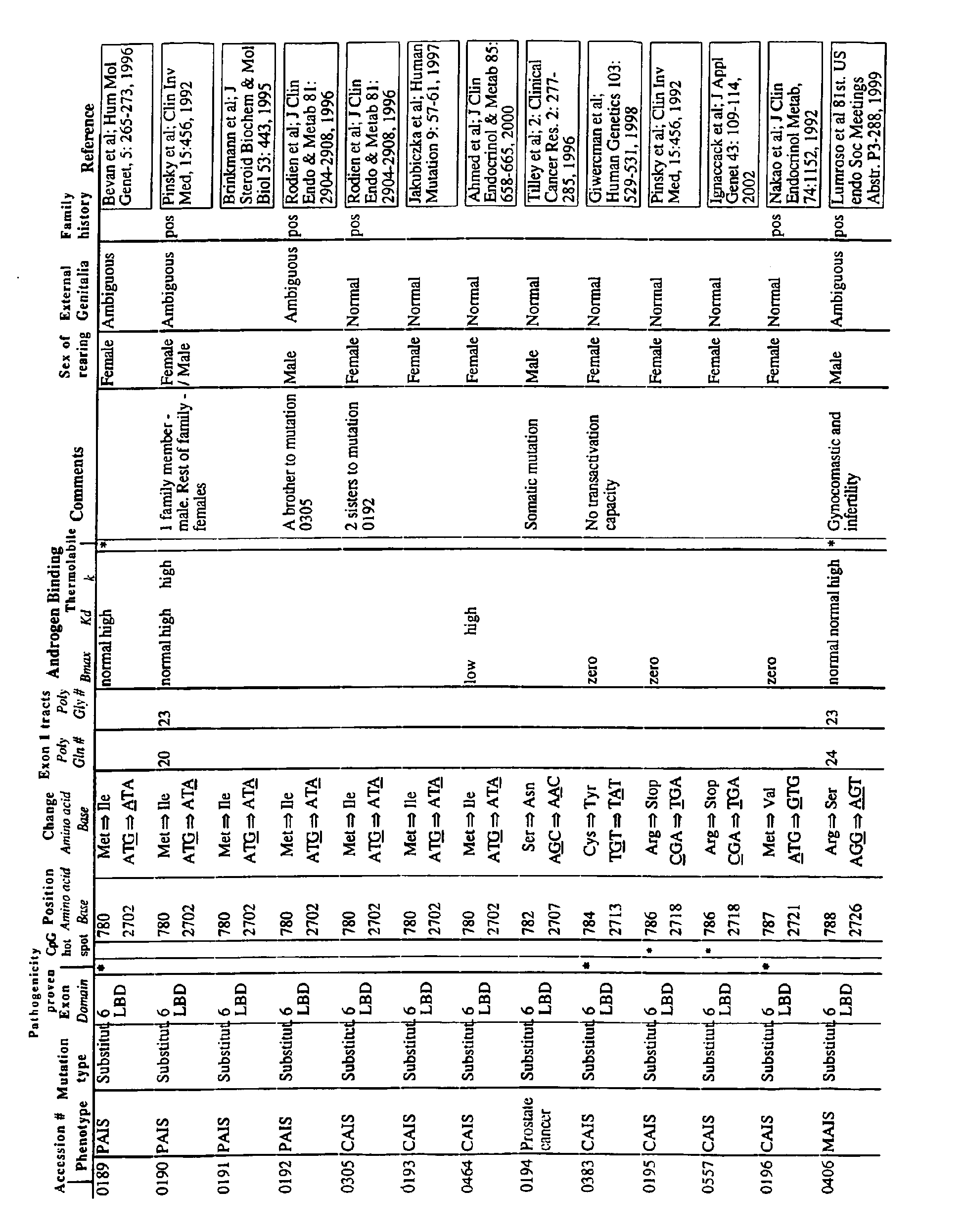

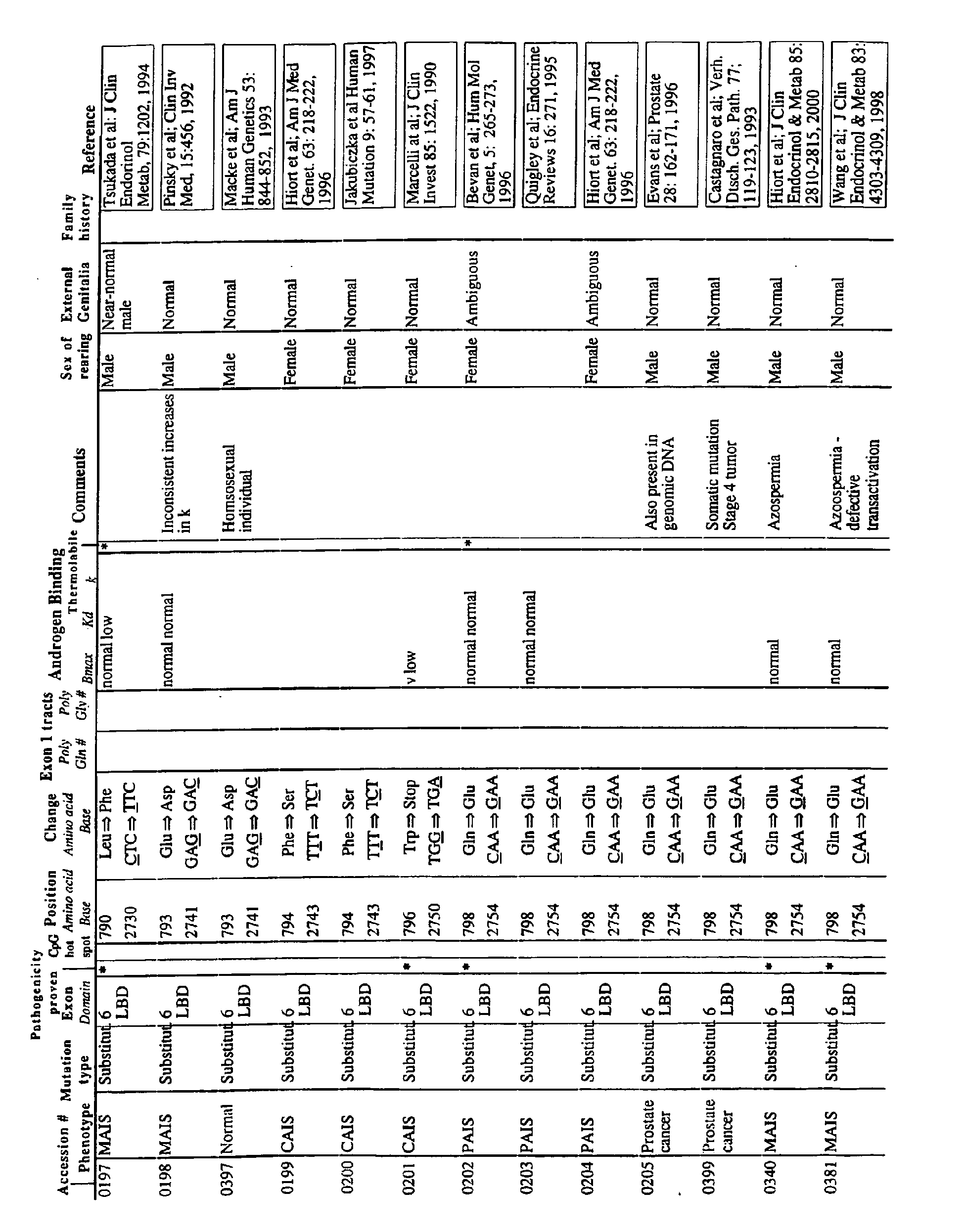

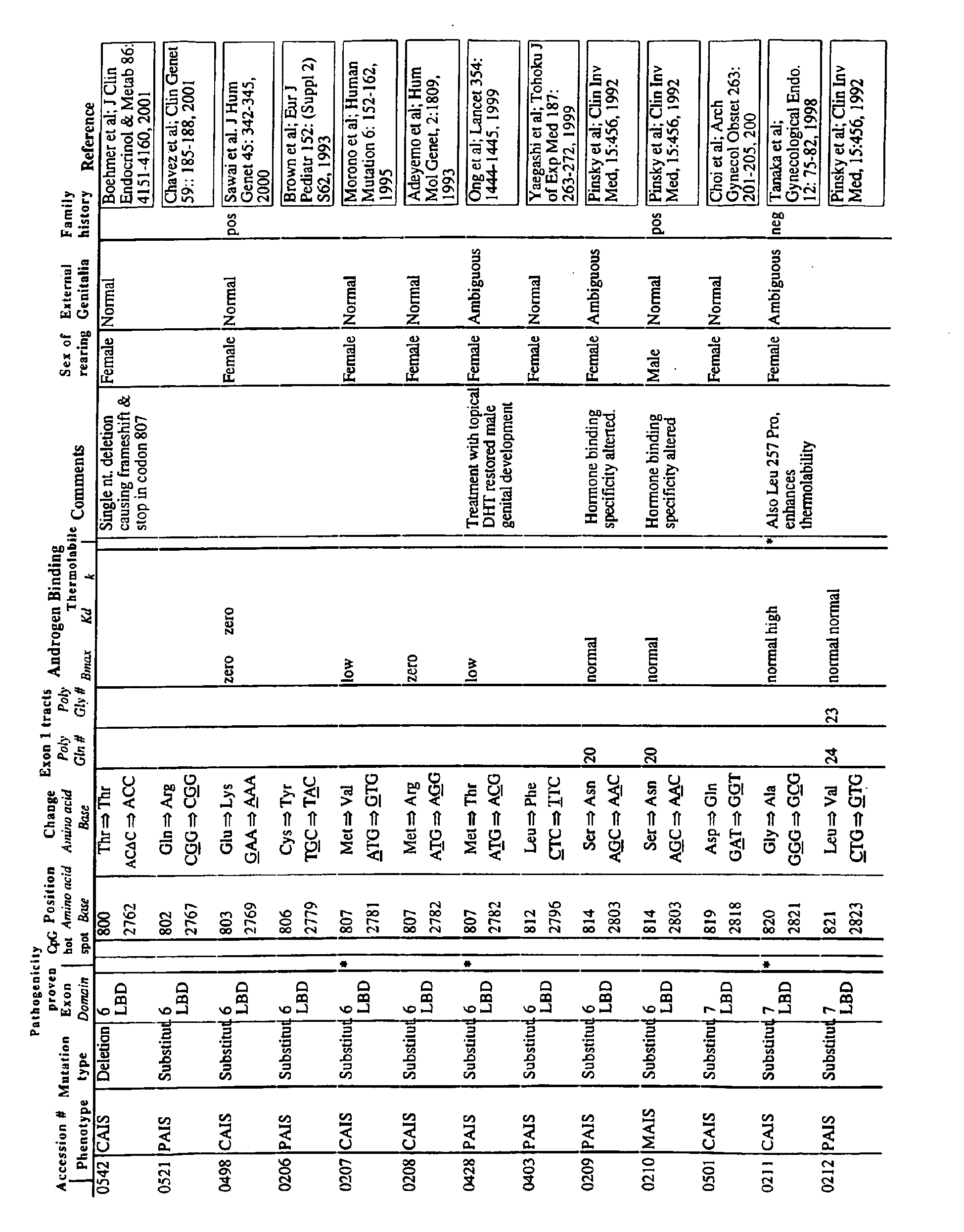

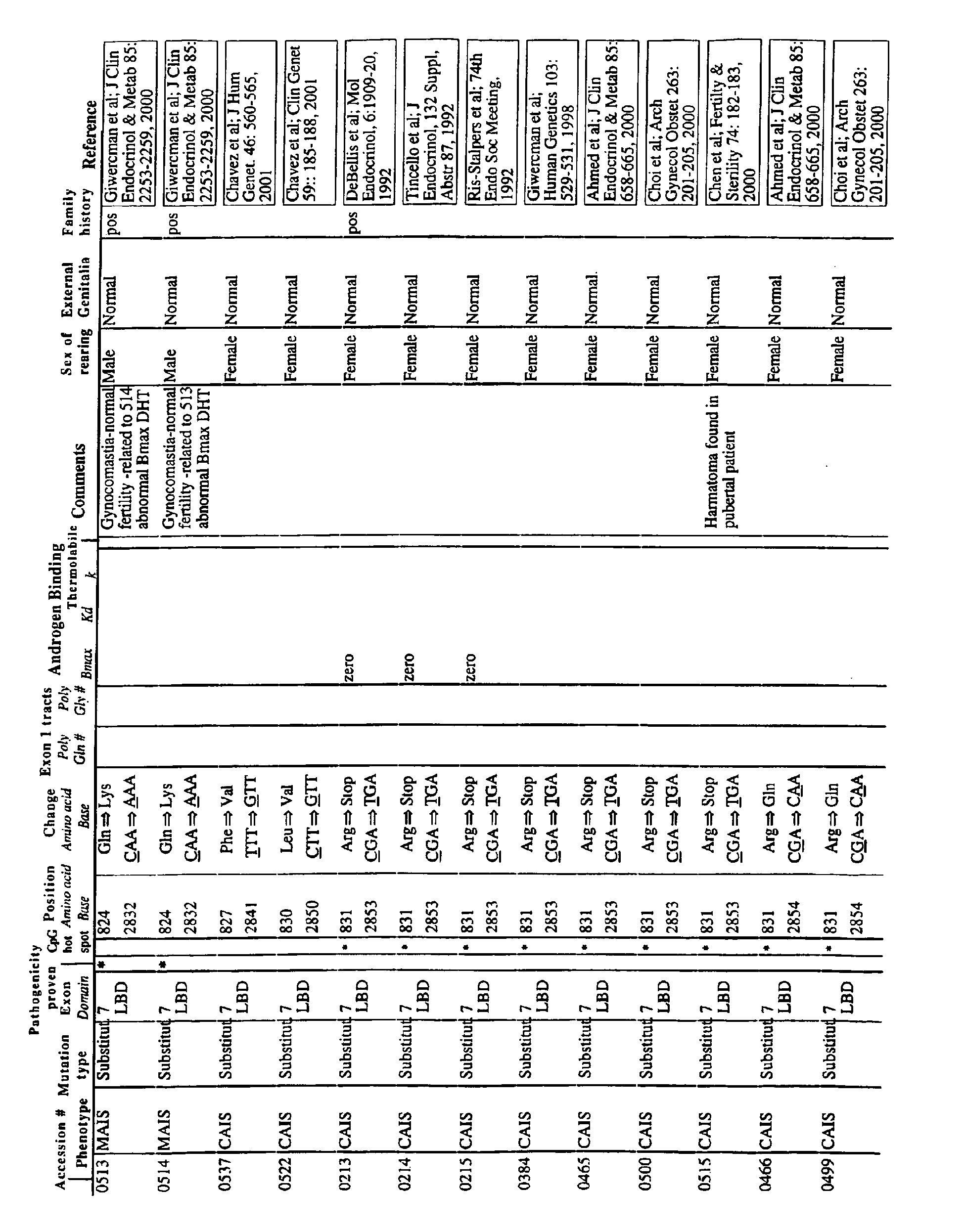

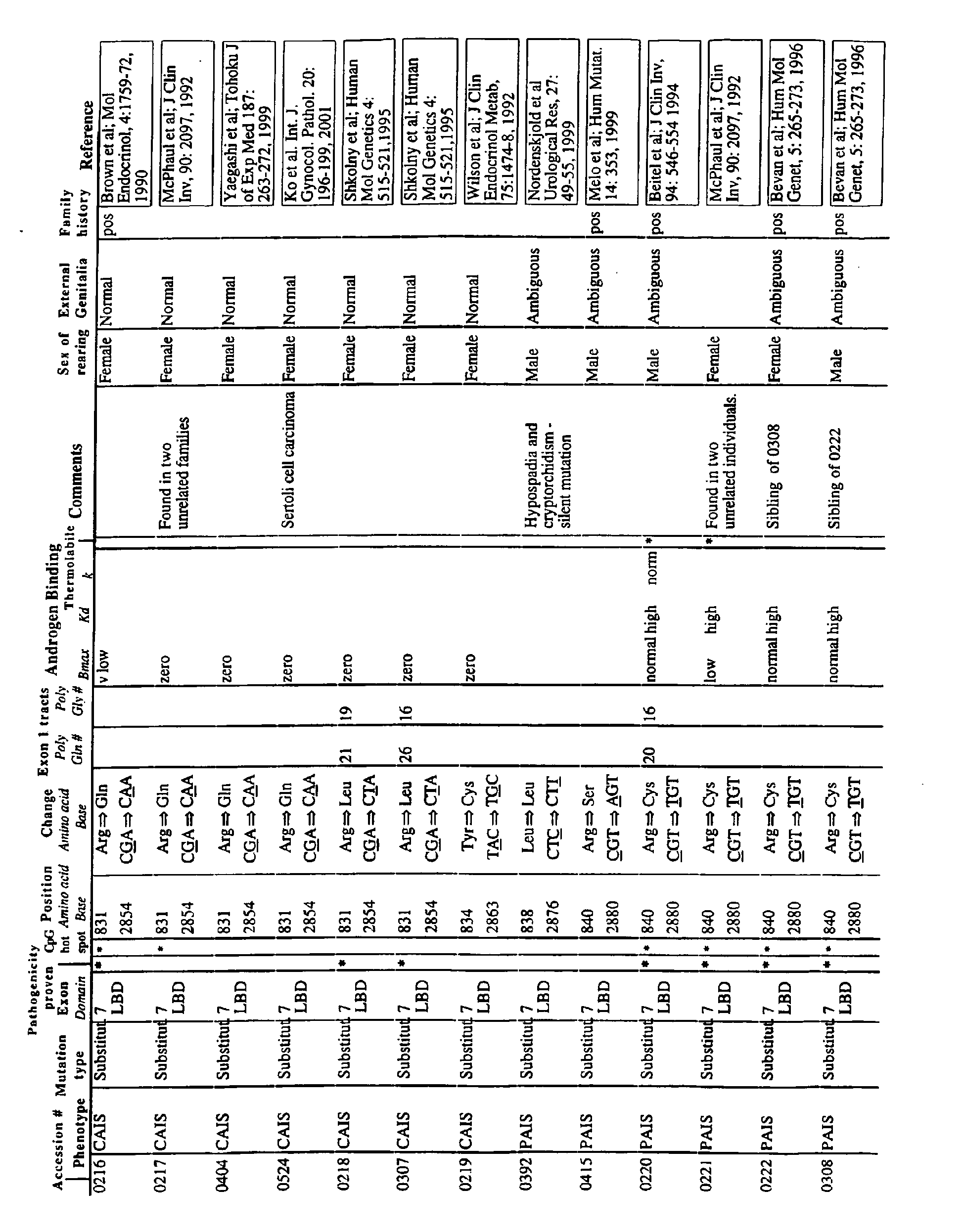

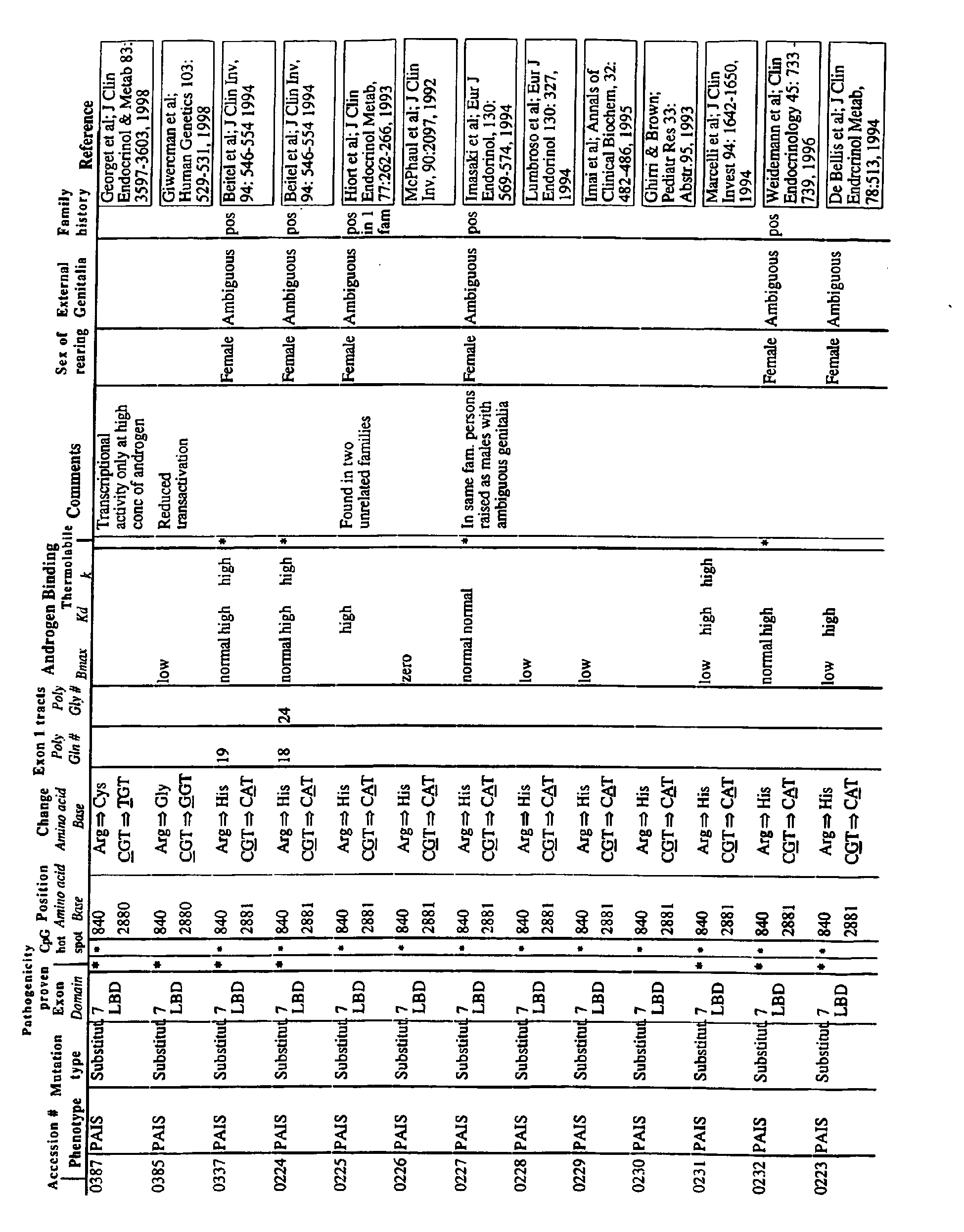

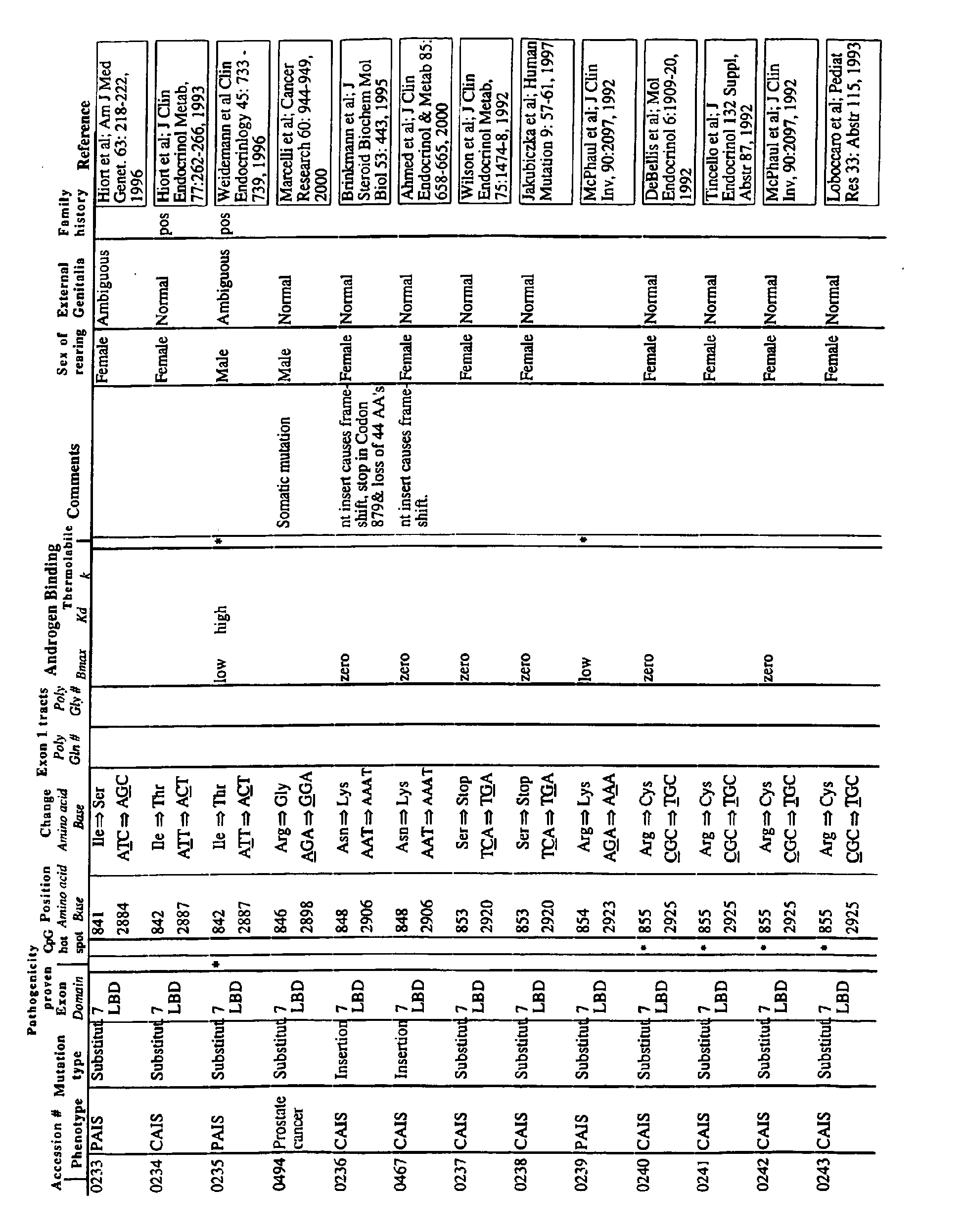

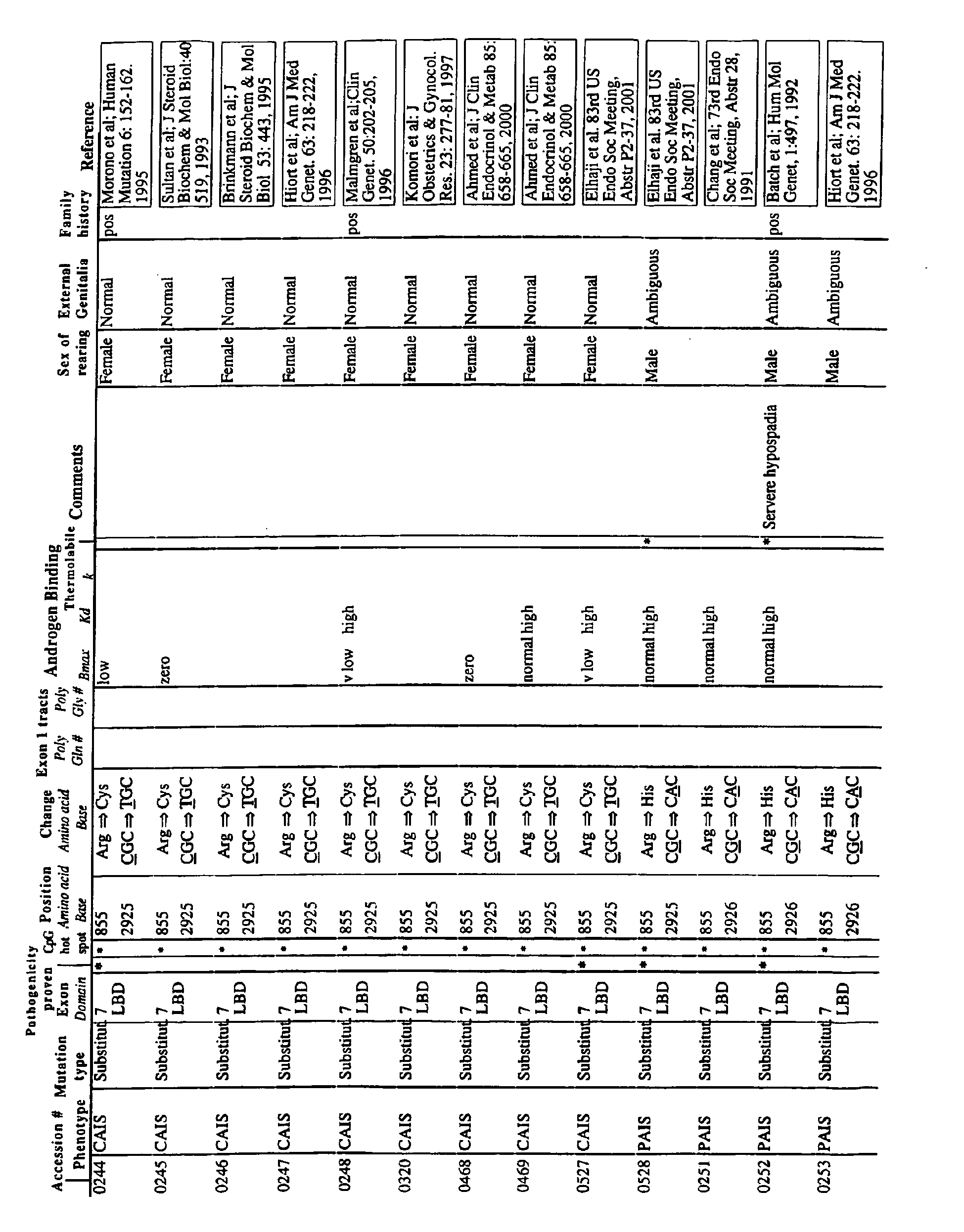

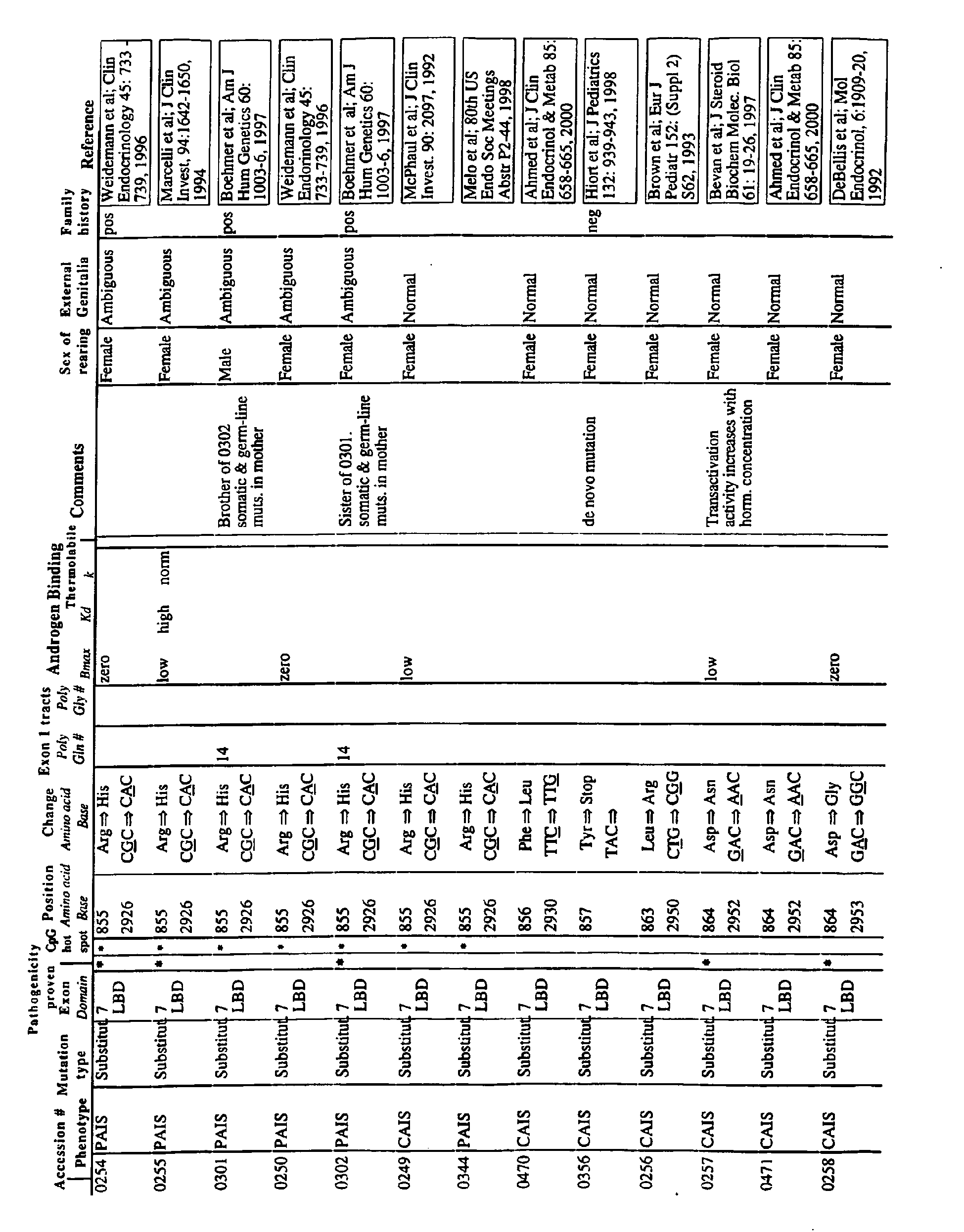

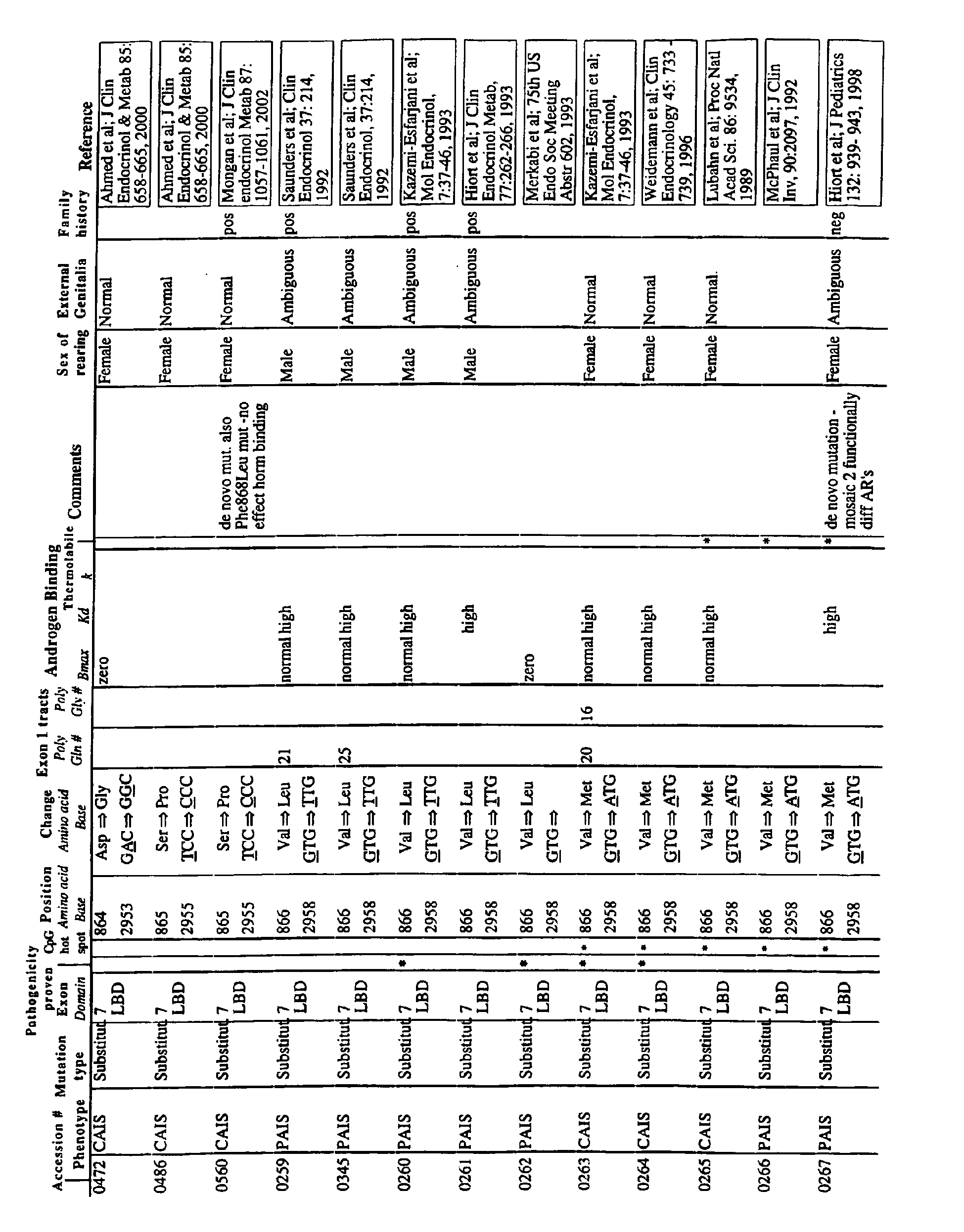

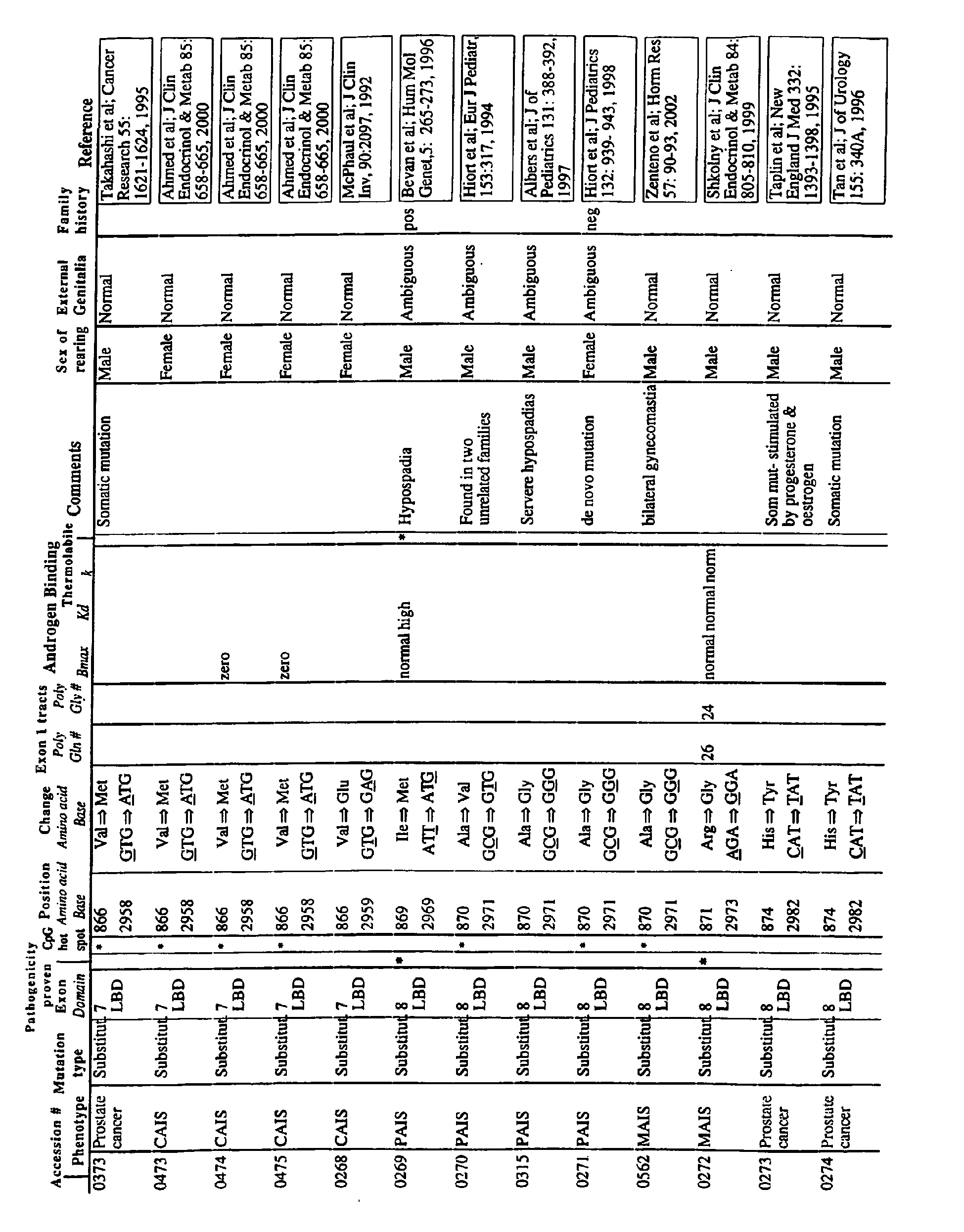

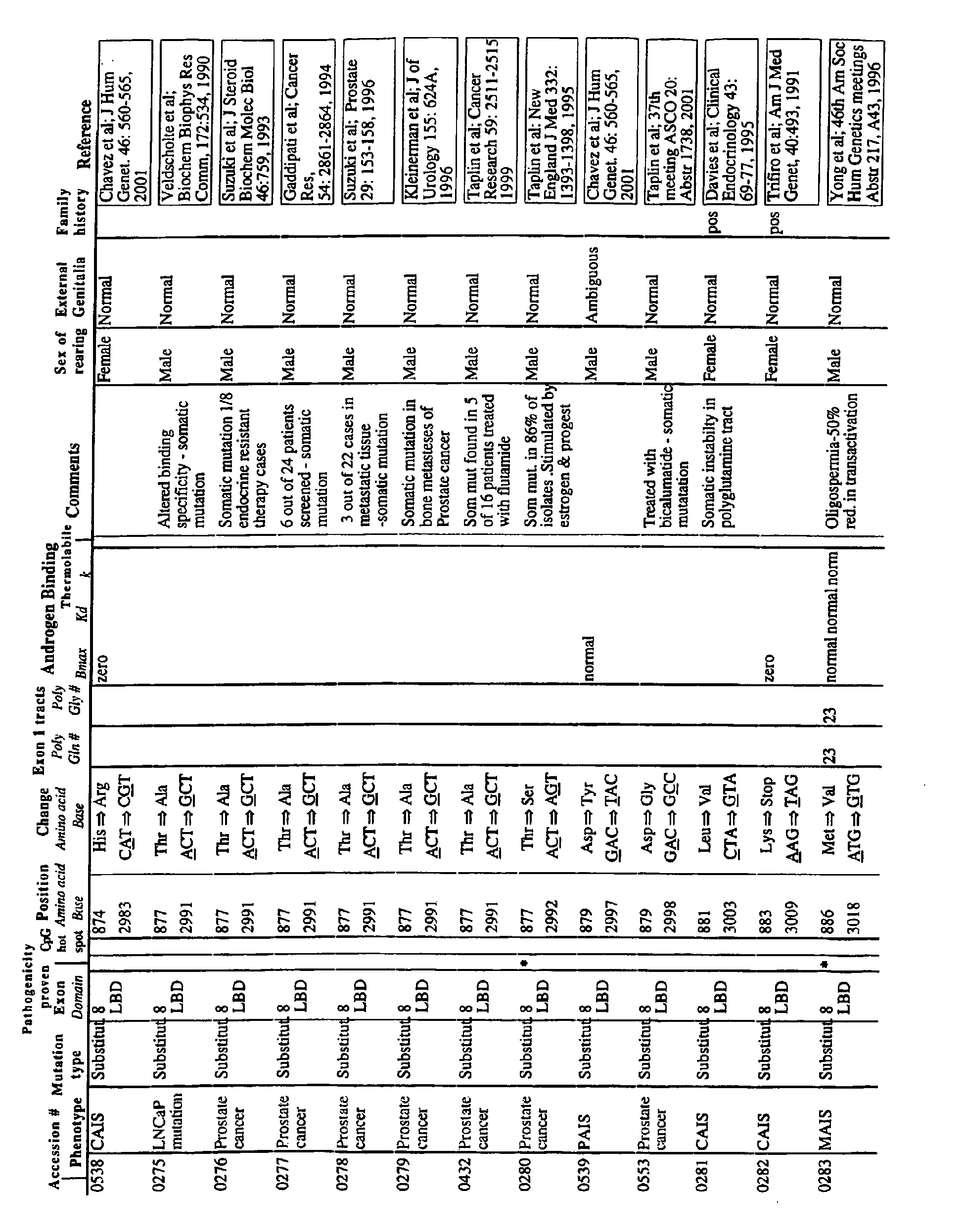

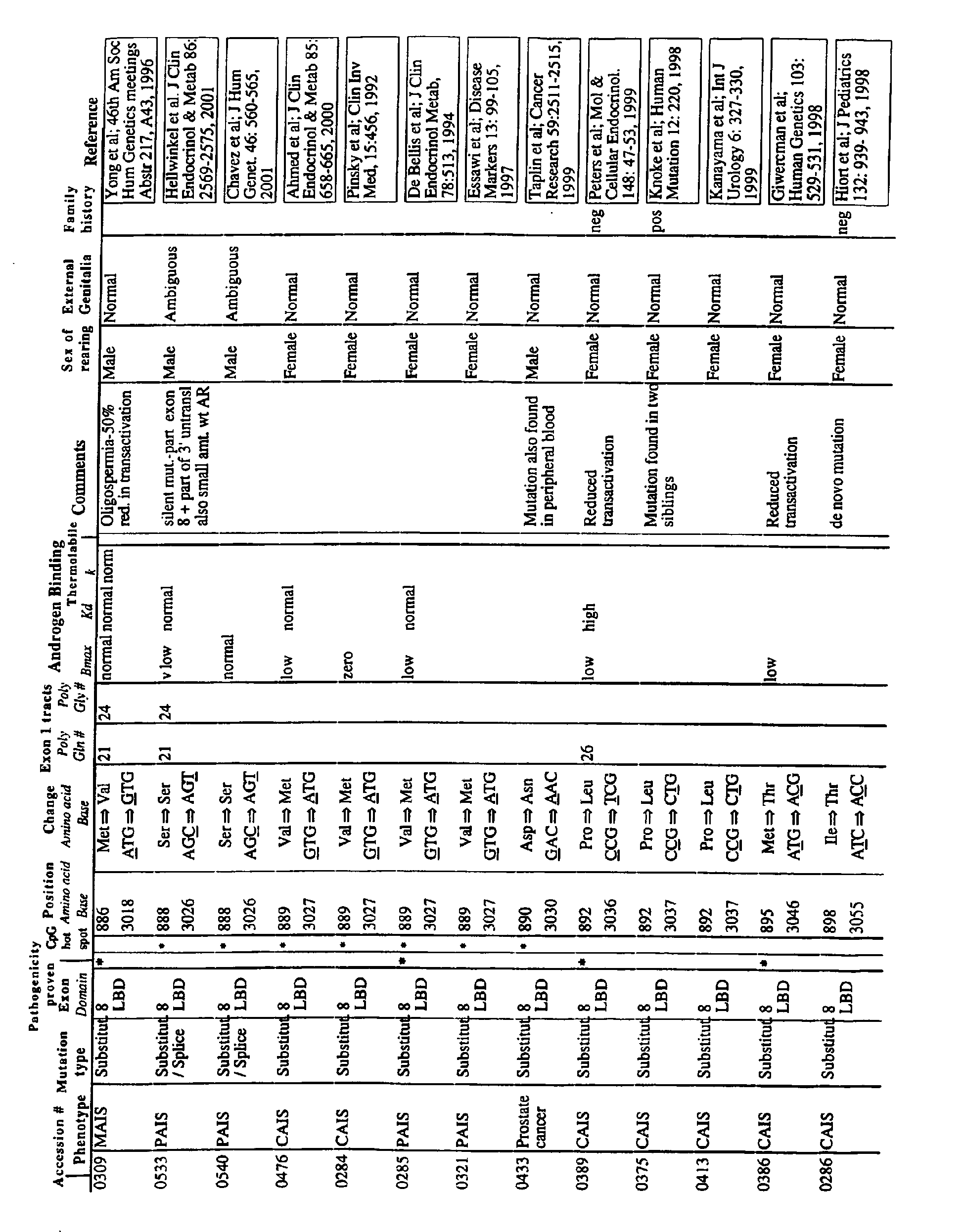

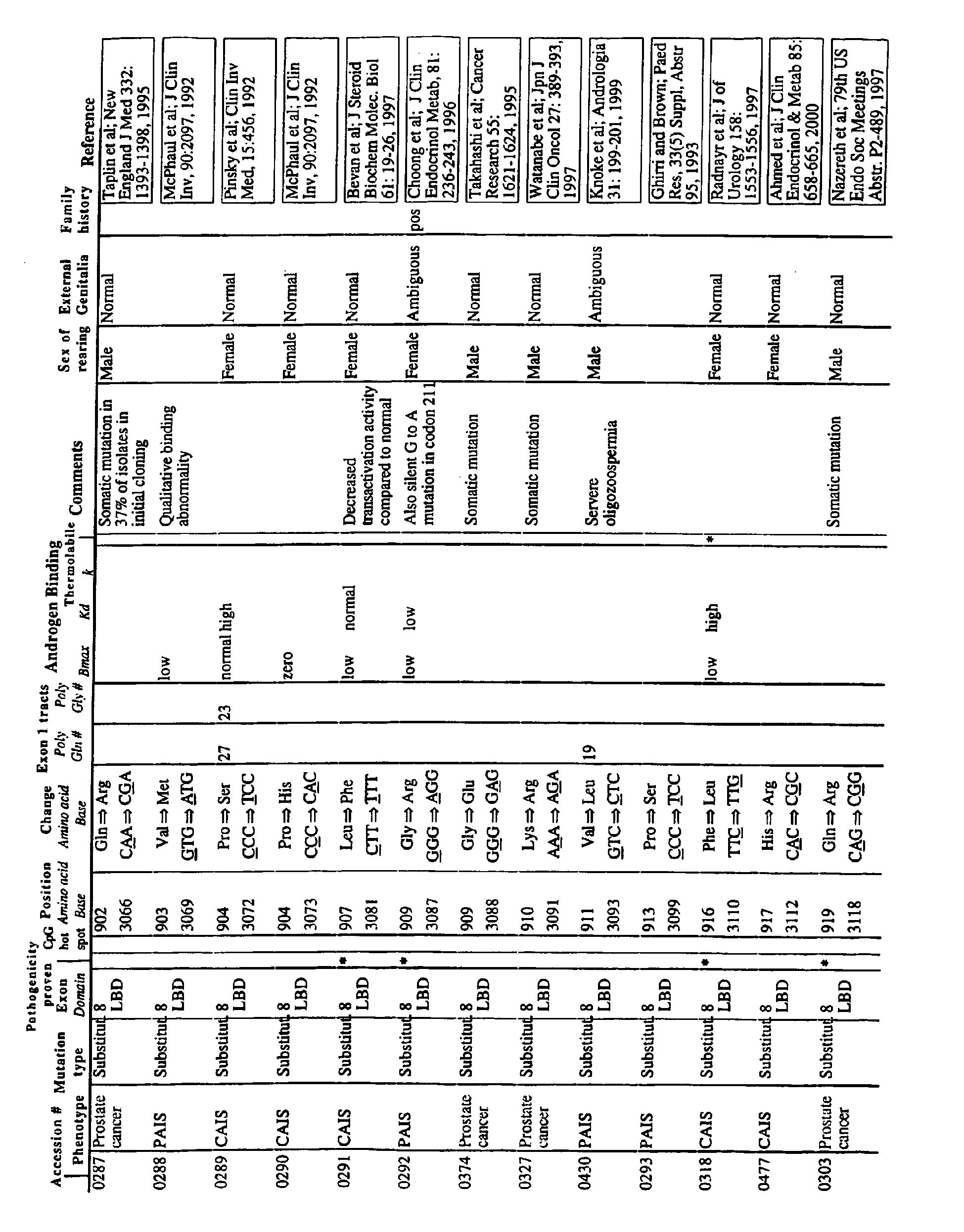

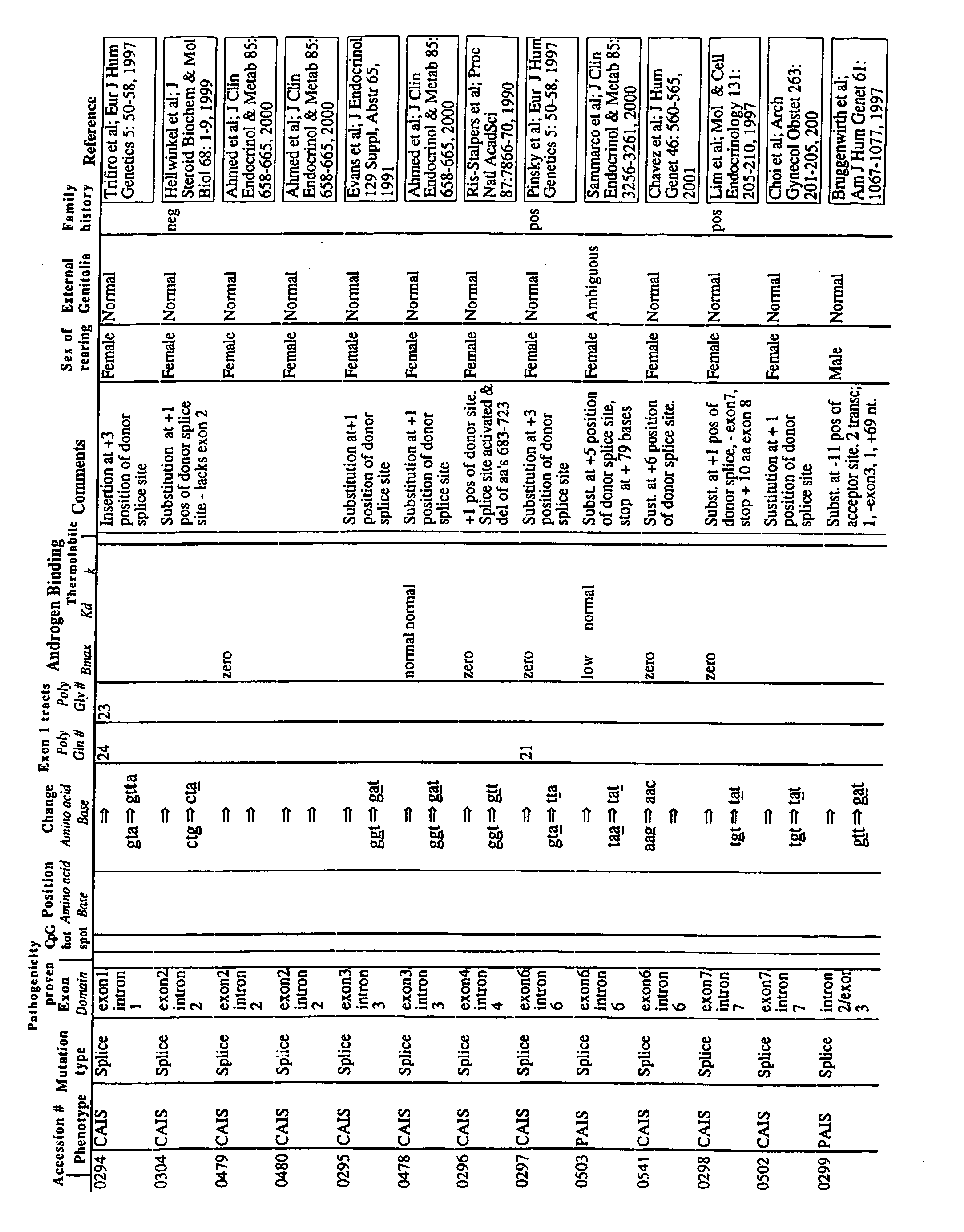

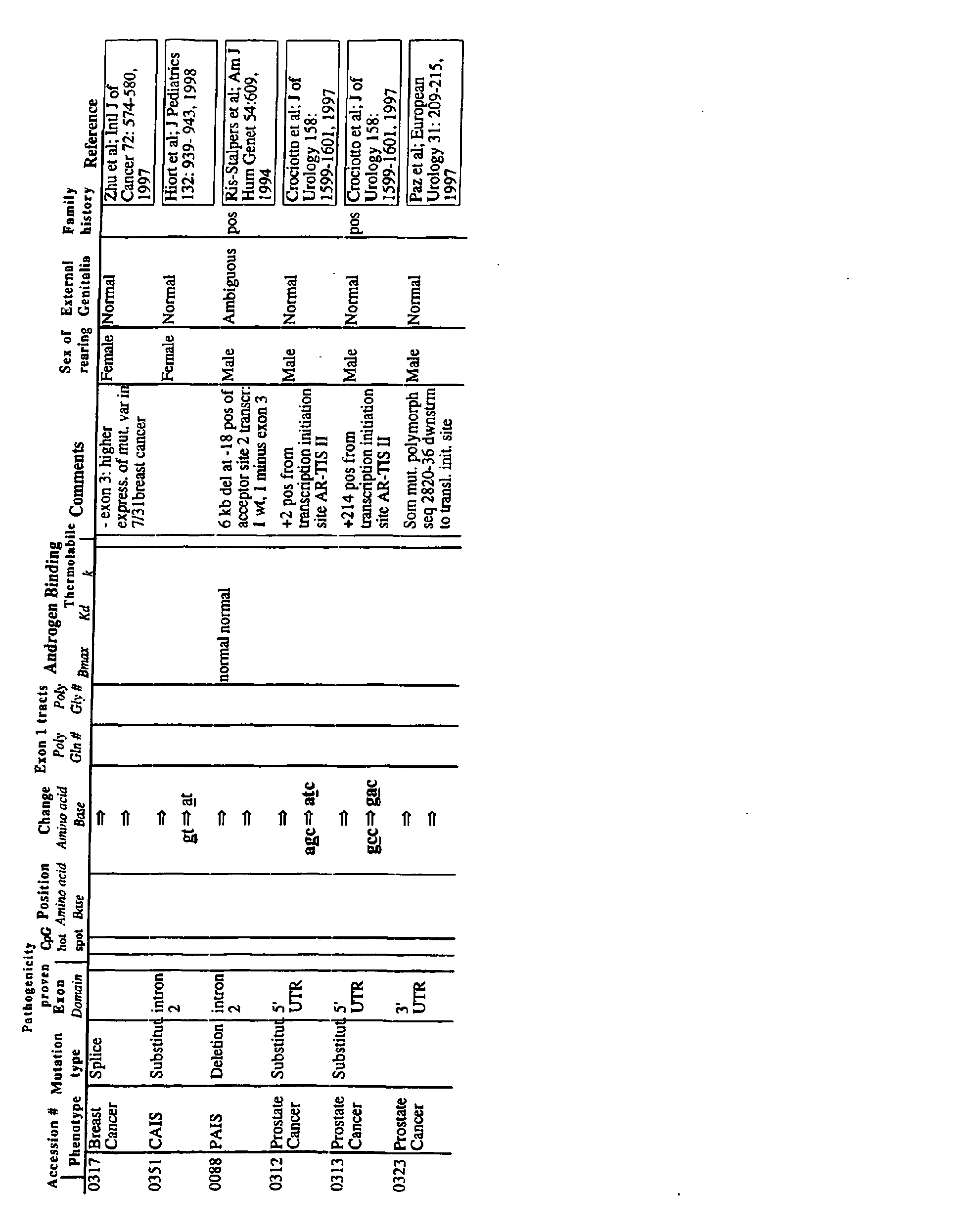

## Revendications

1. Un oligonucléotide double brin **caractérisé en ce qu'**il est constitué de deux séquences oligonucléotidiques complémentaires formant un hybride comprenant chacune à l'une de leurs extrémités 3' ou 5' un à cinq nucléotides non appariés formant des bouts simples brins débordant de l'hybride, l'une desdites séquences oligonucléotidiques étant complémentaire d'une séquence cible appartenant à une molécule d'ADN ou d'ARN d'un gène codant un récepteur aux androgènes muté ou non muté, l'une desdites séquences oligonucléotidiques étant représentée dans la liste de séquence en annexe sous le numéro SEQ ID NO. 15 ou SEQ ID NO. 16.

2. Un oligonucléotide selon la revendication 1, **caractérisé en ce qu'**il est couplé à des substances favorisant ou permettant leur pénétration, ciblage ou adressage dans les cellules.

3. Composition notamment pharmaceutique pour être utilisée à des fins thérapeutiques ou diagnostiques, **caractérisée en ce qu'**elle comprend à titre d'agent actif au moins un oligonucléotide selon l'une quelconque des revendications 1 ou 2.

4. Utilisation d'un oligonucléotide selon l'une quelconque des revendications 1 ou 2 pour le traitement d'un cancer.

5. Utilisation selon la revendication 4, **caractérisée en ce que** ledit cancer est le cancer de la prostate.

## Claims

1. A double-strand oligonucleotide **characterized in that** it consists of two complementary oligonucleotide sequences forming a hybrid, each comprising at one of their 3' or 5' ends, one to five unpaired nucleotides forming single-strand ends extending beyond the hybrid, one of said oligonucleotide sequences being complementary to a target sequence belonging to a DNA or RNA molecule of a gene coding a mutated or nonmutated androgen receptor, one of said oligonucleotide sequences being represented in the sequence listing in the annex under the number SEQ ID NO: 15 or SEQ ID NO: 16.

2. Oligonucleotide according to claim 1, **characterized in that** it is coupled to substances promoting or enabling its penetration, targeting or addressing in cells.

3. Composition, especially pharmaceutical, to be used for therapeutic or diagnostic purposes, **characterized in that** it comprises as active agent at least one oligonucleotide according to either one of claims 1 or 2.

4. Use of an oligonucleotide according to either one of claims 1 or 2, for the treatment of cancer.

5. Use according to claim 4, **characterized in that** said cancer is a prostate cancer.

## Patentansprüche

1. Ein doppelsträngiges Oligonukleotid, **dadurch gekennzeichnet, dass** es aus zwei komplementären Oligonukleotidsequenzen besteht, die ein Hybrid bilden, wobei jede an einem ihrer 3'- oder 5' - Enden ein bis fünf nicht gepaarte Nukleotide umfasst, die einsträngige Enden bilden, die über das Hybrid herausragen, wobei eine der besagten Oligonukleidsequenzen komplementär zu einer Zielsequenz ist, die zu dem DNA- oder RNA-Molekül eines Gens gehört, das einen mutierten oder nicht mutierten Androgen-Rezeptor kodiert, wobei eine der besagten Oligonukleotidsequenzen in der beigefügten Sequenzenliste im Anhang unter der Nummer SEQ ID NO : 15 oder SEQ ID NO : 16 aufgeführt ist.

2. Oligonukleotid gemäß Anspruch 1, **dadurch gekennzeichnet, dass** es an Substanzen gekoppelt ist, die ihre Durchdringung, Targeting oder Adressierung in den Zellen fördern oder ermöglichen.

3. Insbesondere pharmazeutische Zusammensetzung, die zur Therapie oder Diagnosezwecken verwendet werden kann, **dadurch gekennzeichnet, dass** sie als aktive Substanz mindestens einen Oligonukleotiden gemäß einem beliebigen der Ansprüche 1 oder 2 enthält.

4. Verwendung eines Ologonukleotiden gemäß einem beliebigen der Ansprüche 1 oder 2 zur Behandlung von Krebs.

5. Verwendung gemäß Anspruch 4, **dadurch gekennzeichnet, dass** es sich bei besagtem Krebs um Prostatakrebs handelt.
